# EUROPEAN PATENT SPECIFICATION

(11) **EP 4 340 855 B1**
(45) Date of publication and mention of the grant of the patent: **23.09.2026**
(21) Application number: 22769366.0
(22) Date of filing: 13.08.2022
(51) Int. Cl.: A61K 35/36, C12N 5/071, C07K 14/62, C07K 14/515

(54) **A SKIN SUBSTITUTE COMPOSITION AND METHODS OF PRODUCING AND USING THE SAME**
HAUTERSATZZUSAMMENSETZUNG UND VERFAHREN ZUR HERSTELLUNG UND VERWENDUNG DAVON
COMPOSITION DE SUBSTITUT DE PEAU ET SES PROCÉDÉS DE PRODUCTION ET D'UTILISATION

(30) Priority: 13.08.2021 US 202163233196 P
(43) Date of publication of application: 27.03.2024
(73) Proprietor: Triovance Holding LLC, San Diego, California 92119 (US)
(72) Inventor: ESPARZA SILVA, Ana Luisa, San Diego, California 92119 (US); SEGURA PACHECO, Blanca Angélica, San Diego, California 92119 (US)
(74) Representative: J A Kemp LLP
(86) International application number: PCT/IB2022/057587
(87) International publication number: WO 2023/017494

(56) References cited:
- EP-A1- 3 799 882
- WO-A2-03/093418
- LEI PEDRO ET AL: "Efficient Production of Bioactive Insulin from Human Epidermal Keratinocytes and Tissue-Engineered Skin Substitutes: Implications for Treatment of Diabetes", TISSUE ENGENEERING, vol. 13, no. 8, 1 August 2007 (2007-08-01), US, pages 2119 - 2131, XP055982913, ISSN: 1076-3279, DOI: 10.1089/ten.2006.0210
- SUPP DOROTHY M ET AL: "Enhanced vascularization of cultured skin substitutes genetically modified to overexpress vascular endothelial growth factor", JOURNAL OF INVESTIGATIVE DERMATOLOGY, ELSEVIER, NL, vol. 114, no. 1, 1 January 2000 (2000-01-01), pages 5 - 13, XP002387407, ISSN: 0022-202X, DOI: 10.1046/J.1523-1747.2000.00824.X

## Description

### Field

The present disclosure relates in some aspects to a skin substitute composition comprising a stratified epidermis, wherein cells of the stratified epidermis produce, e.g., secrete, a recombinant growth factor and recombinant insulin. In some aspects, the disclosure further relates to methods of manufacturing a skin substitute and methods of using the composition for treatment of a subject, such as for wound healing.

### Background

Skin substitute or skin equivalent compositions are useful to effect wound healing in subjects in need thereof. However, the wound healing capability of such compositions is limited and requires high subject compliance, such as by necessitating several applications of the composition over the course of wound healing. Improved compositions that effect potent wound healing with minimal subject intervention are needed. Provided herein are compositions and methods that meet such needs.

WO 03/093418 describes skin substitutes that deliver therapeutic agents, including insulin, to a subject.

Dorothy M et al. 2000 Journal of Investigative Dermatology vol. 114 describes keratinocytes transduced with a retrovirus expressing proinsulin and furin-cleavable proinsulin,

### Summary

The provided invention encompasses a skin substitute comprising a stratified epidermis comprising a basal layer, a spinous layer, a granular layer and a stratum corneum, wherein cells of the stratified epidermis express a recombinant growth factor and a recombinant insulin: wherein the recombinant growth factor is a vascular endothelial growth factor (VEGF). In some of any embodiments, the recombinant growth factor and recombinant insulin are secretable from cells of the stratified epidermis. In some of any embodiments, the stratified epidermis is 100-200 µm thick. In some of any embodiments, the cells of the stratified epidermis that express the recombinant growth factor and the recombinant insulin comprise cells of the basal layer. In some of any embodiments, the recombinant insulin is or comprises a recombinant human insulin.

In some of any embodiments, the recombinant insulin has (i) the sequence of amino acids set forth in SEQ ID NO: 5, (ii) a functional variant of the recombinant insulin that has a sequence of amino acids that has at least 85%, at least 90%, or at least 95% sequence identity to SEQ ID NO: 5; or (iii) a two-chain form of (i) or (ii) that comprises an A-chain and a B-chain. In some of any embodiments, the A-chain and B-chain are linked by a disulfide bond. In some of any embodiments, the recombinant insulin is encoded by a polynucleotide that encodes (i) the sequence of amino acids set forth in SEQ ID NO: 5, or (ii) a functional variant that has a sequence of amino acids that has at least 85%, at least 90%, or at least 95% sequence identity to SEQ ID NO: 5. In some of any embodiments, the recombinant insulin is an AspB10 insulin analog comprising a histidine to aspartic acid mutation at position 10 in the B chain of the modified human proinsulin compared to wild-type insulin set forth in SEQ ID NO:5

In some of any embodiments, the skin substitute comprises a polynucleotide encoding a proinsulin, the proinsulin comprising at least one furin recognition sequence in place of the endopeptidase Arg31-Arg32 cleavage site or the endopeptidase Lys64-Arg65 cleavage site. In some of any embodiments, the at least one furin recognition sequence is in place of the endopeptidase Arg31-Arg32 cleavage site and the endopeptidase Lys64-Arg65 cleavage site. In some of any embodiments, the at least one furin recognition sequence comprises the consensus sequence R-X-R-R, where X is any amino acid (SEQ ID NO: 8), or R-X-K-R, where X is any amino acid (SEQ ID NO: 9). In some of any embodiments, the at least one furin cleavage site is RTKR (SEQ ID NO: 10) or RQKR (SEQ ID NO: 42).

In some of any embodiments, the recombinant insulin has (i) the sequence of amino acids set forth in SEQ ID NO: 6, (ii) a functional variant that has a sequence of amino acids that has at least 85%, at least 90%, or at least 95% sequence identity to SEQ ID NO: 6; or (iii) a two-chain form of (i) or (ii) that comprises an A-chain and a B-chain. In some of any embodiments, the A-chain and B-chain are linked by a disulfide bond. In some of any embodiments, the recombinant insulin comprises the sequence set forth in SEQ ID NO: 6 or a two-chain form of (i) or (ii) that comprises an A-chain and a B-chain. In some of any embodiments, the A-chain and B-chain are linked by a disulfide bond. In some of any embodiments, the recombinant insulin comprises an A chain set forth in SEQ ID NO: 36 and a B chain set forth in SEQ ID NO: 41. In some of any embodiments, the A-chain and B-chain are linked by a disulfide bond.

In some of any embodiments, the recombinant human insulin is encoded by a polynucleotide that comprises a sequence that has at least at or about 85%, at least at or about 90% or at least at or about 95% sequence identity to the sequence set forth in SEQ ID NO: 2. In some of any embodiments, the recombinant human insulin comprises the sequence set forth in SEQ ID NO: 2.. In some of any embodiments, the recombinant growth factor is a VEGF.

In some of any embodiments, the VEGF is encoded by a polynucleotide sequence that has at least at or about 85%, at least at or about 90%, or at least at or about 95% sequence identity to the sequence set forth in SEQ ID NO: 4. In some of any embodiments, the VEGF is encoded by the polynucleotide sequence that comprises the sequence set forth in SEQ ID NO: 4. In some of any embodiments, the VEGF comprises a sequence that has at least at or about 85%, at least at or about 90% or at least at or about 95% sequence identity to the sequence set forth in SEQ ID NO: 7 or a sequence thereof that lacks the signal peptide. In some of any embodiments, the VEGF comprises the sequence set forth in SEQ ID NO: 7 or a sequence thereof that lacks the signal peptide.

In some of any embodiments, the skin substitute comprises a VEGF and the VEGF comprises a sequence that has at least at or about 85%, at least at or about 90% or at least at or about 95% sequence identity to the sequence set forth in SEQ ID NO: 44. In some of any embodiments, the VEGF comprises the sequence set forth in SEQ ID NO: 44. In some of any embodiments, the recombinant growth factor and the recombinant insulin are encoded by a bicistronic expression cassette comprising a polynucleotide encoding the recombinant growth factor and a polynucleotide encoding a recombinant insulin separated by a bicistronic element.

In some of any embodiments, the bicistronic element is an IRES. In some of any embodiments, polynucleotides encoding the recombinant growth factor and recombinant insulin are operably linked to a promoter. In some of any embodiments, the promoter is a constitutive promoter or an inducible promoter. In some of any embodiments, the promoter is a CAG promoter. In some of any embodiments, the polynucleotide encoding the recombinant growth factor are upstream of the polynucleotide encoding the recombinant insulin in the bicistronic expression cassette.

In some of any embodiments, cells of the stratified epidermis secrete the recombinant growth factor and the recombinant insulin at levels that result in greater improvement in one or more marker(s) of angiogenic reorganization relative to a skin substitute comprising either the recombinant growth factor or recombinant insulin alone. In some of any embodiments, the improvement in one or more marker(s) of angiogenic reorganization can be evaluated in a tube formation assay. In some of any embodiments, the marker of angiogenic reorganization is an increase in the number of nodes or unions, defined as the bond sites of at least three chords..In some of any embodiments, the marker of angiogenic reorganization is an increase in the number of webs, defined as a closed circuit surrounded by two or more nodes. In some of any embodiments, the marker of angiogenic reorganization is an increase in the number of main segments, defined as chords that bond two nodes together

In some of any embodiments, cells of the stratified epidermis continuously secrete a quantifiable level of the recombinant growth factor and the recombinant insulin. In some of any embodiments, cells of the stratified epidermis continuously secrete the recombinant growth factor and the recombinant insulin for up to or about 2 days, up to or about 3 days, up to or about 4 days, up to or about 5 days, up to or about 6 days, up to or about 7 days, up to or about 8 days, up to or about 9 days, up to or about 10 days, up to or about 11 days, up to or about 12 days, up to or about 13 days, or up to or about 14 days. In some of any embodiments, cells of the stratified epidermis continuously secrete the recombinant growth factor and the recombinant insulin for up to or about one week, up to or about two weeks, up to or about three weeks, up to or about one to two weeks, or up to or about two to three weeks.

In some of any embodiments, cells of the stratified epidermis secrete a quantifiable level of the recombinant growth factor and C-peptide that can be detected for up to or about 2 days, up to or about 3 days, up to or about 4 days, up to or about 5 days, up to or about 6 days, up to or about 7 days, up to or about 8 days, up to or about 9 days, up to or about 10 days, up to or about 11 days, up to or about 12 days, up to or about 13 days, or up to or about 14 days. In some of any embodiments, cells of the stratified epidermis secrete a quantifiable level of the recombinant growth factor and C-peptide that can be detected for up to or about one week, up to or about two weeks, up to or about three weeks, up to or about one to two weeks, or up to or about two to three weeks.

In some of any embodiments, cells of the stratified epidermis secrete the recombinant growth factor and the recombinant insulin at levels that reduce the levels of advanced glycation end products (AGEs) in the skin of a subject. In some of any embodiments, the cells of the stratified epidermis are differentiated from keratinocytes. In some of any embodiments, the keratinocytes are human keratinocytes. In some of any embodiments, the keratinocytes are HaCaT keratinocyte cells.

In some aspects, provided herein is a bicistronic expression cassette comprising a polynucleotide encoding a recombinant human growth factor and a recombinant insulin, wherein the recombinant growth factor is a VEGF. In some of any embodiments, the encoded recombinant insulin has (i) the sequence of amino acids set forth in SEQ ID NO:5, or (ii) is a functional variant that has a sequence of amino acids that has at least 85%, at least 90%, or at least 95% sequence identity to SEQ ID NO:5. In some of any embodiments, the polynucleotide encoding the recombinant insulin comprises (i) the sequence of amino acids set forth in SEQ ID NO:5, or (ii) is a functional variant that has a sequence of amino acids that has at least 85%, at least 90%, or at least 95% sequence identity to SEQ ID NO:5.

In some of any embodiments, the encoded recombinant insulin is an AspB10 insulin analog comprising a histidine to aspartic acid mutation at position 10 in the B chain of the modified human proinsulin compared to wild-type insulin set forth in SEQ ID NO:5. In some of any embodiments, the polynucleotide encoding the recombinant insulin encodes a proinsulin comprising at least one furin recognition sequence in place of the endopeptidase Arg31-Arg32 cleavage site or the endopeptidase Lys64-Arg65 cleavage site. In some of any embodiments, the at least one furin recognition sequence is in place of the endopeptidase Arg31-Arg32 cleavage site and the endopeptidase Lys64-Arg65 cleavage site. In some of any embodiments, the at least one furin recognition sequence comprises the consensus sequence R-X-R-R, where X is any amino acid (SEQ ID NO: 8) or R-X-K-R, where X is any amino acid (SEQ ID NO: 9). In some of any embodiments, the at least one furin cleavage site is RTKR (SEQ ID NO: 10) or RQKR (SEQ ID NO: 42).

In some of any embodiments, the encoded recombinant insulin has (i) the sequence of amino acids set forth in SEQ ID NO:6, or (ii) is a functional variant that has a sequence of amino acids that has at least 85%, at least 90%, or at least 95% sequence identity to SEQ ID NO:6.In some of any embodiments, the encoded recombinant insulin comprises the sequence set forth in SEQ ID NO: 6. In some of any embodiments, the polynucleotide encoding recombinant insulin comprises a sequence that has at least at or about 85%, at least at or about 90% or at least at or about 95% sequence identity to the sequence set forth in SEQ ID NO: 2.

In some of any embodiments, the polynucleotide encoding recombinant insulin comprises the sequence set forth in SEQ ID NO: 2..

In some of any embodiments, the recombinant growth factor is a VEGF. In some of any embodiments, the polynucleotide encoding the growth factor comprises a sequence that has at least at or about 85%, at least at or about 90% or at least at or about 95% sequence identity to the sequence set forth in SEQ ID NO: 4. In some of any embodiments, the polynucleotide encoding the growth factor comprises the sequence set forth in SEQ ID NO: 4.In some of any embodiments, the encoded VEGF comprises a sequence that has at least at or about 85%, at least at or about 90% or at least at or about 95% sequence identity to the sequence set forth in SEQ ID NO: 7 or a sequence thereof that lacks the signal peptide.

In some of any embodiments, the encoded VEGF comprises the sequence set forth in SEQ ID NO: 7 or a sequence thereof that lacks the signal peptide. In some of any embodiments, the encoded VEGF comprises a sequence that has at least at or about 85%, at least at or about 90% or at least at or about 95% sequence identity to the sequence set forth in SEQ ID NO: 44. In some of any embodiments, the encoded VEGF comprises the sequence set forth in SEQ ID NO: 44.

In some of any embodiments, the polynucleotide encoding the recombinant growth factor and the polynucleotide encoding the recombinant insulin are separated by a bicistronic element. In some of any embodiments, the bicistronic element is an IRES. In some of any embodiments, the polynucleotides encoding the recombinant growth factor and recombinant insulin are operably linked to a promoter. In some of any embodiments, the promoter is the same. In some of any embodiments, the promoter is a constitutive promoter or an inducible promoter. In some of any embodiments, the promoter is a CAG promoter. In some of any embodiments, the polynucleotide encoding the recombinant growth factor are upstream of the polynucleotide encoding the recombinant insulin in the bicistronic expression cassette.

In some aspects, provided herein is a vector comprising the bicistronic expression cassette of any embodiments provided herein. In some of any embodiments, the vector is a viral vector. In some of any embodiments, the viral vector is an adenoviral vector. In some of any embodiments, the vector is a non-replicative type 5 adenovirus. In some of any embodiments, the non-replicative adenovirus lacks or is deleted in the E1 and E3 region. In some of any embodiments, the bicistronic expression cassette is inserted into the E1 region.

In some aspects, provided herein is a method of manufacturing a skin substitute. In some of any embodiments, the method comprises: 1) differentiating keratinocytes into a stratified epidermis, wherein the stratified epidermis comprises a basal layer, a spinous layer, a granular layer and a stratum corneum; and 2) introducing a bicistronic expression cassette of any of the provided embodiments or the vector of any of the provided embodiments into cells of the stratified epidermis to produce a skin substitute, wherein the skin substitute comprises a recombinant growth factor and recombinant insulin. In some of any embodiments, the introducing is by transduction of the viral vector of any of the provided embodiments.

In some aspects, provided herein is a method of manufacturing a skin substitute. In some of any embodiments, the method comprises: 1) differentiating keratinocytes into a stratified epidermis, wherein the stratified epidermis comprises a basal layer, a spinous layer, a granular layer and a stratum corneum; and 2) transducing a viral vector of any of the provided embodiments to cells of the stratified epidermis to produce a skin substitute, wherein the skin substitute comprises a growth factor and insulin.

In some aspects, provided herein is a method of manufacturing a skin substitute. In some of any embodiments, the method comprises: 1) differentiating keratinocytes into a stratified epidermis, wherein the stratified epidermis comprises a basal layer, a spinous layer, a granular layer and a stratum corneum; and 2) transducing cells of the stratified epidermis with an adenoviral vector that encodes a modified proinsulin and a growth factor to produce a skin substitute, wherein the skin substitute comprises a growth factor and insulin.

In some of any embodiments, at the time of the introducing or transducing, cells of the stratified epidermis express occludin and claudin. In some of any embodiments, cells of the basal layer are introduced or transduced. In some of any embodiments, prior to the differentiating in step 1), the method comprises culturing keratinocytes in a low calcium medium to culture a basal layer for 2 weeks, 3 weeks, 4 weeks, 5 weeks, or 6 weeks. In some of any embodiments, prior to the differentiating in step 1), the method comprises culturing keratinocytes in a low calcium medium to culture a basal layer for at or about 4 weeks.

In some of any embodiments, the low calcium medium comprises a calcium concentration of 0.01-0.1 mM at the time of seeding the cells or during the culturing. In some of any embodiments, the low calcium medium comprises a calcium concentration up to or about 0.05 mM at the time of seeding the cells or during the culturing. In some of any embodiments, the low calcium medium comprises a calcium concentration that is about 0.03 mM at the time of seeding the cells or during the culturing.

In some of any embodiments, the low calcium medium further comprises epidermal growth factor (EGF) and bovine pituitaries extract (BPE). In some of any embodiments, the low calcium medium comprises up to or about 1 ng/ml EGF and up to or about 100 µg/ml BPE at the time of seeding the cells or during the culturing. In some of any embodiments, the low calcium medium comprises up to or about 0.2 ng/ml EGF and up to or about 30 µg/ml BPE at the time of seeding the cells or during the culturing. In some of any embodiments, the keratinocytes are human keratinocytes. In some of any embodiments, the keratinocytes are HaCaT keratinocyte cells.

In some of any embodiments, step 1) comprises culturing the keratinocytes on an extracellular matrix substrate. In some of any embodiments, the extracellular matrix substrate is collagen. In some of any embodiments, the extracellular matrix substrate is human use certified. In some of any embodiments, the keratinocytes are seeded on the extracellular matrix substrate at a cell density of between 5 x 10⁶ cells/mL and 50 x 10⁶ cells/mL. In some of any embodiments, the cell density is at or about 10 x 10⁶ cells/ml, 20 x 10⁶ cells/ml, 30 x 10⁶ cells/ml or 40 x 10⁶ cells/ml, or any value between any of the foregoing. In some of any embodiments, the cell density is at or about 20 x 10⁶ cells/ml. In some of any embodiments, the extracellular matrix substrate is coated on a transwell insert.

In some of any embodiments, the culturing in step (1) is for about 23 to 28 days. In some of any embodiments, the culturing in step (1) comprises a first incubation in low calcium medium and a second incubation in a high calcium medium. In some of any embodiments, first incubation in low calcium medium is for about 3-5 days, and the second incubation in high calcium medium is for about 20-23 days.In some of any embodiments, the low calcium medium comprises 0.01-0.1 mM calcium. In some of any embodiments, the high calcium medium comprises 1.0-3.0 mM. In some of any embodiments, the low calcium medium comprises 0.03 mM calcium, and the high calcium medium comprises 2.4 mM calcium.

In some of any embodiments, the low calcium medium and the high calcium medium further comprise EGF and BPE. In some of any embodiments, the low calcium medium and the high calcium medium comprise 0.05 ng/mL to 1 ng/ml EGF and from 1 µg/ml to 100 µg/ml BPE. In some of any embodiments, the low calcium medium and the high calcium medium comprise at or about 0.2 ng/ml EGF and at or about 30 µg/ml BPE. In some of any embodiments, the high calcium medium further comprises hydrocortisone.

In some of any embodiments, the high calcium medium comprises from 0.1 to 1.0 µg/ml hydrocortisone. In some of any embodiments, the high calcium medium comprises at or about 0.4 µg/ml hydrocortisone. In some of any embodiments, the low calcium medium is a serum free media. In some of any embodiments, the high calcium medium is a serum free medium.

In some of any embodiments, during the second incubation, an air-liquid interface is introduced upon culturing the keratinocytes in the high calcium medium, wherein cells of the basal layer are exposed to the high culture medium but not to the gaseous environment. In some of any embodiments, the low calcium medium is replaced daily during the first incubation. In some of any embodiments, the high calcium medium is replaced daily during the second incubation.

In some of any embodiments, after step 2), the method can further comprise formulating the skin substitute with a cryoprotectant. In some of any embodiments, the cryoprotectant comprises human albumin and glucose. In some of any embodiments, the provided methods further comprise freezing the skin substitute after step 2). In some of any embodiments, the provided methods can further comprising performing a quality control assessment on the skin substitute. In some of any embodiments, the quality control assessment is performed prior to formulating the skin substitute with the cryoprotectant. In some of any embodiments, up to or about 24 hours passes between completion of step 2) and the quality control step. In some of any embodiments, the quality control step comprises detecting one or more polypeptides selected from the group consisting of proinsulin, a modified proinsulin, insulin, an insulin variant, a growth factor, and a variant thereof.

In some of any embodiments, the provided methods can further comprise placing the skin substitute on an absorbent gauze. In some of any embodiments, the keratinocytes comprise immortalized keratinocytes. In some of any embodiments, the keratinocytes comprise cells from an HaCaT cell line, an NM1 cell line, or a NIKS cell line and/or cells derived from the HaCaT cell line, NM1 cell line, or NIKS cell line.

. In some aspects, provided herein is a cryopreserved skin substitute, comprising the skin substitute of any of any of the provided embodiments and a cryoprotectant. In some of any embodiments, the cryoprotectant comprises human albumin (0.02 g/mL) and D-glucose (0.09 g/mL).

In some aspects, provided herein is a skin substitute comprising the skin substitute of any of any of the provided embodiments or the cryopreserved skin substitute of any of the provided embodiments and an absorbent gauze, wherein the cryopreserved skin substitute is overlaid on the absorbent gauze. In some of any embodiments, the absorbent gauze is a Vaseline Petrolatum Gauze. In some of any embodiments, the cryopreserved skin substitute is about 40-50 cm², about 40-45 cm², or about 45-50 cm² in size and the absorbent gauze is about 40-60 cm², about 45-60 cm², about 45-55 cm² in size. In some of any embodiments, the cryopreserved skin substitute is at or about 41 cm², at or about 42 cm², at or about 43 cm², at or about 44 cm², at or about 45 cm², at or about 46 cm², at or about 47 cm² in size and the absorbent gauze is about at or about 47 cm², at or about 48 cm², at or about 49 cm², at or about 50 cm², at or about 51 cm², at or about 52 cm², at or about 53 cm² in size. In some of any embodiments, the cryopreserved skin substitute of any of the provided embodiments or the skin substitute dressing of any of the provided embodiments can be sterile.

In some aspects, provided herein is a container comprising a skin substitute comprising a stratified epidermis comprising a basal layer, a spinous layer, a granular layer and a stratum corneum, wherein cells of the stratified epidermis express a recombinant growth factor and a recombinant insulin: wherein the recombinant growth factor is a vascular endothelial growth factor (VEGF). In some of any embodiments, the container can comprise the skin substitute of any of the provided embodiments, the cryopreserved skin substitute of any of the provided embodiments or the skin substitute dressing any of the provided embodiments. In some of any embodiments, wherein the container is a bag. In some of any embodiments, wherein the container is sterile and/or heat-sealed.

In some aspects, provided herein is a method for preparing a skin substitute dressing, the method comprising placing the skin substitute of any of the provided embodiments or the cryopreserved skin substitute of any of the provided embodiments on an absorbent gauze. In some of any embodiments, the absorbent gauze is a Vaseline Petrolatum Gauze.

In some of any embodiments, the cryopreserved skin substitute is about 40-50 cm², about 40-45 cm², or about 45-50 cm² in size and the absorbent gauze is about 40-60 cm², about 45-60 cm², about 45-55 cm² in size. In some of any embodiments, the cryopreserved skin substitute is at or about 41 cm², at or about 42 cm², at or about 43 cm², at or about 44 cm², at or about 45 cm², at or about 46 cm², at or about 47 cm² in size and the absorbent gauze is about at or about 47 cm², at or about 48 cm², at or about 49 cm², at or about 50 cm², at or about 51 cm², at or about 52 cm², at or about 53 cm² in size.

In some aspects, provided herein is a skin substitute, a cryopreserved skin substitute, or a skin substitute dressing of the provided embodiments for use in a method of promoting wound healing, wherein the method comprises applying to a wound the skin substitute of any of the provided embodiments, the cryopreserved skin substitute of any of the provided embodiments, or the skin substitute dressing of any of the provided embodiments

In some of any embodiments, the skin substitute prevents microbial infection. In some of any embodiments, the skin substitute is applied to an acute wound and/or a chronic wound.

In some of any embodiments, the wound is selected from the group consisting of: a sore, an open wound, an ulcer, and an abscess. In some of any embodiments, the skin substitute is applied to a wound on a diabetic patient. In some of any embodiments, the wound is a diabetic ulcer In some of any embodiments, the wound is a diabetic foot ulcer. In some of any embodiments, the wound is a venous leg ulcer.

### Brief Description of the Drawings

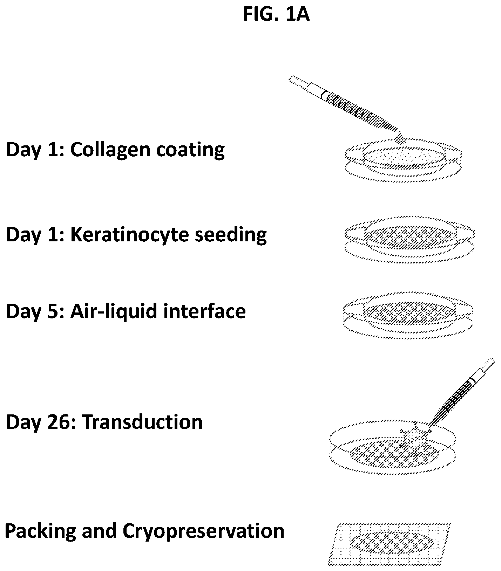

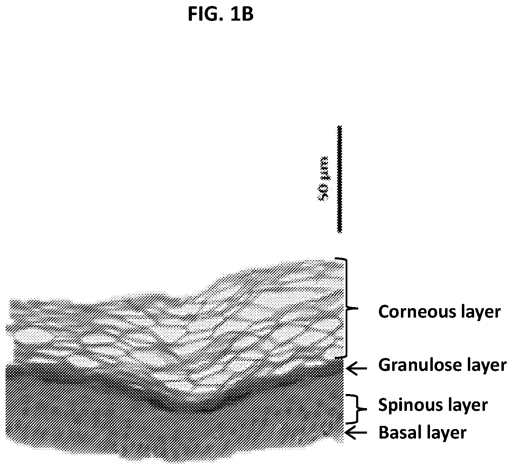

**FIG. 1A** shows a diagram depicting representative steps involved in a method of producing a skin substitute comprising a stratified epidermis. **FIG. 1B** shows a representative example of a paraffin-embedded hematoxylin and eosin stained skin substitute at day 25 of culture on a substrate, wherein the corneous, granulose, spinous, and basal layers formed by differentiated keratinocytes are indicated.

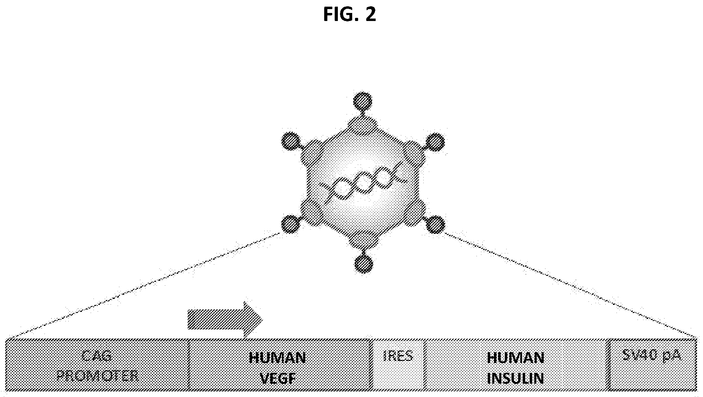

**FIG. 2** shows a graphical representation of a viral vector and an exemplary expression construct comprising a growth factor, insulin, and regulatory elements.

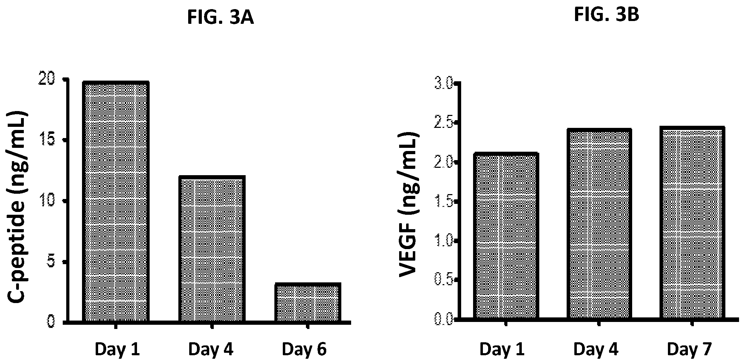

**FIG. 3A** shows the average level of C-peptide (ng/mL) detected on day 1, day 4, and day 6 in an in vitro study of protein release from a skin substitute comprising a stratified epidermis. **FIG. 3B** shows the average level of VEGF (ng/mL) detected on day 1, day 4, and day 7 in an in vitro study of protein release from a skin substitute comprising a stratified epidermis. Experiments were performed in triplicate (n=3).

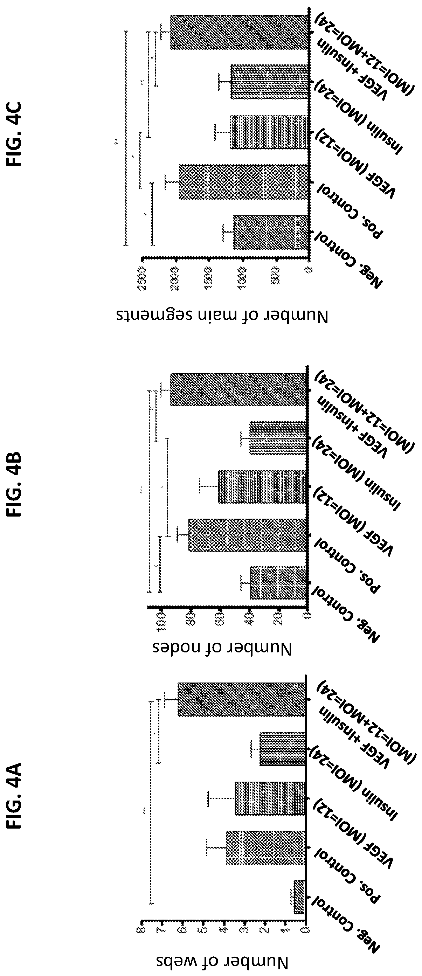

**FIG. 4A****-****FIG. 4C** show the markers of wound healing in response to VEGF (MOI=12), insulin (MOI=24), or VEGF and insulin in combination (MOI=12 and MOI=24, respectively), in an endothelial cell tube formation assay. **FIG. 4A** shows the number of webs observed in response to negative and positive controls, VEGF, insulin, or VEGF+insulin. **FIG. 4B** shows the number of nodes observed in response to negative and positive controls, VEGF, insulin, or VEGF+insulin. **FIG. 4C** shows the number of main segments observed in response to negative and positive controls, VEGF, insulin, or VEGF+insulin. Experiments were performed in triplicate, and data is shown as the average ± standard deviation.

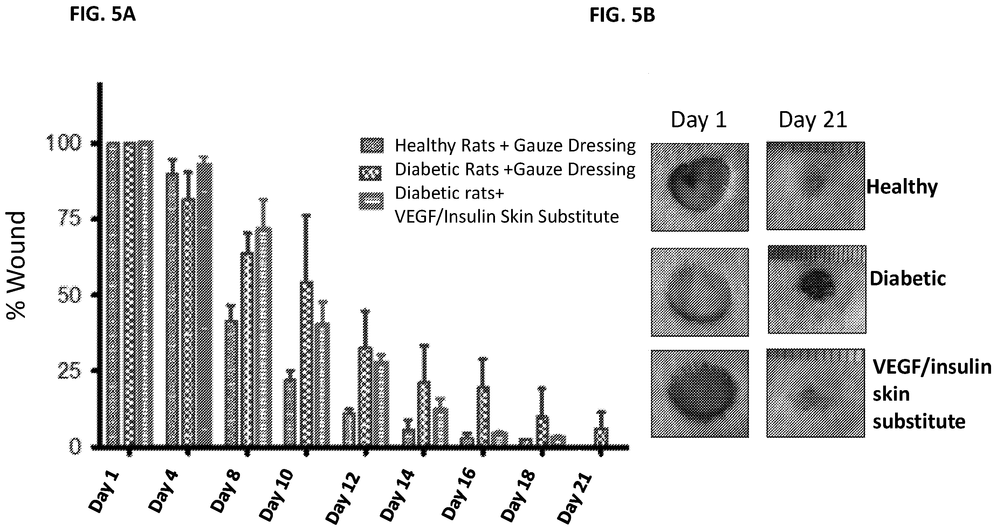

**FIG. 5A** shows the percentage of open wound area over a 21-day period in healthy rats and diabetic rats treated with standard wound dressings, and diabetic rats treated with a skin substitute that secretes both VEGF and insulin. There were a total of seven rats in each group, and data is shown as the average ± standard deviation. **FIG. 5B** shows representative images of wound initiation (day 1) and the extent of wound closure (day 21) in the skin of healthy and diabetic rats treated with standard wound dressings or treated with the VEGF/insulin skin substitute.

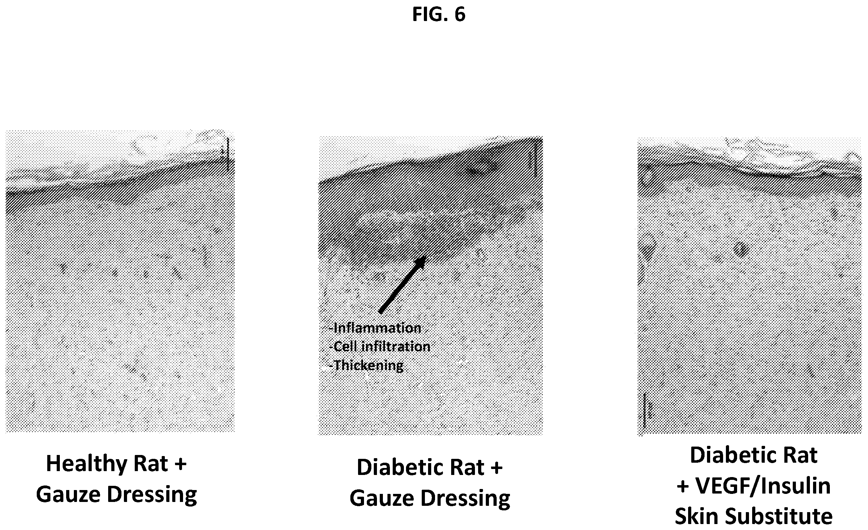

**FIG. 6** shows representative images of paraffin-embedded hematoxylin and eosin stained sections of rat wounds at 21 days post-wound initiation in a healthy rat treated with a gauze dressing (left), a diabetic rat treated with a gauze dressing (middle), and a diabetic rat treated with the VEGF/insulin skin substitute (right).

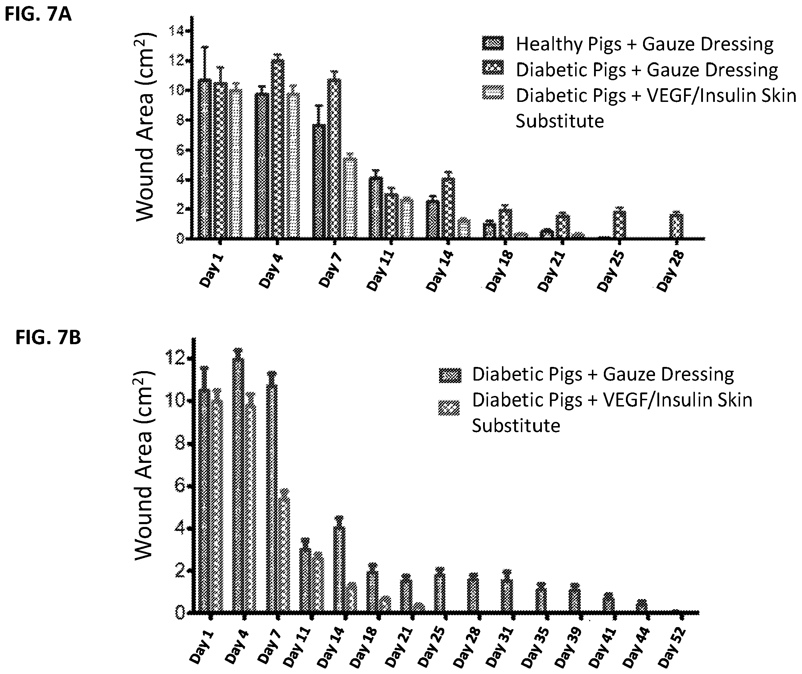

**FIG. 7A** shows a comparison of wound area (cm²) between healthy pigs (n=3) and diabetic pigs (n=3) treated with gauze dressings, and diabetic pigs treated with the VEGF/insulin skin substitute (n=3) over 28 days. **FIG. 7B** shows a comparison of wound area (cm²) between the diabetic pigs treated with gauze dressings and the diabetic pigs treated with the VEGF/insulin skin substitute over 52 days. Data is presented as the average ± standard deviation.

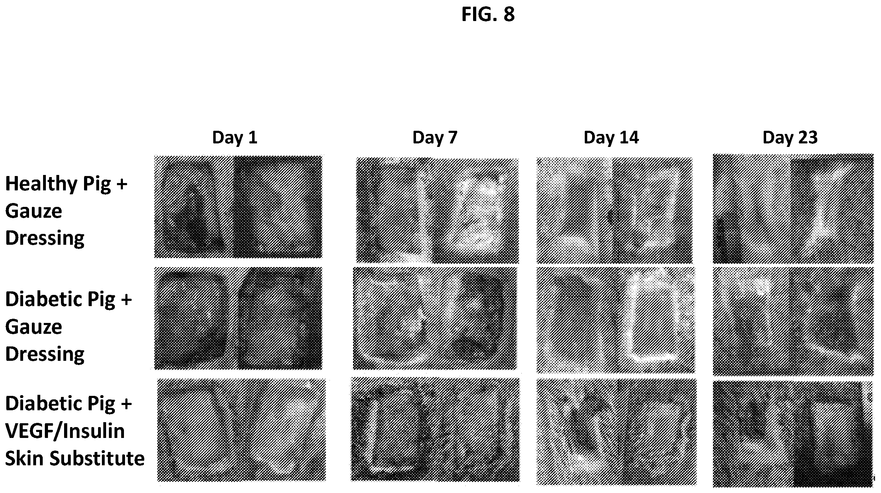

**FIG. 8** shows representative images of wounds in healthy (top) and diabetic pigs (middle) treated with gauze dressings, and diabetic pigs treated with the VEGF/insulin skin substitute (bottom).

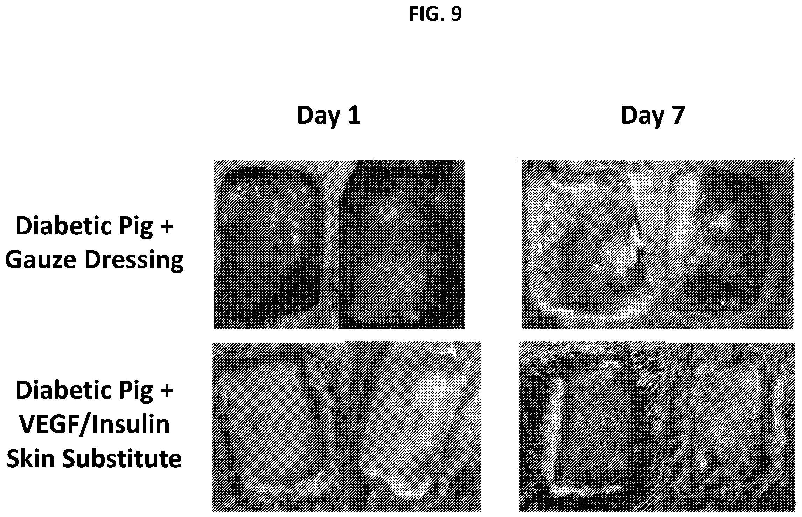

**FIG. 9** shows representative images of the wound area of diabetic pigs treated with gauze dressings (top) and diabetic pigs treated with the VEGF/insulin skin substitute (bottom) at day 1 and day 7 post-wound initiation.

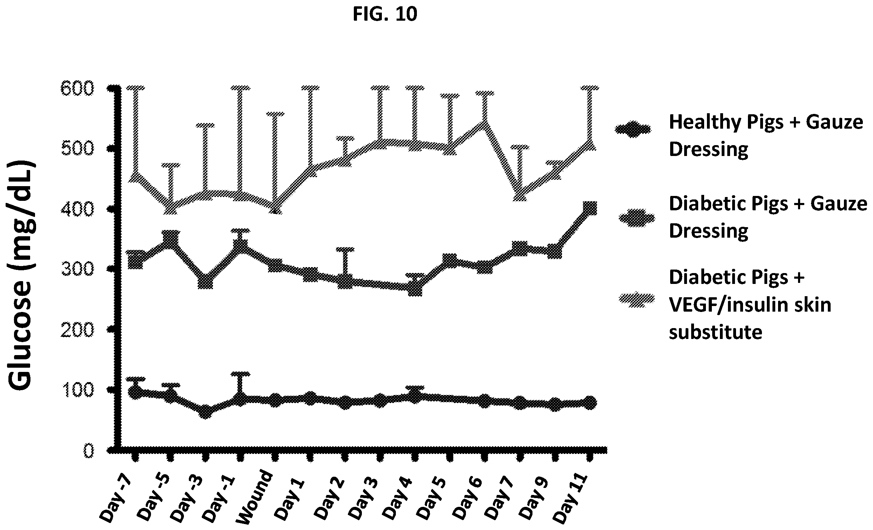

**FIG. 10** shows glucose levels (mg/dL) prior to and up to 11 days post-wound-initiation of healthy and diabetic pigs treated with gauze dressings, and diabetic pigs treated with the VEGF/insulin skin substitute. Data is presented as the average ± standard deviation (n=3).

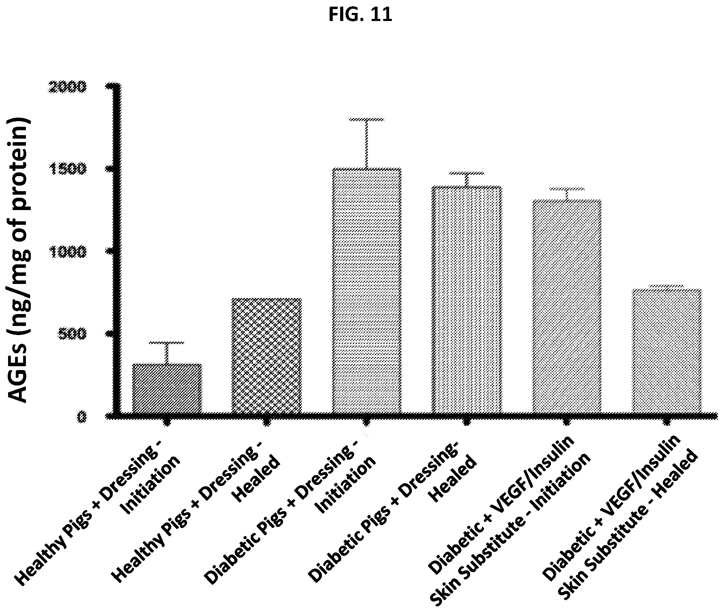

**FIG. 11** shows the level of advanced glycation end products (AGEs) (ng/mg of protein) in skin samples collected at the time of wound initiation and at the time of wound healing from healthy and diabetic pigs treated with gauze dressings, and diabetic pigs treated with the VEGF/insulin skin substitute. Data is presented as the average ± standard deviation (n=3).

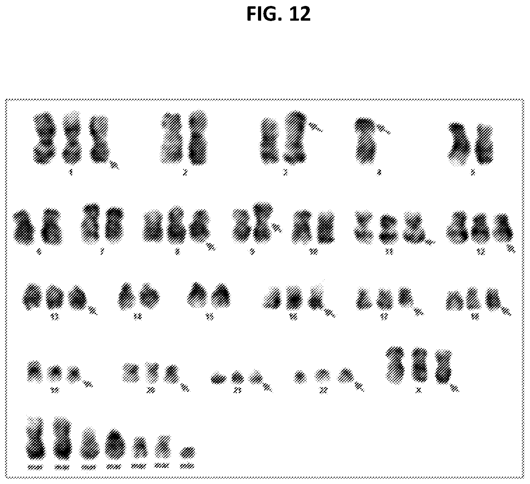

**FIG. 12** depicts a karyogram corresponding to a cytogenetic analysis of HaLow cells (HaCat cells grown in low calcium media without fetal bovine serum) after their propagation in culture.

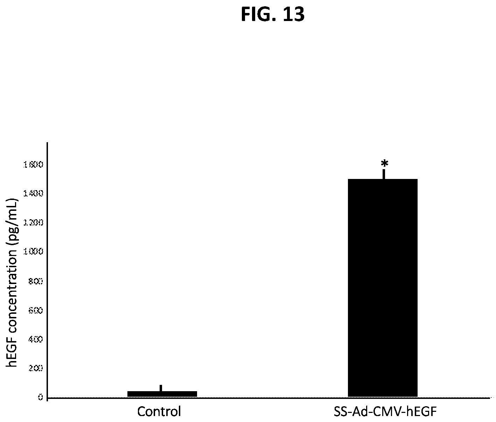

**FIG. 13** depicts a graph showing quantification of human epidermal growth factor (hEGF) expression in skin substitutes transduced with an adenovirus expressing hEGF (Ad-CMV-hEGF). Non-transduced skin substitutes were included as experimental controls. The results are expressed as the mean ± SEM of repeats *p < 0.05 as compared with control.

### Detailed Description

Provided herein are compositions of a skin substitute comprising a stratified epidermis, wherein a recombinant growth factor and recombinant insulin are secretable from cells of the stratified epidermis. In some aspects, provided herein are methods of manufacturing a skin substitute comprising a stratified epidermis, the cells of which produce, e.g., secrete, a recombinant growth factor and recombinant insulin. In other aspects, provided herein are methods of treating a subject in need of wound healing, such as a diabetic subject.

Wound healing is a complex process that is compromised in certain patient populations, e.g., diabetic subjects. High levels of glucose in the blood of diabetic subjects promotes the formation of advanced glycation end products (AGEs). AGEs induce changes both in the structure and the vascularization of the skin, effecting delayed wound healing or prolonged time to wound closure relative to a non-diabetic subject. To further complicate the wound healing process, diabetic patients are prone to acquiring infections, e.g., bacterial and/or fungal infections.

Certain skin substitute compositions are FDA-approved for wound healing applications, specifically to treat diabetic foot ulcers. Some limitations of the skin substitutes that are currently available include high cost, the need for several applications, and limited effectiveness, such as by failing to promote or achieve adequate scarring, a key component of the wound healing process. In contrast, the skin substitute composition provided herein can promote wound healing, including scar formation, in diabetic subjects with few applications. In some cases, only one application is necessary to effect wound healing (e.g., time to wound closure) comparable to that observed in a non-diabetic subject. In a further advantage, the skin substitute provided herein can prevent microbial infection, thereby inhibiting any further complications to the wound healing process.

The skin substitute provided herein is composed of differentiated keratinocytes that form a stratified epidermis. Cells of the basal layer of the stratified epidermis are transduced with a recombinant polynucleotide encoding a growth factor and insulin, thereby facilitating secretion of mature forms of a growth factor and insulin from cells of the stratified epidermis. Levels of VEGF and insulin secreted by the skin substitute are lower than those reportedly necessary to cause tumor induction or a decrease in systemic glucose, respectively. Instead, the combination of VEGF and insulin at levels secretable from the skin substitute can potently promote angiogenesis to a greater extent than either VEGF or insulin alone. The combination can also decrease the amount of AGEs in a subject's skin. Further, secreted levels of a growth factor and insulin can persist over the course of at least 7 days, thereby providing sustained release of the wound healing combination.

The section headings used herein are for organizational purposes only and are not to be construed as limiting the subject matter described.

### I. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known to the skilled person in this technical field. Reference to "about" a value or parameter herein includes (and describes) embodiments that are directed to that value or parameter per se. For example, description referring to "about X" includes description of "X". The term "about" can also encompass variations, which can be up to ± 5%, but can also be ± 4%, 3%, 2%, 1 %, etc. Whether or not modified by the term "about," the claims include equivalents to the quantities.

The term "expression", as used herein, refers to the process by which a polypeptide is produced based on the encoding sequence of a nucleic acid molecule, such as a gene. The process may include transcription, post-transcriptional control, post-transcriptional modification, translation, post-translational control, post-translational modification, or any combination thereof.

As used herein, "a subject" includes any living organism, such as humans and other mammals. Mammals include, but are not limited to, humans, and non-human animals, including farm animals, sport animals, rodents and pets.

As used herein, "operably linked" or "operatively linked" refers to the association of components, such as a DNA sequence, e.g. a heterologous nucleic acid) and a regulatory sequence(s), in such a way as to permit gene expression when the appropriate molecules (e.g. transcriptional activator proteins) are bound to the regulatory sequence. Hence, it means that the components described are in a relationship permitting them to function in their intended manner.

As used herein, "percent (%) sequence identity" and "percent identity" when used with respect to a nucleotide sequence (reference nucleotide sequence) or amino acid sequence (reference amino acid sequence) is defined as the percentage of nucleotide residues or amino acid residues, respectively, in a candidate sequence that are identical with the residues in the reference sequence, after aligning the sequences and introducing gaps, if necessary, to achieve the maximum percent sequence identity. Alignment for purposes of determining percent sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Among the vectors are viral vectors, such as adenoviral vectors.

The term "skin substitute," as used herein, refers to materials that replace and/or enhance one or more functions of the skin, e.g., wound healing, either temporarily or permanently, depending on the characteristics of the composition. The structure of a skin substitute can comprise components similar in structure and function to a mammalian epidermis and/or a mammalian dermis.

The term "stratified epithelium," as used herein, refers to an epithelium comprising more than one layer of epithelial cells. The multiple layers of epithelial cells can be distinguishable by biochemical composition and by visual inspection using microscopy. For example, a completely stratified epithelium can mimic the composition of a human epidermis, comprising a basal layer (stratum basale), a spiny or spinous layer (stratum spinosum), a granular layer (stratum granulosum), and a corneous layer (stratum corneum).

### II. SKIN SUBSTITUTE PRODUCING A GROWTH FACTOR AND INSULIN

Provided herein is a skin substitute composed of a stratified epidermis in which cells therein produce, such as secrete, a growth factor and insulin. In some embodiments, the growth factor and insulin are recombinant sequences that are heterologous to cells of the stratified epidermis. In some embodiments, the skin substitute is composed of keratinocytes. In some embodiments, the skin substitute is composed of differentiated keratinocytes. In some embodiments, the skin substitute is composed of immortalized keratinocytes and/or differentiated immortalized keratinocytes.

In some embodiments, the recombinant growth factor and recombinant insulin are secretable from cells of the skin substitute. In some embodiments, the skin substitute is composed of a stratified epidermis comprising a basal layer, a spinous layer, a granular layer and a stratum corneum, wherein cells of the stratified epidermis express a recombinant growth factor and a recombinant insulin. In some embodiments, the cells of the stratified epidermis that express the recombinant growth factor and recombinant insulin comprise cells of the basal layer. In some embodiments the stratified epidermis is about 50 µm to about 300 µm in thickness. In some embodiments, the stratified epidermis is about 100 µm to about 250 µm thickness. In some embodiments, the stratified epidermis is about 100 µm to about 200 µm thick. In some embodiments, the stratified epidermis is at least or about 50 µm, 75 µm, 100 µm, 125 µm, 150 µm, 175 µm, 200 µm, 225 µm, 250 µm, 275 µm, or 300 µm thick, or has a thickness between any of the foregoing values.

### A. Growth Factor

In embodiments of the provided skin substitute, cells that compose the stratified epidermis (e.g. basal cells) produce and/or secrete a recombinant growth factor. Exemplary recombinant growth factors are described herein. In some embodiments, the recombinant growth factor is secretable from the stratified epidermis. In some embodiments, the recombinant growth factor is secretable from basal cells of the stratified epidermis. In some embodiments, cells that compose the stratified epidermis (e.g. basal cells) also produce and/or secrete a recombinant insulin, such as any described in Section B below.

Growth factors are known in the art. Growth factors include, for example, bone morphogenic protein (BMPs), epidermal growth factor (EGF), erythropoietin (EPO), fibroblast growth factor (FGF), granulocyte colony stimulating factor (G-CSF), granulocyte macrophage colony stimulating factor (GM-CSF), hepatocyte growth factor (HGF), insulin-like growth factor (IGF), platelet derived growth factor (PDGF), transforming growth factor α and β, and vascular endothelial growth factor (VEGF), and isoforms or alternative splice variants thereof.

In some embodiments, the growth factor is a vascular endothelial growth factor (VEGF)..

In some embodiments, the recombinant growth factor is encoded by a polynucleotide that encodes a growth factor sequence that contains a signal peptide to facilitate secretion of the growth factor. In some embodiments, the signal peptide is present in a precursor growth factor sequence and is cleaved to form the mature growth factor that is secretable. In some embodiments, the signal peptide is an endogenous or native signal peptide of the growth factor. In some embodiments, the signal peptide is a heterologous signal peptide that is from a different protein. In some embodiments, the signal peptide is cleaved when the growth factor is expressed from a cell of the skin substitute. In some embodiments, the secretable growth factor sequence lacks the signal peptide. In some embodiments, the growth factor is secretable from the cell. In some embodiments, the recombinant growth factor is secretable from the stratified epidermis. In some embodiments, cells of the stratified epidermis secrete the recombinant growth factor.

In some embodiments, the growth factor is a VEGF-A. VEGF-A is a key mediator of angiogenesis, signaling via the class IV tyrosine kinase receptor family of VEGF Receptors (VEGFRs). Although VEGF-A ligands bind to both VEGFR1 and VEGFR2, they primarily signal via VEGFR2 leading to endothelial cell proliferation, survival, migration and vascular permeability. Distinct VEGF-A isoforms result from alternative splicing.

Exemplary VEGF-A isoforms include, but are not limited to, VEGF 206 of vascular endothelial growth factor A (VEGF-A) polypeptide (SEQ ID NO: 11), isoform VEGF 189 of VEGF-A (SEQ ID NO: 19), isoform VEGF 183 of VEGF-A (SEQ ID NO: 20), isoform VEGF 148 of VEGF-A (SEQ ID NO: 21), isoform VEGF 145 of VEGF-A (SEQ ID NO: 22), isoform VEGF 165B of VEGF-A (SEQ ID NO: 23), isoform VEGF 121 of VEGF-A (SEQ ID NO: 24), isoform VEGF 111 of VEGF-A (SEQ ID NO: 25), isoform VEGF 165 of VEGF-A (SEQ ID NO: 7), isoform L-VEGF165 of VEGF-A (SEQ ID NO: 26), isoform L-VEGF 121 of VEGF-A (SEQ ID NO: 27), isoform L-VEGF 189 of VEGF-A (SEQ ID NO: 28), isoform L-VEGF 206 of VEGF-A (SEQ ID NO: 29), isoform 15 of VEGF-A (SEQ ID NO: 30), isoform 16 of VEGF-A (SEQ ID NO: 31) , isoform 17 of VEGF-A (SEQ ID NO: 32), or isoform 18 of VEGF-A (SEQ ID NO: 33). It is understood that also included are mature sequences thereof that lack a signal peptide following its cleavage when expressed and produced from a cell.

In some embodiments, cells that compose the stratified epidermis (e.g. basal cells) include a polynucleotide that encodes a recombinant human VEGF-A isoform that has at least at or about 80%, at least at or about 85%, at least at or about 90%, or at least at or about 95% sequence identity to the amino acid sequence set forth in any one of SEQ ID NOS: 7, 11 and 19-33, and retains binding to a VEGFR (e.g. VEGFR-2). In some embodiments, cells that compose the stratified epidermis (e.g. basal cells) include a polynucleotide that encodes a recombinant human VEGF-A isoform set forth in any one of SEQ ID NOS: 7, 11 and 19-33. In some embodiments, the polynucleotide encodes a protein containing a signal peptide, which is proteolytically cleaved and removed so that a protein lacking the signal peptide is secreted, such as via the constitutive secretory pathway. In some embodiments, cells of the stratified epidermis of the skin substitute produce, e.g., secrete, the recombinant VEGF-A. In some embodiments, basal cells of the stratified epidermis of the skin substitute produce, e.g., secrete, the recombinant human VEGF-A isoform.

In some embodiments, cells that compose the stratified epidermis (e.g. basal cells) comprise a VEGF-A isoform that has an amino acid sequence that has at least at or about 80%, at least at or about 85%, at least at or about 90%, or at least at or about 95% sequence identity to the amino acid sequence set forth in any one of SEQ ID NOS: 7, 11 and 19-33, and retains binding to a VEGFR (e.g. VEGFR-2). In some embodiments, cells that compose the stratified epidermis (e.g. basal cells) comprise a recombinant VEGF-A isoform set forth in any one of SEQ ID NOS: 7, 11 and 19-33. In some embodiments, the protein lacks the signal peptide, which is proteolytically cleaved and removed so that the encoded protein lacks the signal peptide set forth in any one of SEQ ID NOS: 7, 11 and 19-33 (e.g. lacks amino acid residues 1-26). In some embodiments, the recombinant human VEGF-A is secreted, such as via the constitutive secretory pathway. In some embodiments, cells of the stratified epidermis of the skin substitute produce, e.g., secrete, the recombinant VEGF-A. In some embodiments, basal cells of the stratified epidermis of the skin substitute produce, e.g., secrete, the recombinant human VEGF-A isoform.
In some embodiments, the recombinant human VEGF-A is encoded by a polynucleotide comprising a sequence that has at least at or about 80%, at least at or about 85%, at least at or about 90%, or at least at or about 95% sequence identity to the sequence set forth in SEQ ID NO: 4. In some embodiments, the recombinant human VEGF-A is encoded by a polynucleotide comprising the sequence set forth in SEQ ID NO: 4. In some embodiments, the recombinant human VEGF-A is encoded by a polynucleotide set forth in SEQ ID NO: 4. In some embodiments, the recombinant human VEGF-A comprises a sequence that has at least at or about 80%, at least at or about 85%, at least at or about 90%, or at least at or about 95% sequence identity to the sequence set forth in SEQ ID NO: 7, or a sequence thereof that lacks the signal peptide. In some embodiments, the recombinant human VEGF-A comprises the sequence set forth in SEQ ID NO: 7 or a sequence thereof that lacks the signal peptide. In some embodiments, the recombinant human VEGF-A is set forth in SEQ ID NO: 7 or a sequence thereof that lacks the signal peptide. In some embodiments, cells of the stratified epidermis of the skin substitute produce, e.g., secrete, the recombinant human VEGF-A. In some embodiments, basal cells of the stratified epidermis of the skin substitute produce, e.g., secrete, the recombinant human VEGF-A.

In some embodiments, the recombinant VEGF is encoded by a polynucleotide that encodes a growth factor sequence that contains a signal peptide to facilitate secretion of the VEGF. In some embodiments, the signal peptide is present in a precursor growth factor sequence and is cleaved to form the mature growth factor that is secretable. In some embodiments, the signal peptide is an endogenous or native signal peptide of the growth factor. In some embodiments, the signal peptide is a heterologous signal peptide that is from a different protein. In some embodiments the signal peptide is the sequence set forth as MNFLLSWVHWSLALLLYLHHAKWSQA (SEQ ID NO: 45). In some embodiments, the signal peptide is cleaved when the VEGF is expressed from a cell of the skin substitute. In some embodiments, the secretable VEGF sequence lacks the signal peptide. In some embodiments, the recombinant human VEGF-A comprises a sequence that has at least at or about 80%, at least at or about 85%, at least at or about 90%, or at least at or about 95% sequence identity to the sequence set forth in SEQ ID NO: 44. In some embodiments, the recombinant human VEGF-A comprises the sequence set forth in SEQ ID NO: 44. In some embodiments, the recombinant human VEGF-A is set forth in SEQ ID NO: 44. In some embodiments, the VEGF is secretable from the cell. In some embodiments, the recombinant VEGF is secretable from the stratified epidermis. In some embodiments, cells of the stratified epidermis secrete the recombinant VEGF.

In some embodiments, the growth factor is a member of the VEGF family of proteins. In some embodiments, the growth factor is a vascular endothelial growth factor B (VEGF-B) polypeptide (e.g. SEQ ID NO: 12), or a c-fos induced growth factor (FIGF) polypeptide (also referred to as VEGF-D) (e.g. SEQ ID NO: 13).

In some embodiments, cells that compose the stratified epidermis (e.g. basal cells) comprise a recombinant human growth factor that has an amino acid sequence that has at least at or about 80%, at least at or about 85%, at least at or about 90%, or at least at or about 95% sequence identity to the amino acid sequence set forth in any one of SEQ ID NOS: 12-13. In some embodiments, cells that compose the stratified epidermis (e.g. basal cells) comprise a recombinant human growth set forth in any one of SEQ ID NOS: 12-13. In some embodiments, cells that compose the stratified epidermis (e.g. basal cells) comprise a recombinant human growth set forth in any one of SEQ ID NOS: 12-13. In some embodiments, the protein lacks the signal peptide, which is proteolytically cleaved and removed so that the encoded protein lacks the signal peptide set forth in any one of SEQ ID NOS: 12-13 (see e.g. Sequence Table). In some embodiments, the recombinant human growth factor is secreted, such as via the constitutive secretory pathway. In some embodiments, cells of the stratified epidermis of the skin substitute produce, e.g., secrete, the recombinant human growth factor. In some embodiments, basal cells of the stratified epidermis of the skin substitute produce, e.g., secrete, the recombinant human growth factor.

### B. Insulin

In embodiments of the provided skin substitute, cells that compose the stratified epidermis (e.g. basal cells) produce and/or secrete a recombinant insulin. Exemplary recombinant insulins are described herein. In some embodiments, the recombinant insulin is secretable from the stratified epidermis. In some embodiments, recombinant insulin is secretable from basal cells of the stratified epidermis. In some embodiments, cells that compose the stratified epidermis (e.g. basal cells) also produce and/or secrete a recombinant growth factor, such as any described in Section A above.

Insulin is a hormone that controls glucose levels. Depending on route of administration and dose, insulin can be systemically or locally available. In one example, systemic insulin is used as a therapeutic for glycemic control, such as in diabetic patients. In another example, local insulin activity does not affect systemic glucose levels. In some embodiments, cells of the skin substitute provided herein produce insulin at a level that impacts local glucose levels. In some embodiments, cells of the skin substitute provided herein produce insulin at a level that does not impact systemic glucose levels.

Insulin is produced as a preproprotein that, when expressed in a cell, is processed into a two chain form. Typically, human insulin is translated as a 110 amino acid precursor polypeptide, preproinsulin, containing a 24 amino acid signal peptide that directs the protein to the endoplasmic reticulum (ER) wherein the signal sequence is cleaved, resulting in proinsulin (SEQ ID NO: 5). Proinsulin is processed further to release the 31 amino acid C-peptide, or connecting chain peptide. For wild-type insulin, proinsulin is coordinately cleaved by endopeptidases (e.g. PC-2 and PC-3 endopeptidase) on the carboxylic side of two sites, Arg31Arg32 (B chain/C chain peptide junction) and Lys64Arg65 (C chain/A chain peptide junction) on human proinsulin, to generate the A and B chains of mature insulin, C-peptide, and free basic amino acids. For instance, for wild-type human insulin, the resulting insulin contains a 21 amino acid A-chain (corresponding to amino acid residues 66 to 86 of the proinsulin polypeptide set forth in SEQ ID NO: 5) set forth in SEQ ID NO: 36 and a 30 amino acid B-chain (corresponding to amino acid residues 1 to 30 of the proinsulin polypeptide set forth in SEQ ID NO: 5) set forth in SEQ ID NO: 40, which are cross-linked by disulfide bonds. Typically, a properly cross-linked human insulin contains three disulfide bridges: one between position 7 of the A-chain and position 7 of the B-chain, a second between position 20 of the A-chain and position 19 of the B-chain, and a third between positions 6 and 11 of the A-chain.

In some embodiments, the recombinant insulin is encoded by a polynucleotide that encodes a proinsulin polypeptide to result in insulin polypeptides in single-chain or two-chain forms. In some embodiments, the insulin is a single-chain polypeptide containing the A-chain and B-chain. In some embodiments, the encoded insulin is able to be processed in keratinocytes into a two-chain form containing an A-chain and a B-chain that are linked, such as by disulfide bonds. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain form containing the A-chain and B-chain is secretable from cells of the stratified epidermis (e.g. basal cells).

In some embodiments, the recombinant insulin is a regular insulin that is a native or wild type insulin polypeptide. These include recombinant forms of human insulin, as well as insulins from bovine, porcine and other species. In some embodiments, the recombinant insulin is a recombinant insulin of a regular human insulin marketed as Humulin^{®} R, Novolin^{®} R and Velosulin^{®}. In some embodiments, the recombinant insulin is a recombinant insulin of a regular porcine insulin marketed as Iletin II^{®}.

In some embodiments, the insulin is a recombinant human insulin. In some embodiments, cells that compose the stratified epidermis (e.g. basal cells) include a polynucleotide that encodes a proinsulin precursor form of the insulin. In some embodiments, the precursor of human insulin is human proinsulin. In some embodiments, cells that compose the stratified epidermis (e.g. basal cells) include a polynucleotide that includes a human proinsulin amino acid sequence that has at least at or about 80%, at least at or about 85%, at least at or about 90%, or at least at or about 95% sequence identity to the amino acid sequence set forth in SEQ ID NO: 5. In some embodiments, cells that compose the stratified epidermis (e.g. basal cells) comprise a human proinsulin amino acid sequence that has at least at or about 80%, at least at or about 85%, at least at or about 90%, or at least at or about 95% sequence identity to the amino acid sequence set forth in SEQ ID NO: 5. In some embodiments, the encoded proinsulin is able to be processed in keratinocytes into a two-chain form containing an A-chain and a B-chain that are linked, such as by disulfide bonds. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain form containing the A-chain and B-chain is secretable from cells of the stratified epidermis (e.g. basal cells).

In some embodiments, cells that compose the stratified epidermis (e.g. basal cells) include a polynucleotide that encodes the human proinsulin set forth in SEQ ID NO: 5. In some embodiments, cells that compose the stratified epidermis (e.g. basal cells) comprise the human proinsulin set forth in SEQ ID NO: 5. In some embodiments, the insulin is a single chain polypeptide. In some embodiments, the proinsulin form is processed to a two chain form containing an A chain and a B chain. In some embodiments, a two-chain recombinant form of insulin that contains an A chain and a B chain of SEQ ID NO: 5 is secretable from cells that compose the stratified epidermis (e.g. basal cells). In some embodiments, the encoded insulin is processed to a two chain form containing the A chain set forth in SEQ ID NO: 36 and the B chain set forth in SEQ ID NO: 40. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain recombinant form of insulin that contains the A chain set forth in SEQ ID NO: 36 and the B chain set forth in SEQ ID NO: 40 is secretable from cells that compose the stratified epidermis (e.g. basal cells).

In some embodiments, the recombinant insulin is a variant of a human insulin, such as a functional variant or species or allelic variant, or is a truncated form of human insulin that has activity. In some embodiments, variants of insulin, including allelic and species variants, variants encoded by splice variants and other functional variants, such as insulin analogs or other derivatized or modified forms, including polypeptides that have at least 40%, 45%, 50%, 55%, 60%, 65%, 70%, 75%, 80%, 85%, 90%, 95%, 96%, 97%, 98%, 99% or more sequence identity to human insulin set forth in SEQ ID NO: 5 or to a processed insulin thereof that contains an A and B chain, so long as the insulin binds to the human insulin receptor to initiate a signaling cascade that results in an increase of glucose uptake and storage and/or a decrease of endogenous glucose production. In some embodiments, the encoded proinsulin is able to be processed in keratinocytes into a two-chain form containing an A-chain and a B-chain that are linked, such as by disulfide bonds. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain form containing the A-chain and B-chain is secretable from cells of the stratified epidermis (e.g. basal cells).

For example, a recombinant insulin may include species variants of human insulin. These include, but are not limited to, insulins derived from bovine and porcine. Bovine insulin differs from human insulin at amino acids 8 and 10 of the A chain and amino acid 30 of the B chain (SEQ ID NO: 17). Porcine insulin only differs from human insulin at amino acid 30 in the B chain where, like the bovine sequence, there is an alanine substitution in place of threonine (SEQ ID NO: 18). In some embodiments, cells that compose the stratified epidermis (e.g. basal cells) include a polynucleotide that encodes a proinsulin precursor form of a bovine or porcine insulin, such as a proinsulin form of SEQ ID NO: 17 (e.g. amino acids 25-105 of SEQ ID NO: 17) or a proinsulin form of SEQ ID NO: 18 (e.g. amino acids 25-105 of SEQ ID NO: 18), or a sequence that has at least 40%, 45%, 50%, 55%, 60%, 65%, 70%, 75%, 80%, 85%, 90%, 95%, 96%, 97%, 98%, 99% or more sequence identity to insulin set forth in SEQ ID NO: 17 or SEQ ID NO: 18 or to a processed insulin thereof that contains an A and B chain, so long as the insulin binds to the human insulin receptor to initiate a signaling cascade that results in an increase of glucose uptake and storage and/or a decrease of endogenous glucose production. In some embodiments, the B chain corresponds to amino acids 25-54 of SEQ ID NO: 17 or SEQ ID NO: 18 and the A chain corresponds to amino acids 85-105 of SEQ ID NO: 17 or SEQ ID NO: 18. In some embodiments, the encoded insulin is a single chain polypeptide containing an A chain and a B chain set forth in SEQ ID NO: 17 or SEQ ID NO: 18. In some embodiments, the encoded proinsulin is able to be processed in keratinocytes into a two-chain form containing an A-chain and a B-chain that are linked, such as by disulfide bonds. In some embodiments, the encoded insulin is processed to a two chain form containing an A chain and a B chain set forth in SEQ ID NO: 17 or SEQ ID NO: 18. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain recombinant form of insulin that contains an A chain and a B chain of SEQ ID NO: 17 or SEQ ID NO: 18 is secretable from cells that compose the stratified epidermis (e.g. basal cells).

Also included among variants of insulin are insulin analogs that contain one or more amino acid modifications compared to a human insulin. Exemplary insulin analogs (A and B chains) include fast-acting and longer-acting analog forms or superactive insulins (see e.g. Vajo et al. 2001 Endocrine Reviews 22:706-717). Fast-Acting insulin analogs are modified forms of insulin that typically contain one or more amino acid changes. The analogs are designed to reduce the self-association of the insulin molecule for the purpose of increasing the absorption rate and onset of action as compared to regular insulin. For example, insulin analogs include, but are not limited to, glulisine (LysB3, GluB29), HMR-1 153 (LysB3, IleB28), HMR-1423 (GlyA21, HisB32), insulin aspart (AspB28), insulin lispro (LysB28, ProB29) and AspB10. In every instance above, the nomenclature of the analogs is based on a description of the amino acid substitution at specific positions on the A or B chain of insulin, numbered from the N-terminus of the chain, in which the remainder of the sequence is that of natural human insulin.

In some embodiments, the recombinant insulin is insulin AspB10. Insulin AspB10 is a human insulin analog polypeptide containing a single amino acid change of the B-chain resulting in the substitution of aspartic acid (D) for the naturally occurring histidine (H) at position 10 in wild-type insulin (e.g. substation of H to D). The result of the substitution is a superactive insulin that is absorbed twice as rapidly as regular insulin (e.g. wild type human insulin). In some aspects, insulin AspB10 has increased binding affinity to the insulin receptor as compared to regular insulin (e.g. wild type human insulin). The sequence of the A chain of insulin AspB10 is set forth in SEQ ID NO: 36 and the B-chain is set forth in SEQ ID NO: 41. In some embodiments, cells that compose the stratified epidermis (e.g. basal cells) include a polynucleotide that encodes a proinsulin precursor form of insulin AspB 10 containing the A chain set forth in SEQ ID NO: 36 and the B chain set forth in SEQ ID NO: 41. In some embodiments, the encoded proinsulin is able to be processed in keratinocytes into a two-chain form containing an A-chain and a B-chain that are linked, such as by disulfide bonds. In some embodiments, the encoded insulin is processed to a two chain form containing the A chain set forth in SEQ ID NO: 36 and the B chain set forth in SEQ ID NO: 41. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain recombinant form of insulin that contains the A chain set forth in SEQ ID NO: 36 and the B chain set forth in SEQ ID NO: 41 is secretable from cells that compose the stratified epidermis (e.g. basal cells).

In some embodiments, the recombinant insulin is insulin glargine. By virtue of the addition of two arginines to the C-terminus of the B-chain, the isoelectric point of the glargine insulin is shifted making it more soluble at an acidic pH. An additional amino acid change exists in the A chain (N21G) to prevent deamidation and dimerization resulting from an acid-sensitive asparagine. The sequence of the A chain of glargine insulin is set forth in SEQ ID NO: 34 and the B-chain is set forth in SEQ ID NO: 35. In some embodiments, cells that compose the stratified epidermis (e.g. basal cells) include a polynucleotide that encodes a proinsulin precursor form of insulin glargine containing the A chain set forth in SEQ ID NO: 34 and the B chain set forth in SEQ ID NO: 35. In some embodiments, the encoded proinsulin is able to be processed in keratinocytes into a two-chain form containing an A-chain and a B-chain that are linked, such as by disulfide bonds. In some embodiments, the encoded insulin is processed to a two chain form containing the A chain set forth in SEQ ID NO: 34 and the B chain set forth in SEQ ID NO: 35. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain recombinant form of insulin that contains the A chain set forth in SEQ ID NO: 34 and the B chain set forth in SEQ ID NO: 35 is secretable from cells that compose the stratified epidermis (e.g. basal cells).

In some embodiments, the recombinant insulin is insulin Lispro. Human insulin Lispro is an insulin polypeptide formulation containing amino acid changes at position 28 and 29 of the B-chain such that the Pro-Lys at this position in wild-type insulin is inverted to Lys-Pro. The result of the inversion of these two amino acids is a polypeptide with a decreased propensity to self-associate, which allows for a more rapid onset of action. Specifically, the sequence inversion in the B-chain results in the elimination of two hydrophobic interactions and weakening of two beta-pleated sheet hydrogen bonds that stabilize the dimer (DeFelippis et al., Insulin Chemistry and Pharmacokinetics. In Ellenberg and Rifkin's Diabetes Mellitus 2002 pp. 481-500, McGraw-Hill Professional). Due to the amino acid modification, insulin Lispro is more rapidly acting than regular insulin. The sequence of the A chain of insulin Lispro is set forth in SEQ ID NO: 36 and the B-chain is set forth in SEQ ID NO: 37. In some embodiments, cells that compose the stratified epidermis (e.g. basal cells) include a polynucleotide that encodes a proinsulin precursor form of insulin Lispro containing the A chain set forth in SEQ ID NO: 36 and the B chain set forth in SEQ ID NO: 37. In some embodiments, the encoded proinsulin is able to be processed in keratinocytes into a two-chain form containing an A-chain and a B-chain that are linked, such as by disulfide bonds. In some embodiments, the encoded insulin is processed to a two chain form containing the A chain set forth in SEQ ID NO: 36 and the B chain set forth in SEQ ID NO: 37. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain recombinant form of insulin that contains the A chain set forth in SEQ ID NO: 36 and the B chain set forth in SEQ ID NO: 37 is secretable from cells that compose the stratified epidermis (e.g. basal cells).

In some embodiments, the recombinant insulin is insulin aspart. Human insulin aspart is an insulin polypeptide formulation containing an amino acid substitution at position 28 of the B-chain of human insulin from a proline to an aspartic acid. The modification in insulin aspart confers a negatively-charged side-chain carboxyl group to create charge repulsion and destabilize the monomer-monomer interaction. Further, the removal of the proline eliminates a key hydrophobic interaction between monomers (DeFelippis et al., Insulin Chemistry and Pharmacokinetics. In Ellenberg and Rifkin's Diabetes Mellitus 2002 pp. 481-500, McGraw-Hill Professional). The sequence of the A chain of insulin aspart is set forth in SEQ ID NO: 36 and the B-chain is set forth in SEQ ID NO: 38. In some embodiments, cells that compose the stratified epidermis (e.g. basal cells) include a polynucleotide that encodes a proinsulin precursor form of insulin aspart containing the A chain set forth in SEQ ID NO: 36 and the B chain set forth in SEQ ID NO: 38. In some embodiments, the encoded proinsulin is able to be processed in keratinocytes into a two-chain form containing an A-chain and a B-chain that are linked, such as by disulfide bonds. In some embodiments, the encoded insulin is processed to a two chain form containing the A chain set forth in SEQ ID NO: 36 and the B chain set forth in SEQ ID NO: 38. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain recombinant form of insulin that contains the A chain set forth in SEQ ID NO: 36 and the B chain set forth in SEQ ID NO: 38 is secretable from cells that compose the stratified epidermis (e.g. basal cells).

In some embodiments, the recombinant insulin is insulin glulisine. Human insulin glulisine is an insulin polypeptide formulation containing an amino acid substitution in the B-chain at position B3 from asparagine to lysine and at amino acid B29 from lysine to glutamic acid compared to the sequence of the B-chain of human insulin. The modifications render the polypeptide molecule less prone to self-association compared to human insulin. The sequence of the A chain of insulin glulisine is set forth in SEQ ID NO: 36 and the B-chain is set forth in SEQ ID NO: 39. In some embodiments, cells that compose the stratified epidermis (e.g. basal cells) include a polynucleotide that encodes a proinsulin precursor form of insulin glulisine containing the A chain set forth in SEQ ID NO: 36 and the B chain set forth in SEQ ID NO: 39. In some embodiments, the encoded proinsulin is able to be processed in keratinocytes into a two-chain form containing an A-chain and a B-chain that are linked, such as by disulfide bonds. In some embodiments, the encoded insulin is processed to a two chain form containing the A chain set forth in SEQ ID NO: 36 and the B chain set forth in SEQ ID NO: 39. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain recombinant form of insulin that contains the A chain set forth in SEQ ID NO: 36 and the B chain set forth in SEQ ID NO: 39 is secretable from cells that compose the stratified epidermis (e.g. basal cells).

In some embodiments, the proinsulin form of insulin is modified to promote cleavage of the proinsulin into a two chain form containing the A chain and the B chain. In some cases, human keratinocytes, e.g., HaCaT cells, lack the enzymes necessary to efficiently cleave proinsulin for the production of mature insulin. For instance, the endopeptidases, such as PC-2 and PC-3 are not present or are not present at high enough levels for cleavage of the insulin. Instead, keratinocytes express furin, a calcium-dependent cleavage enzyme that belongs to the subtilisin-like proprotein convertase family of enzymes. In some embodiments, the human proinsulin is a modified human proinsulin. In some embodiments, the modified human proinsulin comprises a sequence that is recognized by an enzyme, e.g., a protease, that is expressed in keratinocytes, e.g., HaCaT cells, which allows the encoded proinsulin to be able to be processed in keratinocytes into a two-chain form containing an A-chain and a B-chain that are linked, such as by disulfide bonds. In some embodiments, the protease is furin and the modified human proinsulin comprises at least one furin recognition sequence. In some embodiments, the modified human proinsulin comprises two furin recognition sequence introduced in place of the sequence containing the Arg31-Arg32 cleavage site (B-C junction) and Lys64-Arg65 cleavage site (C-A junction). In some embodiments, the at least one furin recognition sequence comprises the consensus sequence R-X-R-R, where X is any amino acid (SEQ ID NO: 8) or R-X-K-R, where X is any amino acid (SEQ ID NO: 9). In some embodiments, the furin cleavage site is RTKR (SEQ ID NO: 10). In some embodiments, the furin cleavage site is RQKR (SEQ ID NO: 42).

In some embodiments, the proinsulin is an AspB 10 insulin containing an A chain set forth in SEQ ID NO: 36 and a B chain set forth in SEQ ID NO: 41 in which the proinsulin further contains two furin recognition sequences. In some embodiments, each of the furin recognition sequence comprises the consensus sequence R-X-R-R, where X is any amino acid (SEQ ID NO: 8) or R-X-K-R, where X is any amino acid (SEQ ID NO: 9). In some embodiments, one of the furin cleavage site is RTKR (SEQ ID NO: 10). In some embodiments, one of the furin cleavage site is RQKR (SEQ ID NO: 42). In some embodiments, the modified human proinsulin comprises a sequence that has at least at or about 80%, at least at or about 85%, at least at or about 90%, or at least at or about 95% sequence identity to the amino acid sequence set forth in SEQ ID NO: 6, in which the proinsulin contains the furin recognition sites and the amino acid substitution of Asp at position 10 of the B chain. In some embodiments, the modified human proinsulin comprises the amino acid sequence set forth in SEQ ID NO: 6. In some embodiments, the modified human proinsulin is set forth in SEQ ID NO: 6. In some embodiments, the encoded insulin is processed to a two chain form containing the A chain set forth in SEQ ID NO: 36 and the B chain set forth in SEQ ID NO: 41. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain recombinant form of insulin that contains the A chain set forth in SEQ ID NO: 36 and the B chain set forth in SEQ ID NO: 41 is secretable from cells that compose the stratified epidermis (e.g. basal cells).

In some of any of the provided embodiments, the encoding polynucleotide that encodes a proinsulin is a preproinsulin that further contains a signal peptide to facilitate secretion of the growth factor. In some embodiments, the signal peptide is cleaved from the encoded preproinsulin to form the mature proinsulin that is secretable. In some embodiments, the signal peptide is cleaved when the insulin is expressed from a cell of the skin substitute. In some embodiments, the mature proinsulin form is further processed into a recombinant insulin that is a two chain form containing an A and B chain as described. In some embodiments, the signal peptide is an endogenous or native signal peptide of insulin. In some embodiments, the signal peptide is a heterologous signal peptide that is from a different protein. In some embodiments, the sequence encodes the signal peptide MALWMRLLPLLALLALWGPDPAAA (SEQ ID NO: 43). In some embodiments, the recombinant insulin is secretable from the cell. In some embodiments, the secretable recombinant insulin sequence lacks the signal peptide.

In some embodiments, the recombinant human insulin is encoded by a polynucleotide comprising a sequence that has at least at or about 80%, at least at or about 85%, at least at or about 90%, or at least at or about 95% sequence identity to the sequence set forth in SEQ ID NO: 2, in which the encoded proinsulin contains the furin recognition sites and the amino acid substitution of aspartic acid (Asp, D) at position 10 of the B chain. In some embodiments, the recombinant human insulin is encoded by a polynucleotide comprising the sequence set forth in SEQ ID NO: 2. In some embodiments, the recombinant human insulin is encoded by a polynucleotide set forth in SEQ ID NO: 2. In some embodiments, cells of the stratified epidermis of the skin substitute produce, e.g., secrete, the recombinant insulin. In some embodiments, basal cells of the stratified epidermis of the skin substitute produce, e.g., secrete, the recombinant human insulin. In some embodiments, a two-chain recombinant form of insulin that contains the A chain set forth in SEQ ID NO: 36 and the B chain set forth in SEQ ID NO: 41 is secretable from cells that compose the stratified epidermis (e.g. basal cells).

### C. Exemplary Features of the Skin Substitute

In some embodiments, cells of the stratified epidermis of the skin substitute produce, e.g., secrete, a recombinant human growth factor (e.g. any described in Section II. A) and a recombinant human insulin (e.g. any described in Section II. B), such as, a two-chain insulin form containing the A-chain and B-chain. In some embodiments, basal cells of the stratified epidermis of the skin substitute produce, e.g., secrete, a recombinant human growth factor (e.g. any described in Section II. A) and a recombinant human insulin (e.g. any described in Section II. B), such as, a two chain insulin form containing the A- chain and B-chain.

In some embodiments, the recombinant human growth factor is a VEGF and is encoded by a polynucleotide set forth in SEQ ID NO: 4 and the recombinant human insulin is encoded by a polynucleotide set forth in SEQ ID NO: 2. In some embodiments, the recombinant human growth factor is a VEGF and is set forth in SEQ ID NO: 7 and the recombinant human insulin is set forth in SEQ ID NO: 6 or is a two-chain form thereof that contains an A-chain and a B-chain, such as linked by a disulfide bond. In some embodiments, cells of the stratified epidermis of the skin substitute produce, e.g., secrete, the recombinant VEGF and the recombinant insulin. In some embodiments, basal cells of the stratified epidermis of the skin substitute produce, e.g., secrete, the recombinant human VEGF and the recombinant human insulin. In some embodiments, a VEGF set forth in SEQ ID NO: 7 that lacks amino acid residues 1-26 thereof and a recombinant human insulin that is a two-chain recombinant form of insulin that contains the A chain set forth in SEQ ID NO: 36 and the B chain set forth in SEQ ID NO:41 is secretable from cells that compose the stratified epidermis (e.g. basal cells). In some embodiments, a VEGF set forth in SEQ ID NO: 7 that lacks amino acid residues 1-26 thereof and a recombinant human insulin that is a two-chain recombinant form of insulin that contains the A chain set forth in SEQ ID NO: 36 and the B chain set forth in SEQ ID NO: 40 is secretable from cells that compose the stratified epidermis (e.g. basal cells).

In some aspects, provided herein is a skin substitute that produces, e.g., secretes, a recombinant growth factor and a recombinant insulin at levels that result in significantly greater improvement in one or more marker(s) of angiogenic reorganization relative to either the growth factor or insulin alone, as evaluated in a tube formation assay. An in vitro tube formation assay can provide insight into angiogenesis, the development of new blood vessels from pre-existing vessels (DiCicco-Skinner, J Vis Exp. 2014; (91): 51312). Angiogenesis is a crucial component of various processes, including organ growth, embryonic development, and wound healing. Markers of angiogenesis or of angiogenic reorganization that can be evaluated in a tube formation assay include, but are not limited to presence of, or relative increases of, nodes, webs, and main segments. Nodes, also referred to as unions, can be defined as the bond sites of at least three chords, i.e. connections. Webs can be defined as a closed circuit surrounded by two or more nodes. Main segments can be defined as chords that bond two nodes together.

In some aspects, provided herein is a skin substitute comprising a stratified epidermis, wherein cells of the stratified epidermis continuously secrete a quantifiable level of the recombinant growth factor and the recombinant insulin. In some embodiments, cells of the stratified epidermis continuously secrete the recombinant growth factor and the recombinant insulin for up to or about 2 days, up to or about 3 days, up to or about 4 days, up to or about 5 days, up to or about 6 days, up to or about 7 days, up to or about 8 days, up to or about 9 days, up to or about 10 days, up to or about 11 days, up to or about 12 days, up to or about 13 days, or up to or about 14 days. In some embodiments, cells of the stratified epidermis continuously secrete the recombinant growth factor and the recombinant insulin for up to or about one week, up to or about two weeks, up to or about three weeks, up to or about one to two weeks, or up to or about two to three weeks.

In some embodiments, cells of the stratified epidermis secrete a quantifiable level of the recombinant growth factor and insulin and/or C-peptide. Cleaved from proinsulin, C-peptide is a byproduct of insulin production that is also secreted from cells. C-peptide is produced in equimolar amounts relative to endogenous insulin. It is widely used as a measure of pancreatic beta cell function and to guide the diagnosis and management of diabetes (Leighton et al., Diabetes Ther. 2017; 8(3):475-487).

Methods of detecting and quantifying a growth factor and insulin and/or C-peptide are known in the art. In one example, in vitro detection of a growth factor and insulin and/or C-peptide can include use of an enzyme-linked immunosorbent assay (ELISA). ELISA kits for the detection of C-peptide include, for example, KT-881 C-Peptide ELISA Kit from Epitope Diagnostics, Inc. and ab178641 C-Peptide ELISA Kit from Abcam. Alternatively, gene expression can be quantitated by PCR, e.g., RT-qPCR. A quantifiable level can be defined as any level that is greater than or equal to the limit of quantitation of a particular assay.

In some aspects, provided herein is a skin substitute that produces, e.g., secretes, a recombinant growth factor and a recombinant insulin at levels that reduce advanced glycation end products (AGEs) in the skin of a subject. In some embodiments, the subject is human. AGEs are proteins or lipids that become glycated after exposure to sugars. The accumulation of AGEs, such as in the skin, may interfere with the structure and/or function of cells (Goldin et al., Circulation. 2006;114:597-605). Methods of detecting AGEs are known in the art. In some examples, AGE complexes can be detected using ELISA (see e.g., abx054078, Advanced Glycation End Product (AGE) ELISA Kit, Abexxa and STA-817, OxiSelect^{™} Advanced Glycation End Product (AGE) Competitive ELISA Kit, Cell Biolabs, Inc.), spectrofluorimetric analysis (see e.g., Villa et al., Metabolism 2017;71:64-69), chromatographic, colorimetric, spectroscopic, mass spectrometric, and serological methods (Perrone et al., Oxidative Medicine and Cellular Longevity 2020; vol. 2020, Article ID 3818196, 18 pages).

### III.METHODS OF PREPARING A SKIN SUBSTITUTE

Provided herein are methods of producing a skin substitute composed of a stratified epidermis, wherein the cells of the stratified epidermis produce a growth factor and insulin. In some embodiments, the method comprises the following steps: 1) differentiating keratinocytes into a stratified epidermis, wherein the stratified epidermis comprises a basal layer, a spinous layer, a granular layer and a stratum corneum and 2) introducing a polynucleotide to the stratified epidermis to produce a skin substitute, wherein the skin substitute produces and secretes a growth factor and insulin. In some embodiments, step 1) of the method comprises differentiating immortalized keratinocytes, and the introducing of a polynucleotide in step 2) comprises transduction with a viral vector.

In some embodiments, methods for producing a skin substitute may include: (1) a system in which cultured keratinocytes have been reconstructed into a 3D system to represent human epidermis; (2) a system where keratinocytes (primary or immortalized cells) are cultured in 3D on a substrate; (3) a system in which cultured skin cells have been reconstructed into a 3D system to represent human skin; and/or (4) a system where keratinocytes (primary or immortalized cells) are cultured above a matrix, e.g., a dermal matrix.

In some embodiments, the cells are differentiated so that cells of the stratified epidermis express a tight junction protein, such as occluding or claudin. Proteins of the epidermis contribute to its role as a permeability barrier. For example, tight junctions and desmosomes contribute to barrier-like function of the epidermis. Tight junctions are a multi-protein network that form intercellular connections between cells of the mammalian epidermis. Strands of the tight junction comprise occludin and claudins, a multigene family of proteins. Occludin is an integral plasma-membrane protein that localizes at tight junctions. Claudins, e.g., claudin-1, claudin-2, and claudin-4, are integral membrane proteins. Claudins form the backbone of tight junction strands, and occludin is copolymerized into these strands (Furuse et al., J. Cell Biol. 1999;147(4):891-903). Desmosomes are adhesive protein complexes that localize to intercellular junctions and are responsible for maintaining the mechanical integrity of tissues. Desmosomal cadherins have been shown to act as an attachment receptor for certain types of adenoviruses. For example, adenovirus serotypes Ad3, Ad7, Ad11, and Ad14, but not Ad2 or Ad5, were found to interact with desmoglein 2 (Wang et al., Nat Med. 2011;17(1): 96-104.).

Culture conditions influence the localization and amount of proteins that determine epithelial permeability. For example, growing HaCaT cells to confluent culture has been shown to promote desmosomal hyper-adhesiveness that results in enhanced cell sheet integrity and reduced permeability (Kimura et al., J Invest Dermatol 2007;127, 775-781). Regarding calcium levels in culture, increasing levels of calcium, e.g., from 0.1 mM to 2 mM, has been shown to induce desmosome formation and stratification in human keratinocytes (Watt et al., J Cell Biol. 1984; 99(6):2211-2215). Low or depleted calcium levels, e.g., 0.03 mM Ca²⁺, have been associated with tight junction dissociation and increased permeability. In contrast, exposing normal human keratinocytes to high levels of calcium has been shown to induce stratification and improve barrier function, i.e., reduce permeability. In response to elevated calcium levels (1.8 mM Ca²⁺), increased localization of claudin-1, claudin-4, and occludin were detectable at cell borders and in underlying epidermal layers, likely contributing to enhanced transepithelial electric resistance (Yuki et al., Exp Dermatol. 2007;16(4):324-30).

### A. Differentiation and Culture of Immortalized Keratinocytes

Examples of immortalized keratinocyte cell lines include, but are not limited to HaCaT (Boukamp et al., J Cell Biol. 1988;106:761-771), NM1 (Baden et al., In Vitro Cell Dev Biol. 1987;23:205-213), and NIKS (Allen-Hoffmann et al., J Invest Dermatol. 2000;114:444-455). As described herein, reference to "keratinocytes" includes reference to immortalized keratinocytes. Keratinocytes can be differentiated into a stratified epidermis composed of four morphologically and biochemically distinct layers (basal, spinous, granular layers, and a stratum corneum). Keratinocyte growth and differentiation can be influenced by a variety of factors, including calcium levels in culture, cell density, and temperature. For example, high levels or concentrations of calcium can induce differentiation in HaCaT cells, as can high cell density. One major difference between primary and immortalized keratinocytes, e.g., HaCaT cells, is that differentiated immortalized cells retain their proliferative capacity, but primary keratinocytes stop dividing. Thus, differentiated immortalized keratinocytes, like HaCaT cells, can be propagated indefinitely under particular conditions (Wilson, Methods Mol Biol. 2014;1195:33-41).

### 1. Structural Features and Biochemical Components of a Stratified Epidermis

Keratinocyte morphology can serve as a distinguishing factor between undifferentiated and differentiated cells. A stratified epidermis can be identified and confirmed using microscopy. A stratified epidermis is visually distinct from a simple epithelium, which comprises only one layer of cells. A stratified epidermis is also visually distinct from a pseudostratified epithelium, which comprises a single elongated layer that extends to the basolateral surface of the epithelium. Methods of visually evaluating a skin substitute comprising a stratified epidermis are known to one of skill in the art. For example, electron microscopy can be used to visualize a stratified epithelium. In some examples, scanning electron microscopy can be used to visualize a stratified epithelium.

Detection of certain proteins, e.g., transglutaminases, filaggrin, and laminin, can also aid distinction of differentiated epidermal layers. Methods of detecting certain proteins that can act as markers of differentiation and/or stratification are known to one of skill in the art and can include, e.g, immunofluorescence microscopy (see, e.g, Schoop et al., J. Invest Derm 1999;112(3):343-353), RT-PCR (see, e.g., Kikkawa et al., Biol Pharm Bull. 2010;33(2):307-10), and RNAseq. Keratinocytes are the major cellular component of the epidermis and comprise about 80% of the cells in adult human skin. All epithelia express type I and type II keratins, which range in molecular weight from 40 kDa to 70 kDa. Different epithelial tissues express specific pairs of keratins. The localization and relative amounts of proteins produced by differentiated keratinocytes can be used to distinguish different layers of a stratified epidermis. In some examples, transglutaminases, e.g., the keratinocyte transglutaminase isozyme, TGK, can be detected to distinguish layers of a stratified epidermis. In some examples, fillagrin, a filament-associated protein that binds to keratin fibers, can be detected to distinguish layers of a stratified epidermis. In some examples, laminins, extracellular matrix glycoproteins, can be detected to distinguish layers of a stratified epidermis. In some examples, keratins can be detected to distinguish layers of a stratified epidermis. In some examples, involucrin, a cell envelope protein, can be detected to distinguish layers of a stratified epidermis. In some examples, cadherin adhesion molecules (e.g., N-, E- and P-cadherin, which play a role in barrier function and formation, can be detected to distinguish layers of a stratified epidermis (Allen-Hoffmann, US 2014/0127170)).

Basal layer cells are columnar in shape and produce keratins K5 and K14. In some examples, cells of the basal layer rest on a structure called a basement membrane, which separates a dermis, or dermal equivalent, from the epidermis. Laminins can be found in the extracellular matrix of the basement membrane. In structures where a dermis-epidermis junction is absent, such as skin substitutes having only an epidermal component, laminins can be found in invaginations of the basal layer. In some examples, the expression of laminins, e.g., laminin 5, is detected to determine whether a basement membrane can be formed, such as when the skin substitute is applied to a subject.

The first suprabasal keratinocyte layer is the stratum spinosum (spinous layer), named for the spiny appearance of the many desmosomal contacts between adjacent cells. Keratinocytes in this layer may no longer produce K5 and K14 but, instead, can synthesize differentiation-specific keratins K1 and K10. Keratinocytes can begin to produce involucrin and epidermis-specific transglutaminases in the upper stratum spinosum. Morphologically, spinous cells are larger and more flattened than basal cells (Holbrook, 1994).

As keratinocytes differentiate further, they form the stratum granulosum (granular layer). Tight junction proteins have been identified in the granular layer and in deeper layers of the epidermis (Brandner et al., Open Dermatol. J. 2010; 4:14-20). Cells of this layer are characterized by distinct electron-dense keratohyalin granules containing profilaggrin, the protein precursor of filaggrin (Dale et al., 1994). Granular cells also contain lipid-filled granules that, during the transition zone between the stratum granulosum and stratum corneum, fuse with the plasma membrane and release their contents into the extracellular space, conferring hydrophobicity to the epidermal surface. As the differentiating keratinocytes transition from the granular to the cornified layer, i.e., the corneum layer or stratum corneum, the profilaggrin is cleaved to yield filaggrin, which is involved in the alignment and aggregation via disulfide bonds of keratin bundles called macrofibrils. Macrofibrils are the basic structural unit of the cornified envelope. In normal skin sections, filaggrin is localized in the granular layer, and can be found in cornified sheets (Sandilands et al., J Cell Sci. 2009; 122(9):1285-1294). Antibodies against filaggrin detect profilaggrin as well as its cleavage products.

The uppermost epidermal layer is the stratum corneum. Involucrin can be used as a differentiation marker of the stratum corneum. Cells of this layer, having completed the differentiation process, have lost their nucleus and all metabolic function. The cornified envelope is a highly stable insoluble protein structure formed beneath the plasma membrane that is resistant to detergents and reducing agents and confers strength and rigidity to the terminally differentiated cells of the uppermost epidermal layer. Cells of the corneum, also known as corneocytes, are joined together by modified desmosomes and are ultimately sloughed off in sheets from the skin's surface. For the stratum corneum, or cornified layer, introduction of an air-liquid interface is necessary for keratinocyte differentiation (Pruniéras et al., J Invest Dermatol. 1983 Jul;81(1 Suppl):28s-33s).

### 2. Keratinocyte Cell Culture

A low level of calcium, e.g., about 0.3 mM, in serum-free conditions promotes proliferation of keratinocytes in a basal, undifferentiated phenotype, and supplementation with bovine pituitary extract (BPE) can also enhance proliferation and cell survival. Switching from low to high calcium conditions ("calcium switch") can trigger markers of differentiation, but other factors may contribute to optimal differentiation into epidermal layers. In one example, culturing cells in serum-containing medium and reducing temperature, e.g., from 37°C to 31°C, combined with a calcium switch was found to induce markers of differentiation on a large scale (Borowiec et al., Plos One 2013: 8(10):e77507). Differentiated keratinocytes can revert to their basal state by exposure to low calcium medium. However, a high cell density, e.g., in excess of 75-80% cell confluency, and temperatures exceeding 37°C, can trigger differentiation in HaCaT cells, even under low calcium conditions (Wilson, Methods Mol Biol. 2014;1195:33-41).

In some embodiments, provided herein is a method of culturing keratinocytes in a low calcium medium to culture, e.g., obtain, a basal layer. In some embodiments, provided herein is a method of culturing non-primary keratinocytes in a low calcium medium to culture, e.g., obtain a basal layer. In some embodiments, provided herein is a method of culturing immortalized keratinocytes in a low calcium medium to culture, e.g., obtain, a basal layer. In some embodiments, provided herein is a method of culturing HaCaT keratinocytes in a low calcium medium to culture, e.g., obtain, a basal layer. In some embodiments keratinocytes are cultured for about two to about six weeks in a low calcium medium to form a basal layer. In some embodiments, are cultured for about three to about four weeks in a low calcium medium to form a basal layer. In some embodiments, keratinocytes are cultured for about two weeks, about three weeks, about four weeks, about five weeks, or about six weeks in a low calcium medium to form a basal layer. In some embodiments, keratinocytes are cultured for about four weeks in a low calcium medium to form a basal layer.

In some embodiments, the method provided herein includes a cell culture medium that supports the growth of keratinocytes and/or dermal fibroblasts and can be used to form a basal layer of a stratified epidermis, also referred to as basal keratinocytes. In some embodiments, a serum-free medium can be used to culture a basal layer of the stratified epidermis. In some embodiments, a calcium-free medium can be used to culture the basal layer of the stratified epidermis. In some embodiments, a serum-free and calcium-free medium can be used as an initial medium to culture the basal layer of the stratified epidermis. In some embodiments, the culture medium used to culture the basal layer can be adjusted to a final low level of calcium, e.g., about 0.01 mM Ca²⁺, about 0.02 mM Ca²⁺, about 0.03 mM Ca²⁺, about 0.04 mM Ca²⁺, about 0.05 mM Ca²⁺, about 0.06 mM Ca²⁺, about 0.07 mM Ca²⁺, about 0.08 mM Ca²⁺, about 0.09 mM Ca²⁺, or about 0.1 mM Ca²⁺ to form the basal layer of the stratified epidermis.

In some examples, the culture medium used to culture the basal layer can further comprise endothelial growth factor (EGF) and/or bovine pituitaries extract (BPE). In some embodiments, the low calcium culture medium further comprises about 0.1 ng/ml, about 0.2 ng/ml, about 3 ng/ml, about 4 ng/ml, about 5 ng/ml, or about 6 ng/ml EGF and/or about 10 µg/ml, 20 µg/ml, 30 µg/ml, 40 µg/ml, 50 µg/ml, 60 µg/ml, or 70 µg/ml BPE. In some embodiments, the low calcium culture medium to culture a basal layer further comprises about 0.1 ng/ml, about 0.2 ng/ml, or about 0.3 ng/ml EGF and/or about 20 µg/ml, 30 µg/ml, or 40 µg/ml BPE. In some embodiments, the low calcium culture medium to culture a basal layer further comprises about 0.2 ng/ml EGF and about 30 µg/ml BPE. In some embodiments, the low calcium medium used to culture the basal layer is serum free. In some embodiments, the low calcium medium used to culture the basal layer is serum free keratinocyte culture medium.

In some embodiments, provided herein is a method of exposing basal keratinocytes, which were previously cultured to form a basal layer as described herein, to a "calcium switch," wherein levels of calcium in a culture medium are changed from low to high levels to promote formation of a stratified epithelium. In some aspects, provided herein is a method of culturing basal keratinocytes in a low calcium medium then subsequently culturing the basal keratinocytes in a high calcium medium to form a skin substitute that comprises a stratified epidermis. In some embodiments, provided herein is a method of culturing basal non-primary keratinocytes in a low calcium medium then subsequently culturing the basal non-primary keratinocytes in a high calcium medium to form a skin substitute that comprises a stratified epidermis. In some aspects, provided herein is a method of culturing basal immortalized keratinocytes in a low calcium medium then subsequently culturing the basal immortalized keratinocytes in a high calcium medium to form a skin substitute that comprises a stratified epidermis. In some aspects, provided herein is a method of culturing basal HaCaT keratinocytes in a low calcium medium then subsequently culturing the basal HaCaT keratinocytes in a high calcium medium to form a skin substitute that comprises a stratified epidermis.

In some aspects, provided herein is a method wherein basal keratinocytes can be cultured on a substrate in low calcium medium, prior to the calcium switch (transition from low to high calcium media). The surface contacting the basal keratinocytes, i.e., the substrate, may comprise side walls, and/or can be in the form of an insert or a cup. In some embodiments, the substrate fits into an orifice of a well. In some embodiments the substrate can comprise a surface having a diameter and pores of an appropriate size, e.g., a size that supports the growth and differentiation of keratinocytes into a stratified epidermis. In some embodiments, the substrate can be composed of a variety of pore and diameter sizes. In some embodiments, the substrate is a mesh or a transwell insert, e.g., a transwell insert having a diameter of at least or about 50 mm, at least or about 75 mm, at least or about 100 mm, or at least or about 125 mm, and a pore size of at least or about 1.0 µm, at least or about 2.0 µm, at least or about 3.0 µm, at least or about 4.0 µm, or at least or about 5.0 µm. In some embodiments, the substrate is a transwell insert or a ring, e.g., a cloning ring. In some embodiments, the substrate may comprise a mesh, e.g., a wire mesh, and the seeded basal keratinocytes may be locatable on the mesh, e.g., above and/or below the substrate. In some embodiments, the substrate may be made of plastic or of metal. In some embodiments, basal keratinocytes may be seeded on a liquid-permeable base, e.g. above and/or below a wire mesh or plastic containing pores.

In some embodiments the substrate is coated, e.g., with a gel. In some embodiments, the gel may be collagen, i.e., jellified collagen, and/or a hydrogel. In some embodiments, the surface of the insert can be covered with a neutralized human use certified bovine collagen solution. In some embodiments a solution, e.g., a collagen solution, can be used to coat the substrate, and the solution-coated substrate can then be incubated for a sufficient amount of time until the solution jellifies, e.g., forming a jellified collagen. In some embodiments, the substrate or the coated substrate is washed, e.g., with PBS prior to seeding with basal keratinocytes.

In some embodiments, basal keratinocytes can be seeded onto the substrate, e.g., the coated substrate, in serum free and/or calcium free medium supplemented to a final low level of calcium. In some embodiments, basal keratinocytes can be seeded onto the substrate, e.g., the coated substrate, in serum free and/or calcium free medium supplemented to a final low level of calcium without any additional supplementation. In some embodiments, basal keratinocytes can be seeded onto the substrate, e.g., the coated substrate, in serum free and/or calcium free medium supplemented to a final low level of calcium with additional supplementation. In some embodiments, basal keratinocytes can be seeded onto the substrate, e.g., the coated substrate, in serum free and/or calcium free medium supplemented to a final low level of calcium supplemented with EGF and/or BPE.

In some embodiments, the low calcium culture medium is supplemented to a final low level of Ca²⁺ that is at or about 0.01 mM Ca²⁺, at or about 0.02 mM Ca²⁺, at or about 0.03 mM Ca²⁺, at or about 0.04 mM Ca²⁺, at or about 0.05 mM Ca²⁺, or at or about 0.06 mM Ca²⁺. In some embodiments, the low calcium culture medium further comprises about 0.1 ng/ml, about 0.2 ng/ml, about 3 ng/ml, about 4 ng/ml, about 5 ng/ml, or about 6 ng/ml EGF and/or about 10 µg/ml, 20 µg/ml, 30 µg/ml, 40 µg/ml, 50 µg/ml, 60 µg/ml, or 70 µg/ml BPE. In some embodiments, the low calcium culture medium further comprises about 0.1 ng/ml, about 0.2 ng/ml, or about 0.3 ng/ml EGF and/or about 20 µg/ml, 30 µg/ml, or 40 µg/ml BPE. In some embodiments, the low calcium culture medium further comprises about 0.2 ng/ml EGF and about 30 µg/ml BPE. In some embodiments, the low calcium medium used to culture the keratinocytes on a substrate is serum free. In some embodiments, the low calcium medium used to culture keratinocytes on a substrate is serum free keratinocyte culture medium.

In some embodiments, cells can be seeded to contact a substrate at a density of about 1 x 10⁶ cells/ml, 10 x 10⁶ cells/ml, 20 x 10⁶ cells/ml, 30 x 10⁶ cells/ml, 40 x 10⁶ cells/ml, or 50 x 10⁶ cells/ml. In some embodiments, cells are seeded below the substrate, above the substrate, or below and above the substrate. In some embodiments, cells can be seeded to contact a coated substrate at a density of about 1 x 10⁶ cells/ml, 10 x 10⁶ cells/ml, 20 x 10⁶ cells/ml, 30 x 10⁶ cells/ml, 40 x 10⁶ cells/ml, or 50 x 10⁶ cells/ml. In some embodiments, cells are seeded below the coated substrate, above the coated substrate, or below and above the coated substrate.

In some embodiments, following seeding onto a substrate, e.g., seeded above and below a coated substrate, basal keratinocytes, are incubated for about two to about six days, about three to about five days, or about three to about four days in low calcium medium. In some embodiments, following seeding onto a substrate, e.g. seeded above and below a coated substrate, basal keratinocytes are incubated for about two days, about three days, about four days, about five days, about six days, or about seven days in low calcium medium. In some embodiments, low calcium culture medium is changed daily. In some embodiments, low calcium culture medium is changed every other day. In some embodiments, the low calcium medium is serum free. In some embodiments, the low calcium medium is serum free keratinocyte culture medium.

In some embodiments, provided herein is a method of culturing keratinocytes, wherein introducing an air-liquid interface and increasing calcium levels in culture stimulates keratinocyte differentiation into a stratified epidermis. In some embodiments, the air-liquid interface and increased calcium levels can be introduced on the third, fourth, fifth, sixth, or seventh day of culture in the low calcium medium with the substrate. In some embodiments, upon introduction of the air-liquid interface, low calcium keratinocyte culture medium is discarded and replaced with medium adjusted to a relatively high level of calcium. In some embodiments the high level of calcium is at or about 1-5 mM Ca²⁺, 1-4 mM Ca²⁺, 1-3 mM Ca²⁺, 2-4 mM Ca²⁺, or 2-3 mM Ca²⁺. In some embodiments the high level of calcium is at or about 1.5 mM Ca²⁺ , 1.6 mM Ca²⁺ , 1.7 mM Ca²⁺ , 1.8 mM Ca²⁺ , 1.9 mM Ca²⁺ , 2.0 mM Ca²⁺ , 2.1 mM Ca²⁺, 2.2 mM Ca²⁺, 2.3 mM Ca²⁺, 2.4 mM Ca²⁺, 2.5 mM Ca²⁺, 2.6 mM Ca²⁺, 2.7 mM Ca²⁺, 2.8 mM Ca²⁺, 2.9 mM Ca²⁺, or 3 mM Ca²⁺.

In some embodiments, the high calcium medium is supplemented with EGF, bovine pituitary extract (BPE), and/or hydrocortisone. In some embodiments, the high calcium medium is supplemented with at or about 0.09 ng/ml EGF, at or about 0.1 ng/ml EGF, at or about 0.2 ng/ml EGF, at or about 0.3 ng/ml EGF, at or about 0.4 ng/ml EGF, or at or about 0.5 ng/ml EGF. In some embodiments, the high calcium medium is supplemented with at or about 10 µg/ml BPE, at or about 20 µg/ml BPE, at or about 30 µg/ml BPE, at or about 40 µg/ml BPE, or at or about 50 µg/ml BPE. In some embodiments, the high calcium medium is supplemented with hydrocortisone at or about 0.1 µg/ml, at or about 0.2 µg/ml, at or about 0.3 µg/ml, at or about 0.4 µg/ml, at or about 0.5 µg/ml, at or about 0.6 µg/ml, at or about 0.7 µg/ml, or at or about 0.8 µg/ml. In some embodiments, the high calcium medium is supplemented with about 0.2 ng/ml EGF, 30 µg/ml BPE, and 0.4 µg/ml hydrocortisone. In some embodiments, the high calcium medium is serum free. In some embodiments, the high calcium medium is serum free keratinocyte culture medium.

In some embodiments, keratinocytes can be cultured on a substrate, e.g., above and below a collagen coated substrate, in high calcium medium for about two to four weeks, about two to three weeks, or about three weeks until a stratified epidermis is obtained. In some embodiments, keratinocytes can be cultured in high calcium medium, e.g., above and below a collagen coated substrate, for 14 days, 15 days, 16 days, 17 days, 18 days, 19 days, 20 days, 21 days, 22 days, 23 days, 24 days, 25 days, 26 days, 27 days, or 28 days until a stratified epidermis is obtained. In some embodiments, the high calcium culture medium can be replaced daily until a stratified epidermis is obtained. In some embodiments, the high calcium culture medium can be replaced every other day until a stratified epidermis is obtained.

Upon introduction of an air-liquid interface, the differentiated keratinocytes located above and/or below the substrate, e.g., a collagen coated substrate, may be configured such that the uppermost surface of the cells forming the skin substitute is exposed to the gaseous environment, but not to the tissue culture medium, and/or the cells of the basal layer of the skin substitute are exposed to the tissue culture medium, but not to the gaseous environment. Introduction of an air-liquid interface can facilitate biphasic culturing of skin substitutes, i.e., gaseous and liquid environments. For example, the skin substitute in medium may be configured such that in use the corneum layer is exposed to the gaseous environment, but not to the tissue culture medium, and/or the basal layer and/or the dermis or dermal equivalent is exposed to the tissue culture medium, but not to the gaseous environment. This may be achieved by controlling the height of the liquid interface and/or positioning the location of the substrate, e.g., a collagen coated substrate. In some embodiments, a substrate, e.g., a transwell or insert, may be positioned such that the bottom of the transwell touches the liquid but the liquid does not contact the top of the epidermis/epidermal equivalent.

The keratinocyte culture medium may be at a temperature of about 33.0-37.5°C, e.g. about 34-37.5°C, about 35-37.5°C, about 36-37.5°C or about 37°C. The tissue culture medium may also be at a pH of about 6.1-7.9, e.g. about 6.2-7.7, about 6.3-7.7, about 6.4-7.7, about 6.5-7.7, about 6.6-7.7, about 6.7-7.6, about 6.8-7.6, about 6.9-7.6, about 7-7.6, about 7.1-7.6, about 7.1-7.5 or about 7.2-7.4. The tissue culture medium may comprise about 2-10%, about 2-8%, about 3-7%, about 4-6%, or about 5% CO₂. In some embodiments, the uppermost surface of the skin substitute is not exposed to the tissue culture medium, and the lowermost or basal surface of the skin substitute is not exposed to the gaseous environment.

The skin sample holder may be located in a laminar flow hood in order to maintain sterility. An atmospheric monitor may be used to monitor the conditions in the gaseous environment. In some embodiments, the gaseous environment can have a temperature of below or at about 37°C, e.g. about 10-36°C, about 12- 32°C, about 14-29°C, about 15-25°C, about 18-25°C, about 19-24°C, or about 20-22°C. The gaseous environment may also have a relative humidity at about or below about 90%, e.g., about 0-89%, about 0-85%, about 10-80%, about 15-75%, about 20-74%, about 23-70%, about 25-65%, about 30-50%, about 35-50%, about 40-50%, or about 40-45%.

In some embodiments, the gaseous environment for culture of a skin substitute may comprise less than: 5%, 4%, 3%, 2%, 1%, 0.5%, 0.1%, 0.09%, 0.08%, 0.07%, 0.06%, 0.05%, 0.045% or 0.04% CO₂. In some embodiments, the gaseous environment may comprise 0.02-0.05% or 0.035-0.045% CO₂. In some embodiments, the gaseous environment may comprise 18-25%, 18-24%, 18-23%, 19-23%, 19-22%, 20-22% or about 21% O₂. In some embodiments, the gaseous environment may contain about 78% N₂, and/or about 1% Argon. In some embodiments, the gaseous environment may comprise atmospheric air, compressed air and/or medical air. In some aspects, medical air may refer to sterile compressed air, and medical air may have a composition of gases that is similar to atmospheric air (e.g. approximately 78% N₂ and 21% O₂). In some aspects, the gaseous environment may mimic healthy internal room or physiological conditions.

### B. Engineering a Skin Substitute Comprising a Stratified Epidermis to Deliver a Growth Factor and Insulin

Provided herein are methods of introducing nucleic acid molecules, e.g., polynucleotides, to a skin substitute comprising cells of a stratified epidermis. In some embodiments, provided herein are methods of introducing any desired nucleic acid molecule, a vehicle, a construct or complex containing any nucleic acid molecule. In some embodiments, cells of the stratified epidermis can translate an introduced nucleic acid into a protein for delivery, e.g., secretion, to a subject. In some embodiments, keratinocytes of the stratified epidermis have been transduced with a nucleic acid molecule that has a desired function or that encodes a selected polypeptide with a desired function.

In some embodiments, the polynucleotide encoding a growth factor or insulin as described can be introduced into cells of the stratified epidermis by viral or non-viral methods. In some embodiments, non-viral methods of delivery include introduction of a DNA (e.g., double stranded circular or linear), RNA, a ribozyme, or an aptamer. In some embodiments, the introduction involves using a viral vector containing polynucleotides encoding the recombinant insulin or growth factor. For instance, an adenoviral vector can be used. In some embodiments, the introduced nucleic acid molecule can be provided as a construct containing a heterologous nucleic acid molecule or a transgene.

There are a number of constructs that are known to one of skill in the art for introducing nucleic acid into cells, either in vitro or in vivo. In some embodiments, such constructs include viral based delivery systems, e.g., for transduction, and non-viral based deliver systems, e.g., transfection. In some embodiments, the introduced polynucleotide can be a construct containing a nucleic acid molecule that is delivered in a vector (e.g., a viral vector or an expression vector), a nanoparticle (e.g., a targeted or radiolabeled nanoparticle), or a plasmid. Such constructs are well known in the art and readily adaptable for use with the compositions and methods described herein.

In any of the above provided embodiments, the polynucleotides encoding a recombinant growth factor and a recombinant insulin provided herein can be introduced into cells using recombinant DNA and cloning techniques. To do so, a recombinant DNA molecule encoding a recombinant molecule, e.g., recombinant growth factor or recombinant insulin, is prepared. Methods of preparing such DNA molecules are well known in the art. For instance, sequences coding for the peptides could be excised from DNA using suitable restriction enzymes. Alternatively, the DNA molecule could be synthesized using chemical synthesis techniques, such as the phosphoramidite method. Also, a combination of these techniques could be used. In some instances, a recombinant or synthetic nucleic acid may be generated through polymerase chain reaction (PCR). A DNA insert encoding the recombinant molecule can be cloned into an appropriate transduction/transfection vector as is known to those of skill in the art. Also provided are expression vectors containing the nucleic acid molecules.

In some embodiments, the expression vectors are capable of expressing the recombinant growth factor and recombinant insulin in an appropriate cell of the differentiated stratified epidermis under conditions suited to expression, and secretion, of the protein. In some aspects, nucleic acid molecule or an expression vector comprises the DNA molecule that encodes the recombinant molecules operatively linked to appropriate expression control sequences. Methods of effecting this operative linking, either before or after the DNA molecule is inserted into the vector, are well known. Expression control sequences include promoters, activators, enhancers, operators, ribosomal binding sites, start signals, stop signals, cap signals, polyadenylation signals, and other signals involved with the control of transcription or translation.

In some embodiments, expression of the recombinant molecules are controlled by a promoter or enhancer to control or regulate expression. The promoter is operably linked to the portion of the nucleic acid molecule encoding the recombinant molecule.

The resulting recombinant expression vector having the DNA molecule thereon is used to transform an appropriate host. This transformation can be performed using methods well known in the art. In some embodiments, the resulting expression vector having the DNA molecules thereon is used to transform, such as transduce, an appropriate cell. The introduction can be performed using methods well known in the art. Exemplary methods include those for transfer of nucleic acids, including via viral, e.g., adenoviral, transduction, transposons, and electroporation. In some embodiments, the expression vector is a viral vector. In some embodiments, the nucleic acid is transferred into cells by adenoviral transduction methods.

In some aspects, provided herein are polynucleotides encoding a growth factor and a precursor of human insulin for introduction to keratinocytes of a skin substitute comprising a stratified epidermis. In some embodiments, the introducing step comprises contacting cells of the skin substitute comprising a stratified epidermis with the polynucleotide (e.g. present in a viral vector) for up to or about 10 minutes, up to or about 20 minutes, up to or about 30 minutes, up to or about 45 minutes, up to or about 60 minutes, up to or about 75 minutes, up to or about 90 minutes, or up to or about 120 minutes. In some embodiments, the introducing step comprises contacting a layer, e.g., a basal layer, of the stratified epidermis with the polynucleotide (e.g. present in a viral vector) for up to or about 10 minutes, up to or about 20 minutes, up to or about 30 minutes, up to or about 45 minutes, up to or about 60 minutes, up to or about 75 minutes, up to or about 90 minutes, or up to or about 120 minutes.

### 1. Polynucleotides for Delivery to a Skin Substitute and Secreted Polypeptides Encoded by the Same

In some aspects, provided herein is a method comprising introducing polynucleotides to a skin substitute comprising a stratified epidermis. In some embodiments, the particular polynucleotide delivered or introduced to the skin substitute is or comprises a nucleic acid molecule, whereby expression thereof effects an activity or property that is useful when present in the localized target area and/or when secreted into the bloodstream. In some embodiments, introducing a polynucleotide to a skin substitute comprising a stratified epidermis results in production, e.g., secretion, of one or more encoded polypeptide(s) that has a desired or therapeutic effect. In some embodiments, the delivered or introduced nucleic acid molecule can be translated by cells of the stratified epidermis to produce and/or secrete one or more proteins to effect a desired response, e.g., wound closure in the context of wound healing.

In some embodiments, the nucleic acid molecule can be delivered or introduced as part of a vehicle, e.g., a viral vector, as a complex or construct, or as naked DNA. In some embodiments, the nucleic acid molecule can include a vector or plasmid containing the nucleic acid molecule, such as a viral vector or non-viral vector. In some embodiments, the nucleic acid molecule can be encapsulated in liposomes. In some embodiments, the nucleic acid molecule can be complexed to other agents, such as target ligands or other moieties and delivered as a nanoparticle.

In some embodiments, the polynucleotide introduced to cells of the skin substitute is or comprises a nucleic acid molecule that encodes one or more desired polypeptide(s), e.g., a growth hormone and insulin, or variants thereof. In some embodiments, the encoded polypeptide can be secreted or released from the cells of the skin substitute comprising a stratified epidermis. In some embodiments, the polynucleotide introduced to cells of the skin substitute can encode a growth factor, e.g., VEGF and a hormonal protein, which regulates cell growth, cell differentiation, or cell metabolism, e.g., proinsulin and/or insulin.

### a. Recombinant Growth Factors

Provided herein are methods of introducing a polynucleotide encoding a recombinant growth factor to keratinocytes of a skin substitute. In some embodiments, the methods provided herein comprise transducing cells of skin substitute composed of a stratified epidermis with a polynucleotide encoding a recombinant growth factor. In some embodiments, the polynucleotide molecule can encode a polypeptide that is a growth factor or portions thereof that bind to the receptor or a growth factor receptor or portions thereof that bind to ligand.

In some embodiments, a nucleic molecule encoding a growth factor is introduced to keratinocytes of the stratified epidermis. In some embodiments, keratinocytes of the stratified epidermis are transduced with a polynucleotide encoding the growth factor vascular endothelial growth factor (VEGF). In some embodiments, cells of the stratified epidermis are transduced with a polynucleotide encoding other members of the VEGF family of proteins may be used in the current invention, e.g., a vascular endothelial growth factor B (VEGF-B) polypeptide, or a c-fos induced growth factor (FIGF) polypeptide.

In some embodiments, the polynucleotide encodes a growth factor sequence that contains a signal peptide to facilitate secretion of the growth factor. In some embodiments, the signal peptide is present in a precursor growth factor sequence and is cleaved to form the mature growth factor that is secretable. In some embodiments, the signal peptide is an endogenous or native signal peptide of the growth factor. In some embodiments, the signal peptide is a heterologous signal peptide that is from a different protein. In some embodiments, the signal peptide is cleaved when the growth factor is expressed from a cell of the skin substitute. In some embodiments, the secretable growth factor sequence lacks the signal peptide. In some embodiments, the growth factor is secretable from the cell. In some embodiments, the recombinant growth factor is secretable from the stratified epidermis. In some embodiments, cells of the stratified epidermis secrete the recombinant growth factor.

In some embodiments, the growth factor is a VEGF-A, or is an isoform or an alternatively spliced variant thereof. VEGF-A is a key mediator of angiogenesis, signaling via the class IV tyrosine kinase receptor family of VEGF Receptors (VEGFRs). Although VEGF-A ligands bind to both VEGFR1 and VEGFR2, they primarily signal via VEGFR2 leading to endothelial cell proliferation, survival, migration and vascular permeability. Distinct VEGF-A isoforms result from alternative splicing. Any isoform or alternatively spliced variant of VEGF-A that retains the ability to bind a VEGF-R (e.g. VEGFR2) is contemplated in the provided skin substitutes. Typically, VEGF-A isoforms differ in their length and are designated VEGF xxx, where xxx represents the number of amino acids present in the final protein sequence.

Exemplary VEGF-A isoforms include, but are not limited to, VEGF 206 of vascular endothelial growth factor A (VEGF-A) polypeptide (SEQ ID NO: 11), isoform VEGF 189 of VEGF-A (SEQ ID NO: 19), isoform VEGF 183 of VEGF-A (SEQ ID NO: 20), isoform VEGF 148 of VEGF-A (SEQ ID NO: 21), isoform VEGF 145 of VEGF-A (SEQ ID NO: 22), isoform VEGF 165B of VEGF-A (SEQ ID NO: 23), isoform VEGF 121 of VEGF-A (SEQ ID NO: 24), isoform VEGF111 of VEGF-A (SEQ ID NO: 25), isoform VEGF 165 of VEGF-A (SEQ ID NO: 7), isoform L-VEGF165 of VEGF-A (SEQ ID NO: 26), isoform L-VEGF 121 of VEGF-A (SEQ ID NO: 27), isoform L-VEGF 189 of VEGF-A (SEQ ID NO: 28), isoform L-VEGF 206 of VEGF-A (SEQ ID NO: 29), isoform 15 of VEGF-A (SEQ ID NO: 30), isoform 16 of VEGF-A (SEQ ID NO: 31) , isoform 17 of VEGF-A (SEQ ID NO: 32), or isoform 18 of VEGF-A (SEQ ID NO: 33). It is understood that also included are mature sequences thereof that lack a signal peptide following its cleavage when expressed and produced from a cell.

In some embodiments, the polynucleotide encodes a recombinant human VEGF-A isoform that has at least at or about 80%, at least at or about 85%, at least at or about 90%, or at least at or about 95% sequence identity to the amino acid sequence set forth in any one of SEQ ID NOS: 7, 11 and 19-33, and retains binding to a VEGFR (e.g. VEGFR-2). In some embodiments, the polynucleotide encodes a recombinant human VEGF-A isoform set forth in any one of SEQ ID NOS: 7, 11 and 19-33. In some embodiments, the polynucleotide encodes a protein containing a signal peptide, which is proteolytically cleaved and removed so that a protein lacking the signal peptide is secreted, such as via the constitutive secretory pathway. In some embodiments, the polynucleotide encodes a VEGF-A isoform that has an amino acid sequence that has at least at or about 80%, at least at or about 85%, at least at or about 90%, or at least at or about 95% sequence identity to the amino acid sequence set forth in any one of SEQ ID NOS: 7, 11 and 19-33, and retains binding to a VEGFR (e.g. VEGFR-2). In some embodiments, the polynucleotide encodes a recombinant VEGF-A isoform set forth in any one of SEQ ID NOS: 7, 11 and 19-33. In some embodiments, the polynucleotide encodes a protein that lacks the signal peptide, which is proteolytically cleaved and removed, e.g., the encoded protein lacks the signal peptide set forth in any one of SEQ ID NOS: 7, 11 and 19-33 (e.g., lacks amino acid residues 1-26). In some embodiments, the polynucleotide comprises a sequence that has at least at or about 80%, at least at or about 85%, at least at or about 90%, or at least at or about 95% sequence identity to the sequence set forth in SEQ ID NO: 4. In some embodiments, the polynucleotide comprises the sequence set forth in SEQ ID NO: 4. In some embodiments, the polynucleotide is set forth in SEQ ID NO: 4. In some embodiments, the polynucleotide encodes a recombinant human VEGF-A that comprises a sequence that has at least at or about 80%, at least at or about 85%, at least at or about 90%, or at least at or about 95% sequence identity to the sequence set forth in SEQ ID NO: 7, or a sequence thereof that lacks the signal peptide. In some embodiments, the polynucleotide encodes a recombinant human VEGF-A that comprises the sequence set forth in SEQ ID NO: 7 or a sequence thereof that lacks the signal peptide. In some embodiments, the polynucleotide encodes a recombinant human VEGF-A set forth in SEQ ID NO: 7 or a sequence thereof that lacks the signal peptide.

In some embodiments, the polynucleotide that encodes a growth factor sequence contains a signal peptide to facilitate secretion of the VEGF. In some embodiments, the signal peptide is present in a precursor growth factor sequence and is cleaved to form the mature growth factor that is secretable. In some embodiments, the signal peptide is an endogenous or native signal peptide of the growth factor. In some embodiments, the signal peptide is a heterologous signal peptide that is from a different protein. In some embodiments the signal peptide is the sequence set forth as MNFLLSWVHWSLALLLYLHHAKWSQA (SEQ ID NO: 45). In some embodiments, the signal peptide is cleaved when the VEGF is expressed from a cell of the skin substitute.

In some embodiments, the growth factor is a member of the VEGF family of proteins. In some embodiments, the polynucleotide encodes a growth factor that is a vascular endothelial growth factor B (VEGF-B) polypeptide (e.g. SEQ ID NO: 12) or a c-fos induced growth factor (FIGF) polypeptide (also referred to as VEGF-D) (e.g. SEQ ID NO: 13),..

In some embodiments, the polynucleotide encodes a recombinant human growth factor that has an amino acid sequence that has at least at or about 80%, at least at or about 85%, at least at or about 90%, or at least at or about 95% sequence identity to the amino acid sequence set forth in any one of SEQ ID NOS: 12-13. In some embodiments, the polynucleotide encodes a recombinant human growth set forth in any one of SEQ ID NOS: 12-13. In some embodiments, the polynucleotide encodes a recombinant human growth factor set forth in any one of SEQ ID NOS: 12-13. In some embodiments, the encoded protein lacks the signal peptide, which is proteolytically cleaved and removed so that the encoded protein lacks the signal peptide set forth in any one of SEQ ID NOS: 12-13 (see e.g. Sequence Table).

In some embodiments, keratinocytes of the skin substitute comprising a polynucleotide encoding a growth factor secrete or release a growth factor. In some embodiments, keratinocytes of the skin substitute comprising a polynucleotide encoding a growth factor secrete or release a mature growth factor. In some embodiments, keratinocytes of the skin substitute comprising a polynucleotide molecule encoding a growth factor secrete or release a mature growth factor, wherein the growth factor comprises VEGF. In some embodiments, the polynucleotide encoding the secreted VEGF comprises a sequence that has at least at or about 80%, at least at or about 85%, at least at or about 90%, or at least at or about 95% sequence identity to the sequence set forth in SEQ ID NO: 4. In some embodiments, the polynucleotide encoding VEGF comprises the sequence set forth in SEQ ID NO: 4. In some embodiments, the VEGF isoform comprises the sequence that has at least at or about 80%, at least at or about 85%, at least at or about 90% or at least at or about 95% sequence identity to the amino acid sequence set forth in SEQ ID NO: 7. In some embodiments, the VEGF isoform comprises the amino acid sequence set forth in SEQ ID NO: 7. In some embodiments, cells of the basal layer of the stratified epidermis secrete the recombinant growth factor.

### b. Recombinant Insulin

Insulin is a polypeptide composed of 51 amino acid residues that is 5808 Daltons in molecular weight. It is produced in the beta-cell islets of Langerhans in the pancreas. An exemplary human insulin is translated as a 110 amino acid precursor polypeptide, containing a 24 amino acid signal peptide to ER, the signal sequence is cleaved, resulting in proinsulin. The proinsulin molecule is subsequently converted into a mature insulin by actions of proteolytic enzymes, known as prohormone convertases (e.g., PC1/3) and by actions of the exoprotease carboxypeptidase E (CPE) (Ramzy et al, Diabetes 2020; 69(7): 1451-1462). This cleavage results in removal of 4 basic amino acid residues and the remaining 31 amino acid C-peptide or connecting chain (corresponding to amino acid residues 57 to 87 of the preproinsulin polypeptide). The resulting insulin contains a 21 amino acid A-chain (corresponding to amino acid residues 66 to 86 of the proinsulin polypeptide) and a 30 amino acid B-chain (corresponding to amino acid residues 1 to 30 of the proinsulin polypeptide), which are crosslinked by disulfide bonds. Typically, mature insulin contains three disulfide bridges: one between position 7 of the A-chain and position 7 of the B-chain, a second between position 20 of the A-chain and position 19 of the B-chain, and a third between positions 6 and 11 of the A-chain.

In some embodiments, the methods provided herein comprise introducing polynucleotides to keratinocytes of the skin substitute that encode a precursor of recombinant human insulin. In some embodiments, the methods provided herein comprise introducing polynucleotides to keratinocytes of the skin substitute that encode proinsulin.

In some embodiments, the polynucleotide encodes a recombinant regular insulin that is a native or wild type insulin polypeptide. These include recombinant forms of human insulin, as well as insulins from bovine, porcine and other species. In some embodiments, the recombinant insulin is a recombinant insulin of a regular human insulin marketed as Humulin^{®} R, Novolin^{®} R and Velosulin^{®}. In some embodiments, the recombinant insulin is a recombinant insulin of a regular porcine insulin marketed as Iletin II^{®}.

In some embodiments, the polynucleotide encodes a recombinant human insulin. In some embodiments, the polynucleotide encodes a proinsulin precursor form of the insulin. In some embodiments, the precursor of human insulin is human proinsulin. In some embodiments, the polynucleotide encodes a human proinsulin amino acid sequence that has at least at or about 80%, at least at or about 85%, at least at or about 90%, or at least at or about 95% sequence identity to the amino acid sequence set forth in SEQ ID NO: 5. In some embodiments, the polynucleotide that encodes the human proinsulin set forth in SEQ ID NO: 5.

In some embodiments, the polynucleotide encodes a recombinant insulin that is a variant of a human insulin, such as a functional variant or species or allelic variant or is a truncated form of human insulin that has activity. In some embodiments, such polynucleotides that encode variants of insulin, including allelic and species variants, variants encoded by splice variants and other functional variants, such as insulin analogs or other derivatized or modified forms, encode an insulin that has at least 40%, 45%, 50%, 55%, 60%, 65%, 70%, 75%, 80%, 85%, 90%, 95%, 96%, 97%, 98%, 99% or more sequence identity to the sequence of human insulin set forth in SEQ ID NO: 5 or to a processed insulin thereof that contains an A and B chain, so long as the insulin binds to the human insulin receptor to initiate a signaling cascade that results in an increase of glucose uptake and storage and/or a decrease of endogenous glucose production.

In some embodiments, the polynucleotide encodes a recombinant insulin that is a species variants of human insulin. These include, but are not limited to, insulins derived from bovine and porcine. Bovine insulin differs from human insulin at amino acids 8 and 10 of the A chain and amino acid 30 of the B chain (SEQ ID NO: 17). Porcine insulin only differs from human insulin at amino acid 30 in the B chain where, like the bovine sequence, there is an alanine substitution in place of threonine (SEQ ID NO: 18). In some embodiments, the polynucleotide encodes a proinsulin precursor form of a bovine or porcine insulin, such as a proinsulin form of SEQ ID NO: 17 (e.g. amino acids 25-105 of SEQ ID NO: 17) or a proinsulin form of SEQ ID NO: 18 (e.g. amino acids 25-105 of SEQ ID NO: 18), or a sequence that has at least 40%, 45%, 50%, 55%, 60%, 65%, 70%, 75%, 80%, 85%, 90%, 95%, 96%, 97%, 98%, 99% or more sequence identity to insulin set forth in SEQ ID NO: 17 or SEQ ID NO: 18 or to a processed insulin thereof that contains an A and B chain, so long as the insulin binds to the human insulin receptor to initiate a signaling cascade that results in an increase of glucose uptake and storage and/or a decrease of endogenous glucose production.

In some embodiments, the polynucleotide encodes a variant of human insulin that contains one or more amino acid modifications compared to a human insulin. Exemplary insulin analogs (A and B chains) include fast-acting and longer-acting analog forms or superactive insulins (see e.g. Vajo et al. 2001 Endocrine Reviews 22:706-717). Fast-Acting insulin analogs are modified forms of insulin that typically contain one or more amino acid changes. The analogs are designed to reduce the self-association of the insulin molecule for the purpose of increasing the absorption rate and onset of action as compared to regular insulin. For example, insulin analogs include, but are not limited to, glulisine (LysB3, GluB29), HIVm-1 153 (LysB3, IleB28), HMR-1423 (GlyA21, HisB32), insulin aspart (AspB28), insulin lispro (LysB28, ProB29) and AspB10. In every instance above, the nomenclature of the analogs is based on a description of the amino acid substitution at specific positions on the A or B chain of insulin, numbered from the N-terminus of the chain, in which the remainder of the sequence is that of natural human insulin.

In some embodiments, the polynucleotide encodes a recombinant insulin that is insulin AspB10. Insulin AspB10 is a human insulin analog polypeptide containing a single amino acid change of the B-chain resulting in the substitution of aspartic acid (D) for the naturally occurring histidine (H) at position 10 in wild-type insulin (e.g. substation of H to D). The result of the substitution is a superactive insulin that is absorbed twice as rapidly as regular insulin (e.g. wild type human insulin). In some aspects, insulin AspB10 has increased binding affinity to the insulin receptor as compared to regular insulin (e.g. wild type human insulin). In some embodiments, the polynucleotide encodes a proinsulin precursor form of insulin AspB10 containing the A chain set forth in SEQ ID NO: 36 and the B chain set forth in SEQ ID NO: 41.

In some embodiments, the polynucleotide encodes a recombinant insulin that is insulin glargine. By virtue of the addition of two arginines to the C-terminus of the B-chain, the isoelectric point of the glargine insulin is shifted making it more soluble at an acidic pH. An additional amino acid change exists in the A chain (N21G) to prevent deamidation and dimerization resulting from an acid-sensitive asparagine. In some embodiments, the polynucleotide encodes a proinsulin precursor form of insulin glargine containing the A chain set forth in SEQ ID NO: 34 and the B chain set forth in SEQ ID NO: 35.

In some embodiments, the polynucleotide encodes a recombinant insulin that is insulin Lispro. Human insulin Lispro is an insulin polypeptide formulation containing amino acid changes at position 28 and 29 of the B-chain such that the Pro-Lys at this position in wild type insulin is inverted to Lys-Pro. The result of the inversion of these two amino acids is a polypeptide with a decreased propensity to self-associate, which allows for a more rapid onset of action. Specifically, the sequence inversion in the B-chain results in the elimination of two hydrophobic interactions and weakening of two beta-pleated sheet hydrogen bonds that stabilize the dimer (DeFelippis et al., Insulin Chemistry and Pharmacokinetics. In Ellenberg and Rifkin's Diabetes Mellitus 2002 pp. 481-500, McGraw-Hill Professional). Due to the amino acid modification, insulin Lispro is more rapidly acting then regular insulin. In some embodiments, the polynucleotide encodes a proinsulin precursor form of insulin Lispro containing the A chain set forth in SEQ ID NO: 36 and the B chain set forth in SEQ ID NO: 37.

In some embodiments, the polynucleotide encodes a recombinant insulin that is insulin aspart. Human insulin aspart is an insulin polypeptide formulation containing an amino acid substitution at position 28 of the B-chain of human insulin from a proline to an aspartic acid. The modification in insulin aspart confers a negatively-charged side-chain carboxyl group to create charge repulsion and destabilize the monomer-monomer interaction. Further, the removal of the proline eliminates a key hydrophobic interaction between monomers (DeFelippis et al., Insulin Chemistry and Pharmacokinetics. In Ellenberg and Rifkin's Diabetes Mellitus 2002 pp. 481-500, McGraw-Hill Professional). In some embodiments, the polynucleotide encodes a proinsulin precursor form of insulin aspart containing the A chain set forth in SEQ ID NO: 36 and the B chain set forth in SEQ ID NO: 38.

In some embodiments, the polynucleotide encodes a recombinant insulin that is insulin glulisine. Human insulin glulisine is an insulin polypeptide formulation containing an amino acid substitution in the B-chain at position B3 from asparagine to lysine and at amino acid B29 from lysine to glutamic acid compared to the sequence of the B-chain of human insulin. The modifications render the polypeptide molecule less prone to self-association compared to human insulin. In some embodiments, the polynucleotide encodes a proinsulin precursor form of insulin glulisine containing the A chain set forth in SEQ ID NO: 36 and the B chain set forth in SEQ ID NO: 39.

In some embodiments, the polynucleotide encodes a proinsulin form of insulin that is modified to promote cleavage of the proinsulin into a two chain form containing the A chain and the B chain. In comes cases, human keratinocytes, e.g., HaCaT cells, lack the enzymes necessary to efficiently cleave proinsulin for the production of mature insulin. For instance, the endopeptidases, such as PC-2 and PC-3 are not present or are not present at high enough levels for cleavage of the insulin. Instead, keratinocytes express furin, a calcium-dependent cleavage enzyme that belongs to the subtilisin-like proprotein convertase family of enzymes. In some embodiments, the polynucleotide encodes a human proinsulin that is a modified human proinsulin. In some embodiments, the encoded modified human proinsulin comprises a sequence that is recognized by an enzyme, e.g., a protease, that is expressed in keratinocytes, e.g., HaCaT cells, which allows the encoded proinsulin to be able to be processed in keratinocytes into a two-chain form containing an A-chain and a B-chain that are linked, such as by disulfide bonds. In some embodiments, the protease is furin and the modified human proinsulin comprises at least one furin recognition sequence. In some embodiments, the encoded modified human proinsulin comprises two furin recognition sequence introduced in place of the sequence containing the Arg31-Arg32 cleavage site (B-C junction) and Lys64-Arg65 cleavage site (C-A junction). In some embodiments, the at least one furin recognition sequence comprises the consensus sequence R-X-R-R, where X is any amino acid (SEQ ID NO: 8), or R-X-K-R, where X is any amino acid (SEQ ID NO: 9). In some embodiments, the furin cleavage site is RTKR (SEQ ID NO: 10). In some embodiments, the furin cleavage site is RQKR (SEQ ID NO: 42).

In some embodiments, the polynucleotide encodes a proinsulin that is an AspB10 insulin containing an A chain set forth in SEQ ID NO: 36 and a B chain set forth in SEQ ID NO: 41 in which the proinsulin further contains two furin recognition sequences. In some embodiments, each of the furin recognition sequence comprises the consensus sequence R-X-R-R, where X is any amino acid (SEQ ID NO: 8), or R-X-K-R, where X is any amino acid (SEQ ID NO: 9). In some embodiments, one of the furin cleavage site is RTKR (SEQ ID NO: 10). In some embodiments, one of the furin cleavage site is RQKR (SEQ ID NO: 42). In some embodiments, the polynucleotide encodes a modified human proinsulin that comprises a sequence that has at least at or about 80%, at least at or about 85%, at least at or about 90%, or at least at or about 95% sequence identity to the amino acid sequence set forth in SEQ ID NO: 6, in which the proinsulin contains the furin recognition sites and the amino acid substitution of Asp at position 10 of the B chain. In some embodiments, the polynucleotide encodes a modified human proinsulin that comprises the amino acid sequence set forth in SEQ ID NO: 6. In some embodiments, the polynucleotide encodes a modified human proinsulin that is set forth in SEQ ID NO: 6.

In some of any of the provided embodiments, the polynucleotide is a preproinsulin that further contains a signal peptide to facilitate secretion of the growth factor. In some embodiments, the signal peptide is cleaved from the encoded preproinsulin to form the mature proinsulin that is secretable. In some embodiments, the signal peptide is cleaved when the insulin is expressed from a cell of the skin substitute. In some embodiments, the mature proinsulin form is further processed into a recombinant insulin that is a two chain form containing an A and B chain as described. In some embodiments, the signal peptide is an endogenous or native signal peptide of insulin. In some embodiments, the signal peptide is a heterologous signal peptide that is from a different protein. In some embodiments, the sequence encodes the signal peptide MALWMRLLPLLALLALWGPDPAAA (SEQ ID NO: 43).

In some embodiments, the polynucleotide comprises a sequence that has at least at or about 80%, at least at or about 85%, at least at or about 90%, or at least at or about 95% sequence identity to the sequence set forth in SEQ ID NO: 2, in which the encoded proinsulin contains the furin recognition sites and the amino acid substitution of aspartic acid (Asp, D) at position 10 of the B chain. In some embodiments, the polynucleotide comprises the sequence set forth in SEQ ID NO: 2. In some embodiments, the polynucleotide is set forth in SEQ ID NO: 2.

### 2. Expression Constructs and Regulatory Elements

In some aspects, provided herein are expression constructs and regulatory elements that facilitate expression of a recombinant growth factor and recombinant insulin in cells of a skin substitute comprising a stratified epidermis. In some embodiments, cells of the stratified epidermis, e.g., cells of the basal layer, are transduced with an expression construct comprising the regulatory elements described herein. In some embodiments, the expression cassette is a bicistronic expression cassette in which the polynucleotide encoding the growth factor and the polynucleotide encoding the insulin are separated in the expression cassette by a bicistronic element.

### a. Promoters

The polynucleotides described herein can be driven by a promoter or enhancer to control or regulate their expression. In some embodiments, the promoter is operably linked to the coding region of a nucleic acid of which relatively high expression is desired. In some embodiments, the promoter is operably linked to the coding region of a nucleic acid that, following translation, would require post-translational modifications. Non-limiting examples of promoters include cytomegalovirus (CMV), simian virus 40 (SV40), phosphoglycerate kinase 1 (PGK1), ubiquitin C (Ubc), human beta actin, CAG, TRE, UAS, Ac5, polyhedron, CaMKIIa, GALl, GAL 10, TEF1, GDS, ADH1, CaMV35S, Ubi, HI, U6, SSFV, MNDU3, and EFl-a (alternatively named Efla).

In some embodiments, promoters can be tissue specific. A tissue specific promoter allows for the production of a protein in a certain population of cells that have the appropriate transcriptional factors to activate the promoter. Numerous promoters are commercially available and widely known in the art; an exemplary sequence can be found at Entrez Gene ID 1915. In some embodiments, the promoter is selected from the group of a cytomegalovirus immediate-early promoter (CMV), a simian virus 40 early promoter (SV40), or a Rous sarcoma virus LTR promoter (RSV).

Any strong promoter known to those skilled in the art can be used for driving the expression of DNA. The promoter can be a constitutive promoter, such as a CMV promoter, a tissue-specific promoter, an inducible or regulatable promoter. In some embodiments, the polynucleotide to be introduced into cells contains an inducible promoter operably linked to the coding region, such that expression of the nucleic acid is controllable by controlling the presence or absence of the appropriate inducer of transcription.

In some embodiments, the promoter is a regulated promoter and transcription factor expression system, such as the published tetracycline-regulated systems or other regulatable systems (see, e.g., WO01/30843), to allow regulated expression of the encoded polypeptide. Exemplary of other promoters, are tissue-selective promoters, such as those described in U.S. Pat. No. 5,998,205, including, for example, a fetoprotein, DF3, tyrosinase, CEA, surfactant protein and ErbB2 promoters. An exemplary regulatable promoter system is the Tet-On (and Tet-Off) system available, for example, from Clontech (Palo Alto, Calif.). This promoter system allows the regulated expression of the transgene controlled by tetracycline or tetracycline derivatives, such as doxycycline. Other regulatable promoter systems are known (see e.g., U.S. Patent Publication No. 2002-0168714, entitled "Regulation of Gene Expression Using Single-Chain, Monomeric, Ligand Dependent Polypeptide Switches," which describes gene switches that contain ligand binding domains and transcriptional regulating domains, such as those from hormone receptors).

In some embodiments, the promoter is a constitutive promoter. Exemplary promoters include, but are not limited to, a CMV promoter, a truncated CMV promoter, a human serum albumin promoter or a C-1-antitrypsin promoter. In some embodiments, the promoter is a truncated CMV promoter in which binding sites for known transcriptional repressors have been deleted. CMV-derived promoters can be human or of simian origin. In some embodiments, the promoter is an inducible promoter. For example, the promoter is the inducible ecdysone promoter. Other examples of promoters include steroid promoters, such as estrogen and androgen promoters, and metallothionein promoters. In some embodiments, the enhancer can be a tissue specific- or non-specific enhancer. For example, the enhancer is a liver-specific enhancer element. Exemplary enhancer elements include, but are not limited to, human serum albumin (HSA) enhancers, human prothrombin (HPrT) enhancers, C-1-microglobulin enhancers, intronic aldolase enhancers and apolipoprotein E hepatic control region.

In some embodiments, a promoter such as an animal virus-derived promoter, a mammalian cell-derived promoter, or a hybrid promoter of both promoter and the like can be used without limitation. In many cases it is desirable to express a gene, including a therapeutic gene, at a relatively high level. Examples of high-expression promoters include a CMV promoter (Foecking M. K. et al., Gene 1986;45:101-105) and a CAG promoter (Niwa H. et al., Gene 1991;108:193-200), and the like. The CMV promoter consists of an enhancer and a promoter of the immediate early (IE) gene of cytomegalovirus (CMV), and the CAG promoter consists of an IE enhancer of CMV, chicken β-actin promoter, a splice acceptor and poly(A) sequence of rabbit β-globin. Thus, both of the CMV promoter and the CAG promoter contain an enhancer of the IE gene of CMV (Boshart M. et al., Cell 1985;41:521-530). As used herein, this enhancer of the IE gene of CMV may be termed simply as a "CMV enhancer."

Examples of a constitutive promoter include a CAG promoter, a CMV promoter, an EF-1α promoter, an SRα promoter, an SV40 promoter, an RSV promoter, an adenovirus major late promoter (MLP), and the like. Examples of an inducible promoter include a metallothionein gene promoter, a mouse mammary tumor virus (MMTV) promoter, and the like. Furthermore, there can be used a system in which the expression of a constitutive promoter is induced by tetracycline or ecdysone. Expression vectors and expression-inducing systems having such a promoter are commercially available or available from public agencies. Commercial ones, if available, can be purchased from Invitrogen Inc., Clontech Inc., etc.

In some embodiments, in addition to the above promoter containing the CMV enhancer, promoters similarly derived from viruses such as a SV40 promoter, a Rous sarcoma virus (RSV) promoter (Takebe Y. et al., Mol. Cell. Biol. 1988;8:466-472) can also be used.

In some aspects, provided herein is a viral vector having a CAG promoter that expresses genes such as a growth factor and insulin, or variants thereof. In some embodiments, the nucleic acid sequence encoding the protein that will undergo the most post-translational modifications precedes any other nucleotides downstream from the promoter, e.g., a CAG promoter. In some embodiments, the nucleic acid sequence encoding a growth factor is upstream from the nucleic acid sequence encoding insulin, or variants thereof, downstream from the promoter. These embodiments should not be construed as limiting, as other specific or promiscuous promoters with different transgenes that codify for other proteins may also be used. The gene to be inserted into the adenovirus vector of the present invention is not specifically limited, and a gene encoding a protein such as a growth factor and a hormone, e.g., insulin, and the like may be used.

In some embodiments, the nucleotide sequence of the CAG promoter can be replaced by a nucleotide sequence comprising a nucleotide sequence having at least 60% sequence identity or similarity to SEQ ID NO: 1. In some embodiments, the preferred nucleotide sequence is at least 60%, 65%, 70%, 75%, 80%, 85%, 90%, 95%, 97%, 98%, 99%, or 100% similar to SEQ ID NO: 1.

In some embodiments, the coding sequences encoding each of the different polypeptide chains can be operatively linked to a promoter, which can be the same or different. In some embodiments, the nucleic acid molecule can contain a promoter that drives the expression of the polynucleotide encoding the growth factor and the polynucleotide encoding the insulin.

### b. Bicistronic element

In some embodiments, expression cassettes containing encoding polynucleotide can be multicistronic (bicistronic or tricistronic, see *e.g.,* U.S. Patent No. 6,060,273). In some embodiments, transcription units can be engineered as a bicistronic unit containing a bicistronic element, which allows co-expression of gene products by a message from a single promoter. In some embodiments, the bicistronic element is an IRES (internal ribosome entry site). In some embodiments, the bicistronic element may be a self-cleavage sequence, such as 2A sequence (e.g. P2A, FTA or T2A).

Internal ribosome entry sites (IRES) are sequences which initiate translation from an internal initiation codon (usually AUG) within a bi- or multicistronic RNA transcript continuing multiple protein coding regions. IRES have been characterized in encephalomyocarditis virus and related picornaviruses (e.g., Jackson et al., RNA 1995;1:985-1000 and Herman, Trends in Biochemical Sciences 1989;14(6):219-222). IRES sequences are also detected in mRNAs from other viruses such as cardiovirus, rhinovirus, aphthovirus, hepatitis C virus (HCV), Friend murine leukemia virus (FrMLV) and Moloney murine leukemia virus (MoMLV). The presence of IRES in cellular RNAs has also been described. Examples of cellular mRNAs containing IRES include those encoding immunoglobulin heavy-chain binding protein (BiP), vascular endothelial growth factor (VEGF), fibroblast growth factor 2, insulin-like growth factor, translational initiation factor eIF4G, and the yeast transcription factors TFIID and HAP4 (e.g., Macejak et al., Nature 1991;353:90-94; Oh et al., Genes Dev. 1992;6:1643-1653; Vagner et al., Mol. Cell. Biol. 1995;15:35-44; He et al., PNAS 1996;93:7274-7278; He et al., Gene 1996;175:121-125; Tomanin et al., Gene 1997;193:129-140; Gambotto et al., Cancer Gene Therapy 1999;6:45-53; Qiao et al., Cancer Gene Therapy 1999;6:373-379). Expression vectors containing IRES elements have been described. See, for example, PCT/US98/03699 and PCT/EP98/07380.

In some embodiments, the viral vector described herein comprises one or more transgenes. In one example, the vector encodes two transgenes, for example, a transgene encoding a growth factor and a transgene encoding insulin, or variants thereof. In some embodiments, the same regulatory element exerts transcriptional control over the first and second transgenes, and optionally, one transgene is under the translational control of an internal ribosome entry site. In some embodiments, different elements regulate the transcription of each of the two transgenes, and one transgene is optionally under the translational control of an IRES.

### c. 3' Untranslated Region (UTR)

A 3'-untranslated region (3'-UTR) is typically the part of an mRNA which is located between the protein coding region (i.e., the open reading frame) and the poly(A) sequence of the mRNA. A 3'-UTR of the mRNA is not translated into an amino acid sequence. The 3'-UTR sequence is generally encoded by the gene which is transcribed into the respective mRNA during the gene expression process. The genomic sequence is first transcribed into premature mRNA, which comprises optional introns. The pre-mature mRNA is then further processed into mature mRNA in a maturation process. This maturation process comprises the steps of 5'-capping, splicing the pre-mature mRNA to excise optional introns and modifications of the 3'-end, such as polyadenylation of the 3'-end of the pre-mature mRNA and optional endo- or exonuclease cleavages etc. A 3'-UTR can correspond to the sequence of a mature mRNA, which is located 3' to the stop codon of the protein coding region, preferably immediately 3' to the stop codon of the protein coding region, and which extends to the 5'-side of the poly(A) sequence, preferably to the nucleotide immediately 5' to the poly(A) sequence. The term "corresponds to" indicates that the 3'-UTR sequence may be an RNA sequence, such as in the mRNA sequence used for defining the 3'-UTR sequence, or a DNA sequence which corresponds to such RNA sequence. The term "a 3'-UTR of a gene", such as "a 3'-UTR of an insulin gene," is the sequence that corresponds to the 3'-UTR of the mature mRNA derived from this gene, i.e., the mRNA obtained by transcription of the gene and maturation of the pre-mature mRNA. The term "3'-UTR of a gene" encompasses the DNA sequence and the RNA sequence of the 3'-UTR.

In some embodiments, a suitable 3'-UTR sequence may be operably linked to a nucleotide sequence encoding the desired transgene or transgenes. In some embodiments, a 3'-UTR sequence may be operably linked to a nucleotide sequence encoding a growth factor and insulin, or variants thereof. In some embodiments, suitable 3'-UTR regions may be those naturally associated with the nucleotide sequence or may be derived from different genes, such as for example the bovine growth hormone 3'-UTR region (bGH polyadenylation signal, SV40 polyadenylation signal, SV40 polyadenylation signal and enhancer sequence. In the context of the invention, when one refers to "SV40," the SV40 polyadenylation signal is referenced. When one refers to "SV40 enhancer sequence," the SV40 polyadenylation signal and enhancer sequence are referenced.

In some embodiments, the 3'-UTR sequence comprises a poly-A tail, also called a 3'-poly(A) tail or poly(A) sequence. A poly-A tail is a long sequence of adenosine nucleotides added to the 3'-end of an RNA molecule. Polyadenylation is the addition of a poly(A) sequence to a nucleic acid molecule, such as an RNA molecule, e.g., to a premature mRNA. Polyadenylation may be induced by a polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

In some embodiments, expression construct described herein may comprise a 3'-UTR region comprising a poly-A tail that has up to about 400 adenosine nucleotides, e.g., from about 25 to about 400, from about 50 to about 400, from about 50 to about 300, from about 50 to about 250, from about 60 to about 250 adenosine nucleotides, from about 70 to about 250 adenosine nucleotides, from about 80 to about 250 adenosine nucleotides, from about 90 to about 250 adenosine nucleotides, from about 100 to about 250 adenosine nucleotides, from about 100 to about 200 adenosine nucleotides, or from about 100 to about 150 adenosine nucleotides.

### 3. Viral vectors for transduction

A virus can be used as gene delivery vehicle when an exogenous nucleic acid sequence is inserted into a viral vector. Provided herein are viral vectors that contain a polynucleotide encoding a recombinant growth factor (e.g. any as described herein, such as a VEGF) and a polynucleotide encoding a recombinant insulin, such as a recombinant human insulin (e.g. any as described). Also provided herein are viral vectors that contain any of the expression cassettes, such as bicistronic expression cassettes, as described herein.

Viruses are useful in delivering nucleic acid molecules, e.g., polynucleotides, in vivo because they are efficient at transferring viral DNA into host cells. They can infect and be taken up by specific target cells depending on the viral attachment proteins (e.g., capsid or glycoproteins), and they can be manipulated to remove non-essential genes and add heterologous nucleic acid molecules. Many viral vectors are known to those skilled in the art. Examples of viruses that can be used in the methods herein include, but are not limited to, adenoviruses, adeno-associated viruses, alphaviruses, baculoviruses, hepadenaviruses, poxviruses, herpesviruses, retroviruses, lentiviruses, orthomyxoviruses, papovaviruses, paramyxoviruses, and parvoviruses. The choice of virus is within the level of one of skill in the art and is dependent on a number of factors, such as the desire for replication or integration of viral DNA, the tropism of the virus, and/or the immunogenicity of the virus.

Such viruses and derivatives thereof, are well-known and available to one of skill in the art. For example, many are available from the American Type Culture Collection (ATCC, Rockville, Md.) or from commercial vendors (e.g., Vector Biolabs, Philadelphia, Pa.; Applied Biological Materials, Inc., Richmond, British Columbia, Canada). Viral vectors for use in generating recombinant viruses include replication-competent viruses and replication-defective viruses. In replication-defective viruses, the virus typically lacks one or more genes associated with viral replication and, in some cases, cannot replicate beyond the first cycle of infection. In order to produce replication defective viruses, transfer vectors, packaging vectors or helper virus may be required. For example, a packaging vector can be provided as a cosmid or in a cell line that provides the viral structural proteins for packaging of the defective vector. The viral vectors also can contain expression cassettes that include regulatory elements, such as promoters and enhancers, operably linked to a transgene of choice. Any suitable promoter can be used. Suitable promoters and enhancers are widely available in the art for use in the viral vector of choice.

### a. Adenoviral Vectors

Adenoviral vectors have several advantages for use as gene delivery vehicles, including tropism for both dividing and non-dividing cells, minimal pathogenic potential, ability to replicate to high titer for preparation of vector stocks, and the potential to carry large inserts (see, e.g., Berkner Curr. Top. Micro. Immunol. 1992;158:39-66; Jolly et al. Cancer Gene Therapy 1994;1:51-64). Adenovirus is a nuclear DNA virus with a genome of about 36 kb, which has been well-characterized through studies in classical genetics and molecular biology (Horwitz, M. S., "Adenoviridae and Their Replication in Virology, 2nd edition, Fields, B. N., et al., eds., Raven Press, New York, 1990). The genome is classified into early (known as E1-E4) and late (known as L1-L5) transcriptional units, referring to the generation of two temporal classes of viral proteins. The demarcation between these events is viral DNA replication. Adenoviruses exhibit a natural tropism for epithelial cells of the respiratory and gastrointestinal tract. Adenovirus can also infect liver cells, such as hepatocytes and endothelial cells, which can occur upon clearance of the virus into the liver after systemic administration. Penton base and fiber proteins on the surface of the virus are responsible for the virus tropism. Multiple interactions between adenoviral particles and the host cell are required to promote efficient cell entry (Nemerow, Virology 2000;274:1-4).

For subgroup C adenoviruses, such as adenovirus 2 and 5 (Ad2 or Ad5), the viral entry pathway has been well characterized and is believed to involve two separate cell surface events. First, a high affinity interaction between the adenoviral fiber knob and coxsackie-adenovirus receptor (CAR) mediates the attachment of the adenovirus particle to the cell surface. A subsequent association of penton with the cell surface integrins αᵥβ₃ and αᵥβ₅, which act as co-receptors, potentiates virus internalization. CAR, which is expressed in many human tissues including lung epithelial cells (Bergelson et al., Science 1997;275:1320-1323), appears to function as a cellular receptor for most adenoviral Subgroups, except Subgroup B (Bergelson et al., Science 1997;275:1320-1323; Roelvink et al., J. Virol. 1998;72:7909-7915). In some embodiments, the adenovirus used to transduce cells of a skin substitute comprising a stratified epidermis is a type 5 adenovirus. In some embodiments, the adenovirus used to transduce basal cells of a skin substitute comprising a stratified epidermis is a type 5 adenovirus.

Adenovirus includes over 50 serotypes that are grouped into six distinct subgroups, A to F. Any of these adenovirus serotypes, which are available from the American Type Culture Collection (ATCC, Rockville, Md.) and other commercial and non-commercial providers can be used in the methods herein or used as a source for further modification as is known in the art. Also, any other serotype of adenovirus available from any other source can be used or further modified. For instance, an adenovirus can be of Subgroup A (e.g., serotypes 12, 18, 31), Subgroup B (e.g., serotypes 3, 7, 11a, 11 p. 14, 16, 21, 34, 35, 50), Subgroup C (e.g., serotypes 1, 2, 5, 6), Subgroup D (e.g., serotypes 8, 9, 10, 13, 15, 17, 19, 19p. 20, 22-30, 32, 33, 36-39, 42-49, 51), Subgroup E (e.g., serotype 4), Subgroup F (e.g., serotypes 40, 41), or any other adenoviral serotype. In certain embodiments, the adenovirus is a subgroup C adenovirus or derived from a Subgroup C adenovirus. In a preferred example, the adenovirus is a Subgroup C type 5 adenoviruses. Adenoviral vectors are available in the art (e.g., available from the American Type Culture Collection (ATCC, Rockville, Md.)), and the sequences of the wild-type adenovirus proteins from many different adenovirus serotypes are well known in the art (see e.g., Roberts et al. J. Biol. Chem. 1984;259:13968-13975; Chroboczek et al. Virology 1992;186:280-285; Sprengel et al. J. Virol. 1994;68:379-389; Chillon et al. J. Virol. 1999;73:2537-2540; Davison et al. J. Mol. Biol. 1993;234:1308-1316; www.binfgmu.edu/wiki/index.php/Human Adenovirus Genome Sequences and Annotations). Adenoviral vectors are widely available to the skilled artisan, for example from the American Type Culture Collection (ATCC) or other commercial or non-commercial provider. From the ATCC, adenoviruses are available as ATCC numbers VR-1 to VR-1616. For example, wild type adenovirus type 5 is available as VR-5 and VR-1082. Any of a number of recombinant or modified adenoviruses can be generated that are derived from any of the above serotypes, as described in the art and herein or by any suitable method known to one of skill in the art.

Adenovirus vectors for use in the methods described herein can include defective adenovirus vectors that contain at least one deletion in the first early gene region (E1-E4). Modifications to adenoviral vectors include deletions known in the art. Such as deletions may be made in one or more of the E1, E2a, E2b, E3, or E4 coding regions. For example, adenovirus vectors for gene therapy can be prepared by substitution of a heterologous nucleic acid molecule in place of the E1, E2a, E2b, E3 and/or E4 genes. Deletion can be effected using restriction endonucleases. For example, the E1a region can be deleted using convenient restriction endonuclease sites within the E1a region. Often, a portion of E3 is also deleted by restriction endonuclease addition so as to permit the insertion of a larger piece of foreign DNA while still satisfying the size constraints required for packaging into new viral particles. Due to deletion of these regions, the cloning capacity of an adenovirus vector can be about 8 kb. Such adenoviral vectors are typically referred to as replication defective adenovirus due to the at least one deletion in the first viral early gene region, such as E1, which includes the E1a and E1b regions.

In some embodiments, the adenovirus used to transduce cells of a skin substitute comprising a stratified epidermis is a replication-deficient type 5 adenovirus. In some embodiments, the adenovirus used to transduce cells of a skin substitute comprising a stratified epidermis is a replication-deficient type 5 adenovirus with a deletion at region E1. In some embodiments, the adenovirus used to transduce cells of a skin substitute comprising a stratified epidermis is a replication-deficient type 5 adenovirus with a deletion at region E3. In some embodiments, the adenovirus used to transduce cells of a skin substitute comprising a stratified epidermis is a replication-deficient type 5 adenovirus with deletions at regions E1 and E3. In some embodiments, the adenovirus used to transduce basal cells of a skin substitute comprising a stratified epidermis is a replication-deficient type 5 adenovirus with a deletion at region(s) E1 and/or E3.

Deletion of the early genes, such as viral regions E1 and E3, renders the recombinant adenovirus defective for replication and incapable of producing infectious viral particles in subsequently infected target cells. Thus, to permit early gene deleted adenovirus genome replication, such as E1-deleted adenovirus genome replication, and to produce virus particles requires a system of complementation which provides the missing gene product. For example, E1 complementation is typically provided by a cell line expressing E1. Such as the human embryonic kidney packaging cell line, i.e. an epithelial cell line, called 293 (deposited with the ATCC under Accession No. CRL-1573). Cell line 293 contains the E1 region of adenovirus, which provides E1 gene region products to "support the growth of E1-deleted virus in the cell line (see e.g., Graham et al., J. Gen. Virol. 36:59-71, 1977). Additionally, cell lines that are usable for production of defective adenovirus having a portion of the adenovirus E4 region have been reported (see, e.g., International published Appl. No. WO 96/22378). E3 also can be deleted from the vector, but since it is not required for vector production, it can be omitted from the complementing producer cell. Complementing producer cell lines and methods of generating complementing producer cell lines are known in the art (see e.g., Morris et al., BMC Biotechnology 2010;10(92)).

The benefit of the use of replication deficient viruses as vectors is that they are limited in the extent to which they can spread to other cell types, since they can replicate within an initial infected cell, but are unable to form new infectious viral particles. Multiple deficient adenoviral vectors and complementing cell lines have also been described (see, e.g., International PCT Publication Nos. WO95/34671, U.S. Pat. No. 5,994,106). The construction of replication-defective adenoviruses has been described (Berkner et al., J. Virol. 1987;61:1213-20; Massie et al., Mol. Cell. Biol. 1986;6:2872-83; Haj-Ahmad et al., J. Virol. 1986;57:267-74; Davidson et al., J. Virol. 1987;61:1226-39; Zhanget al., BioTechniques 1993;15:868-72; Berkner Nuc. Acids Res. 1983; 11:6003 : Ghosh-Choudhury Biochem. Biophys. Res. Commun., 1987;147:964; Gilardi et al., FEBS 1990;267:60; Mittal Virus Res. 1993;28:67; Yang PNAS 1993;90:4601; and International published PCT WO1995/026411).

Adenovirus vectors also include "gutless" or "gutted" vectors in which all viral genes are removed leaving only the inverted terminal repeats (ITRs) necessary for vector propagation. Such adenoviral vectors are designated pseudoadenoviral vectors (PAVs) because they are derived from the genome of an adenovirus that contain minimal cis-acting nucleotide sequences required for the replication and packaging of the vector genome. PAVs vectors contain the 5' ITR and the 3' ITR nucleotide sequences that contain the origin of replication, and the cis-acting nucleotide sequences required for packaging of the PAV genome. They can be modified to contain one more transgenes with appropriate regulatory elements (e.g., promoter or enhancers). PAVs have a carrying capacity of far more than 8 kb in size and up to 36 kb in size, since they contain deletions of most viral coding sequences (see e.g., U.S. Pat. Nos. 5,882,887 or 5,670,488; PCT Publication No. WO96/40955, WO97/ 25466, WO95/29993, WO97/00326; Morral et al. Hum. Gene Ther. 1998;10:2709-2716, Kochanek et al. PNAS 1996;93:5731-5736; Parks et al. PNAS 1996;93:13565-13570; Lieber et al. J. Virol. 1996;70:8944-8960; Fisher et al. J. Virol. 1996;217:11-22).

PAVs are grown by co-infection of the producing cells with a "helper" virus (such as using an E1-deleted adenovirus vector), where the packaging cells express the E1 gene products. The helper virus trans-complements the missing adenovirus functions, including production of the viral structural proteins needed for particle assembly. For example, a helper adenovirus vector genome and a gutless adenoviral vector genome are delivered to packaging cells. The cells are maintained under standard cell maintenance or growth conditions, whereby the helper vector genome and the packaging cell together provide the complementing proteins for the packaging of the adenoviral vector particle. Such gutless adenoviral vector particles are recovered by standard techniques. The helper vector genome can be delivered in the form of a plasmid or similar construct by standard transfection techniques, or it can be delivered through infection by a viral particle containing the genome. Such viral particle is commonly called a helper virus. Similarly, the gutless adenoviral vector genome can be delivered to the cell by transfection or viral infection.

Adenoviruses also include replication-conditional adenoviruses, which are viruses that replicate in certain types of cells or tissues but not in other types as a result of placing adenoviral genes essential for replication under control of a heterologous promoter (discussed above; see, also U.S. Pat. No. 5,998.205, U.S. Pat. No. 5,801,029 and U.S. application Ser. No. 10/081,969, published as US2003/0104625 and corresponding International PCT Publication No. WO2002/ 067861).

Adenoviruses also include those that have been modified to contain a targeting ligand to increase infection of specific target cells that express receptors (proteins, lipids, carbohydrates, or portions thereof) for the targeting ligand, for example, to alter the tropism of the virus. While adenoviral vectors, and others, hold much promise for therapeutic applications, their usefulness is limited by the widespread tissue distribution of CAR, which restricts delivery of adenoviral vectors to specific cell types. Furthermore, the absence of CAR and/or C, integrin receptors on certain cells in vivo restricts the cell or tissue types that can be targeted by adenoviral vectors. Thus, adenovirus also include those that have been modified by reducing or ablating binding to native receptors and/or engineering capsid proteins, such as the HI loop, C terminus of fiber, the L1 loop of hexon or the RGD loop of penton base, or the capsid protein IX, to incorporate target ligands for a desired cell receptor or tissue-specific receptor (see, e.g., Krasnykh et al., Mol. Ther, 2000; 1(5):P391-405 and Wickham, Gene Ther. 2000; 7:110-4). A capsid protein can be modified, for example, by addition of a target ligand or substitution of the fiber with other types of adenovirus fiber. The target ligand can be any protein, or portion thereof, that binds to a moiety in or on a cell. Such as a cell surface protein, lipid, carbohydrate or other moiety. For example, the target ligand includes, but is not limited to, growth factors, adhesion molecules, cytokines, protein hormones, neuropeptides (neurotransmitters) and single-chain antibodies, or a suitable portion thereof. In other examples, adenovirus vectors can be conjugated with adaptor molecules, such as antibody and fusion protein containing an anti-Ad single-chain antibody (sclv) or the extracellular domain of CAR with the targeting ligand, or chemically modified with polymers, e.g., polyethylene glycol (PEG) moieties, that contain the targeting ligands (see e.g., Mizuguchi et al. (2004) Hum. Gene Ther.15:1034-44; Eto et al. (2008) Int. J. Pharm., 354:3-8).

Any of the above adenoviruses, or any known in the art, can be modified to contain a desired heterologous nucleic acid molecule for use as a delivered agent herein. The adenovirus containing the desired heterologous nucleic acid sequence can be prepared by any technique known to persons skilled in the art (Levrero et al., Gene 1991;101(2):195-201, EP185 573; Graham, EMBO.J. 3 (1984) 2917; WO95/26411). In particular, such viruses can be prepared by homologous recombination between an adenovirus vector and a plasmid carrying the heterologous DNA sequence. The homologous recombination can occur after co-transfection of the adenovirus vector and plasmid into an appropriate cell line. The cell line used is generally one that is transformable. The transfection can be performed in the presence of a reagent that directs adenoviral particle entry into producer cells. Such reagents include, but are not limited to, polycations and bifunctional reagents.

In some embodiments, if the adenovirus is a defective adenovirus (due to deletion of an early gene, e.g., E1 and/or E3, or fiber protein), the cell line the adenovirus is packaged or grown in contains the sequences capable of complementing the defective adenovirus genome part, such as in integrated form in order to avoid risks of recombination. Examples of complementing cell lines include, but are not limited to, the human embryonic kidney line 293 (HEK293) (Graham et al., J Gen Virol. 1977; 36(1):59-74) which contains the left-hand part of the genome of an Ad5 adenovirus. A complementing cell also includes, for example, a cell of the PER.C6 cell line, which contains the adenoviral E1 gene (PER.C6 is available, for example, from Crucell, the Netherlands; deposited under ECACC accession no. 96022940; see, also Fallaux et al. Hum Gene Ther. 1998; 9(13):1909-17; U.S. Pat. No. 5,994,128). Another example of a complementing cell line is the A549-derived cell line, AE1-2a (see e.g., Gorziglia et al. J Virol. 1996; 70(6):4173-4178 and Von Seggern et al. (1998) J. Gen. Virol. 1998; 79, 1461-1468). In some embodiments, the adenoviruses which have multiplied in a complementing cell or cell line are recovered and purified according to conventional molecular biology techniques.

References illustrating the use of adenoviruses in gene therapy include, but are not limited to Vorburger and Hunt, The Oncologist 2002;7:46-59 and St. George, Gene Therapy 2003; 10:1135-1141.

### b. Adeno-Associated Virus (AAV)

Viral vectors for use as delivery agents include adeno-associated virus (AAV). AAV is a single-stranded human DNA parvovirus whose genome has a size of 4.6 kb. The AAV genome contains two major genes: the rep gene and the cap gene. The rep gene codes for the rep proteins (Rep. 76, Rep. 68, Rep. 52 and Rep 40). The cap gene codes for AAV replication, rescue, transcription and integration, while the cap proteins form the AAV viral particle. AAV derives its name from its dependence on an adenovirus or other helper viruses (e.g., herpesviruses) to supply essential gene products that permit AAV to undergo a productive infection (i.e., reproduce itself in the host cell). In the absence of helper virus, AAV integrates as a provirus into the host cell's chromosome until it is rescued by superinfection of the host cell with a helper virus, usually adenovirus (Muzyczka, Curr. Top. Micro. Immunol. 1992;158:97-129).

AAV viruses can be integrated into the cellular genome. The mechanism of integration is mediated by the presence of inverted terminal repeat (ITRs) at both ends of the AAV genome, which contain cis-acting nucleotide sequences required for virus replication, rescue, packaging and integration. The integration function of the ITR mediated by the rep protein in trans permits the AAV genome to integrate into a cellular chromosome after infection in the absence of helper virus. The site of integration for AAV is well-established and has been localized to chromosome 19 of humans (Kotin et al., PNAS 1990;87:2211-2215). Knowledge of the integration site reduces the danger of random insertional events into the cellular genome that can activate or inactivate host genes or interrupt coding sequences. AAV is also useful for gene therapy applications because its host range is broad, exhibiting tropism for many cell types. AAV can also infect both non-dividing and dividing cells.

AAV vectors can be derived from any naturally occurring AAV serotype, including AAV-1, AAV-2, AAV-3, AAV-4, AAV-5, AAV-6, AAV-7, AAV-8 or AAV-9. Such viruses are well known and available to one of skill in the art (see e.g. Grimm et al. (2003) Current Gene Therapy, 3:28.1- 304; Muramatsu et al. (1996) Virol., 221:208-217; Chiorini et al. (1997) J. Virol. 71:6823-6833; Chiorini (1999) J. Virol., 73: 1309-1319; Rutledge et al. (1998) J. Virol, 72:309-319; Xiao et al. (1999). Virol., 73:3994-4003; Gao et al. (2002) Proc Natl. Acad. Sci., 99:1 1854-11859; Kotin (1994) Human Gene Therapy, 5:793-801). Other serotypes also are known and available and include AAV-8 to AAV-12. For example, many AAV vectors are available from American Type Culture Collection (ATCC, Rockville, Md.; see e.g. VR-197, VR-645, VR-646, VR-680, VR-681, VR-1449, VR-1523, VR-1616). Also available are compatible host cells and helper virus. AAV vectors also include "pseudotyped" AAV vectors, in which the AAV-2 vector genome is cross-packaged into the capsids of the other AAV serotypes (Burger et al., Mol Ther. 2004; 10(2):302-17 and U.S. Pat. No. 7,094,604). Such pseudotyped AAV vectors overcome limitations of AAV-2-derived serotypes, such as their inefficiency in transducing some cells, such as liver or muscle cells.

Many AAV vectors exhibit widespread transduction throughout multiple tissues, such as skeletal and cardiac muscles, following delivery methods that achieve systemic expression. These include, for example, AAV serotypes-6, -8 and -9. In particular, AAV vectors include an adenovirus associated serotype 9 (AAV-9; GenBank Accession No. AY530629.1; Gao et al. J. Virol., 2004; 78:6381-6388). AAV-9 is a vector that can bypass the blood brain barrier to target the central nervous system (CNS) (see e.g. Foust et al., Nature Biotechnology, 2009; 27:59-65; Duque et al. Mol. Ther:, 2009; 17:1187-1196). Hence, in examples of neurodegenerative diseases or other diseases herein that affect or are associated with the brain or CNS, AAV-9 can be used as the delivered agent to encode a protein of interest for delivery systemically (e.g. delivery to the liver or portion thereof for expression in the blood).

AAV vectors include recombinant AAV vectors that contain a heterologous nucleic acid of interest. Procedures for generating such vectors are known to one of skill in the art. For example, standard approaches to the generation of AAV vectors requires transfection of a host cell with an AAV vector genome containing a nucleic acid molecule of interest flanked by the AAV ITR sequences, transfection of the host cell by a plasmid encoding the genes for the AAV rep and cap proteins that are required in trans, and infection of the transfected cell with a helper virus to supply the non-AAV helper functions required in trans (Muzyczka Curr. Top. Micro. Immunol., 1992; 158:97-129 and U.S. Pat. No. 5,139,941). The helper virus can be an adenovirus or other helper virus. The helper virus proteins activate transcription of the AAV rep gene, and the rep proteins then activate transcription of the AAV cap genes. The cap proteins then utilize the ITR sequences to package the AAV genome into a viral particle.

Alternatively, recombination of AAV virions can be helped using a plasmid containing helper function genes, in combination with infection by one of the well-known helper viruses that can be used as the source of replicative functions (see e.g. U.S. Pat. No. 5,622,856 and 5,139,941). Similarly, the skilled artisan can make use of a plasmid containing accessory function genes, in combination with infection by wild-type AAV, to provide the necessary replicative functions. A triple transfection method also can be used to produce recombinant virions (rAAV), which is a method that does not require helper virus (see e.g., U.S. Pat. No. 6,001,650). This is accomplished by use of three vectors for rAAV virion production: an AAV helper function vector, an accessory function vector, and a rAAV vector.

References illustrating the use of AAV viruses in gene therapy include, but are not limited to, Sheridan, Nature Biotechnology 2011; 29:121-128.

### c. Retroviral Vectors

Viral vectors for use as a delivery agents include a retroviral vectors. Retroviral vectors are well suited for delivering nucleic acid into cells because of their ability to deliver an unrearranged single copy gene into a broad range of rodent, primate and human somatic cells. Retroviral vectors integrate into the genome of host cells. Unlike other viral vectors, they only infect dividing cells. Retroviruses are RNA viruses such that the viral genome is RNA. When a host cell is infected with a retrovirus, the genomic RNA is reverse transcribed into a DNA intermediate, which is integrated very efficiently into the chromosomal DNA of infected cells. This integrated DNA intermediate is referred to as a provirus. Transcription of the provirus and assembly into infectious virus occurs in the presence of an appropriate helper virus or in a cell line containing appropriate sequences permitting encapsulation without coincident production of a contaminating helper virus. A helper virus is not required for the production of the recombinant retrovirus if the sequences for encapsulation are provided by co-transfection with appropriate vectors.

The retroviral genome and the proviral DNA have three genes: the gag, the pol and the env, which are flanked by two long terminal repeat (LTR) sequences. The gag gene encodes the internal structural (matrix, capsid, and nucleocapsid) proteins and the env gene encodes viral envelope glycoproteins. The pol gene encodes products that include the RNA-directed DNA polymerase reverse transcriptase that transcribes the viral RNA into double-stranded DNA, integrase that integrate the DNA produced by reverse transcriptase into host chromosomal DNA, and protease that acts to process the encoded gag and pol genes. The 5' and 3' LTRs serve to promote transcription and polyadenylation of the virion RNAs. The LTR contains all other cis-acting sequences necessary for viral replication.

Retroviral vectors are described by Coffin et al., Retroviruses, Cold Spring Harbor Laboratory Press (1997). Exemplary of a retrovirus is Moloney murine leukemia virus (MMLV) or the murine stem cell virus (MSCV). Retroviral vectors can be replication-competent or replication-defective. Typically, a retroviral vector is replication-defective in which the coding regions for genes necessary for additional rounds of virion replication and packaging are deleted or replaced with other genes. Consequently, the viruses are not able to continue their typical lytic pathway once an initial target cell is infected. Such retroviral vectors, and the necessary agents to produce such viruses (e.g., packaging cell line) are commercially available (see e.g. retroviral vectors and systems available from Clontech, such as Catalog number 634401, 631503, 631501, and others, Clontech, Mountain View, Calif.).

Such retroviral vectors can be produced as delivery agents by replacing the viral genes required for replication with the nucleic acid molecule to be delivered. The resulting genome contains an LTR at each end with the desired gene or genes in between. Methods of producing retrovirus are known to one of skill in the art (see e.g. WO 1995/26411). The retroviral vector can be produced in a packaging cell line containing a helper plasmid or plasmids. The packaging cell lines provide the viral proteins required for capsid production and the virion maturation of the vector (e.g., gag, pol and env genes). Typically, at least two separate helper plasmids (separately containing the gag and pol genes; and the env gene) are used so that recombination between the vector plasmid cannot occur. For example, the retroviral vector can be transferred into a packaging cell line using standard methods of transfection, such as calcium phosphate mediated transfection. Packaging cell lines are well known to one of skill in the art, and are commercially available. An exemplary packaging cell line is GP2-293 packaging cell line (Catalog Numbers 631505, 631507, 631512, Clontech). After sufficient time for virion production, the virus is harvested. If desired, the harvested virus can be used to infect a second packaging cell line, for example, to produce a virus with varied host tropism. The end result is a replicative incompetent recombinant retrovirus that includes the nucleic acid of interest but lacks the other structural genes such that a new virus cannot be formed in the host cell.

References illustrating the use of retroviral vectors in gene therapy include: Clowes et al., Clin. Invest. 1994;93:644-651; Kiem et al., Blood 1994;83:1467-1473; Salmons and Gunzberg, Human Gene Therapy 1993;4:129 141; Grossman and Wilson, Curr. Opin. in Genetics and Devel. 1993;3:110-114; Sheridan, Nature Biotechnology 2011;29:121; Cassani et al., Blood 2009;114:3546-3556.

### d. Lentiviral Vectors

Lentiviruses are a subclass of retroviruses. Exemplary lentiviruses are human immunodeficiency virus (HIV), simian immunodeficiency virus (SIV), and feline immunodeficiency virus (FIV). Unlike other retroviruses, lentiviruses are able to integrate into the genome of non-dividing cells. Thus, for example, lentiviral vectors have been reported to deliver genes to primary livers cells efficiently and permanently, integrating into the genome of non-dividing primary liver cells (Lewis and Emerman, J. Virol. 1994;68:510-6). Lentiviral vectors also do not suffer from the same transcription silencing mechanism as MMLV retroviral vectors. Lentiviruses differ from other retroviruses in that they have karyophilic determinants contained in several virion proteins, such as matrix or VPR, which interact with the nuclear import machinery and mediate active transportation of the viral pre-integration complex through the nucleopore. Therefore, lentiviral integration into the genome of the host cells is not dependent on cell division.

Similar to other retroviruses, lentiviruses contain gag, pol and env genes that are the main genes coding for viral proteins. In addition, there also are other accessory genes that are involved in regulation of synthesis, processing of viral RNA and other replicative functions (e.g., Tat and Rev in HIV). These are flanked by two long terminal repeat (LTR) sequences. The replication cycle is initiated by binding of a viral glycoprotein to a host cell receptor, fusion of the membranes, and entry of the virus into the cell. Upon entry the virus is uncoated and reverse transcription takes place leading to the formation of a pre-integration complex (PIC). It is the other accessory genes that play a role in the formation of a PIC and the ability of lentiviruses to infect non-dividing cells by actively entering the nucleus of a cell through the nuclear envelope via the PIC. Once the provirus enters the nuclear envelope, it integrates itself into the host genome.

Exemplary lentivirus vectors are based on HIV-1, HIV-2, SIV or FIV. In order to generate safe lentiviral vectors, packaging cell lines are created that contain several plasmid vectors, for example a four plasmid vector system. For example, a first plasmid contains accessory proteins (e.g., tat, brf, vpr and nef) deleted such that it contains only the promoter, gag and pol and the Psi packaging sequence that allows the transcribed viral RNA to be incorporated into the assembly of new virus, a second plasmid contains the reverse transcriptase, a third plasmid contains the env gene replaced with the Vesicular Stomatitis Virus Envelope Protein (VSV-G), and a fourth plasmid is the vector of interest by replacing the viral genes required for replication with the nucleic acid molecule to be delivered.

Such lentiviral vectors, and systems and methods of producing lentivirus, are known in the art (see e.g., Buchshacher and Wong-Staal, Blood 2000; 95:2499-2504; Blomer et al., J. Virol. 1997;71:6641-9; Choi et al., Stem Cells 2001;19:236-46; U.S. Pat. No. 6,218, 186). The lentiviral vectors are replication defective and do not contain the genes required for replication. To produce a lentivirus, several packaging plasmids are transfected into a packaging cell line, generally derivatives of HEK 293 or other similar cell line (e.g. 293FT cells, Catalog number R700-07, Invitrogen, Life Technologies, Carlsbad, Calif.); 293LTV cell line, catalog number LTV-100, Cell Biolabs, Inc., San Diego, Calif.; Lenti Pac 293Ta Cell Line, Catalog Number CLV-PK-01, GeneCo poeia, Rockville, Md.). The packaging plasmids separately encode virion proteins (e.g., capsid and reverse transcriptase) and the nucleic acid molecule to be delivered by the vector (which can be transfected into the packaging cell lines). A single-stranded RNA viral genome is transcribed, which is packaged into the virion. Methods of generating lentiviral vectors are well known to one of skill in the art (see e.g., Naldine et al., Science 1996;272:263-267). Lentiviral vectors and systems for producing virus are commercially available (see e.g., Lentiviral expression vectors such as pSMPUW Lentiviral vector and derivatives thereof and Lentiviral Expression and Packaging Systems available from Cell Biolabs, Inc.).

Lentiviral vectors have been used in gene therapy applications (see e.g., Manilla et al., Human Gene Therapy 2005;16:17-25; Sheridan, Nature Biotechnology 2011;29:121). In particular, lentiviral vectors have been used for the delivery of short-interfering RNA (siRNA) (Sachdeva et al., Journal of Medical Virology 2007;79:118-26).

### C. Cryopreservation and Storage

In some aspects, provided herein is a method of manufacturing a skin substitute, wherein the method comprises: 1) differentiating keratinocytes into a stratified epidermis, wherein the stratified epidermis comprises a basal layer, a spinous layer, a granular layer and a stratum corneum; and 2) introducing a polynucleotide into cells of the stratified epidermis to produce a skin substitute, wherein the skin substitute comprises a growth factor and insulin. In some aspects, provided herein is a method of manufacturing a skin substitute, wherein the method comprises: 1) differentiating keratinocytes into a stratified epidermis, wherein the stratified epidermis comprises a basal layer, a spinous layer, a granular layer and a stratum corneum; and 2) transducing a viral vector comprising a polynucleotide into cells of the stratified epidermis to produce a skin substitute, wherein the skin substitute comprises a growth factor and insulin. In some embodiments, the methods provided herein further comprise methods of cryopreserving and storing a skin substitute comprising a stratified epidermis, the cells of which produce, e.g., secrete, a growth factor and insulin. In some embodiments, the methods provided herein further comprise methods of performing a quality control assessment on the skin substitute prior to cryopreservation and storage.

In some embodiments, the skin substitute comprising a stratified epidermis is cryopreserved. In some embodiments, the skin substitute comprising a stratified epidermis is formulated with a cryoprotectant prior to cryopreservation. In some embodiments, the cryoprotectant comprises albumin and a monosaccharide. In some embodiments, the cryoprotectant comprises human albumin and a glucose, e.g., D-glucose. In some embodiments, the cryoprotectant does not comprise DMSO.

In some embodiments, a quality control assessment is performed prior to formulating the skin substitute comprising a stratified epidermis with the cryoprotectant. The quality control assessment can include, but is not limited to, identification and/or detection of certain proteins, e.g., markers of epidermal differentiation, assessments of potency, and assessments of purity. The quality control assessments can further include assessments of sterility and safety.

In some embodiments, the quality control assessment comprises identifying and/or detecting genes associated with a viral genome of the vector used for transduction. In some embodiments, the quality control assessment comprises identifying and/or detecting genes associated with an adenoviral vector. In some embodiments, adenoviral genes are identified and/or detected to distinguish a replication-incompetent virus from a replication-competent virus. In some embodiments, the quality control assessment comprises identifying and/or detecting levels of adenoviral genes E1, E4. In some embodiments, the quality control assessment comprises identifying and/or detecting levels of molecules associated with the skin substitute, e.g., a recombinant growth factor, recombinant insulin, filaggrin, laminin, and transglutaminase using methods known to those of skill in the art, such as by PCR, e.g., qPCR, and/or immunohistochemistry.

In some embodiments, the quality control assessment comprises detecting molecules secreted from the skin substitute, e.g., a recombinant growth factor and C-peptide, such as with use of ELISA. In some embodiments, the quality control assessment comprises evaluating the potency of a recombinant growth factor and recombinant insulin secreted from the skin substitute, such as with use of an angiogenesis assay, e.g., an endothelial tube formation assay. In some embodiments, the quality control assessment comprises an assessment of purity, e.g., the purity of bovine collagen, using methods known to one of skill in the art. In some embodiments, the quality control assessment comprises an assessment of sterility, such as by detecting endotoxin using methods known to one of skill in the art, e.g., PCR. In some embodiments, all components of a skin substitute as provided herein undergo screening for infectious agents. In some embodiments, keratinocyte, e.g., HaCaT, master cell banks are screened for tumorigenicity and chromosomal abnormalities.

In some embodiments, the packing or storage of the cryopreserved skin substitute comprises use of a dressing, e.g., an absorbent material such as an absorbent gauze, wherein the cryopreserved skin substitute is overlaid on the dressing. In some embodiments the size of the cryopreserved skin substitute overlaid on the dressing, e.g., gauze, is about 30-55 cm², about 30-50 cm², about 35-45 cm², about 40-50 cm², about 40-45 cm², or about 45-50 cm². In some embodiments, the size of the cryopreserved skin substitute overlaid on the dressing, e.g., gauze, is at or about 30 cm², at or about 31 cm², at or about 32 cm², at or about 33 cm², at or about 34 cm², at or about 35 cm², at or about 36 cm², at or about 37 cm², at or about 38 cm², at or about 39 cm², at or about 40 cm², at or about 41 cm², at or about 42 cm², at or about 43 cm², at or about 44 cm², at or about 45 cm², at or about 46 cm², at or about 47 cm², at or about 48 cm², at or about 49 cm², or at or about 50 cm².

In some embodiments, the packing or storage of the cryopreserved skin substitute comprises use of an absorbent gauze, e.g., wherein the cryopreserved skin substitute is overlaid on the absorbent gauze. In some embodiments, the dressing comprises an absorbent gauze. In some embodiments, the absorbent gauze is a Vaseline Petrolatum gauze. In some embodiments, the size of the dressing, e.g., gauze, upon which the skin substitute is laid upon is about 40-60 cm², about 45-60 cm², about 45-55 cm², or about 50-60 cm². In some embodiments, the size of the dressing, e.g., gauze, upon which the cryopreserved skin substitute is laid upon is at or about 40 cm², at or about 41 cm², at or about 42 cm², at or about 43 cm², at or about 44 cm², at or about 45 cm², at or about 46 cm², at or about 47 cm², at or about 48 cm², at or about 49 cm², at or about 50 cm², at or about 51 cm², at or about 52 cm², at or about 53 cm², at or about 54 cm², at or about 55 cm², at or about 56 cm², at or about 57 cm², at or about 58 cm², at or about 59 cm², or at or about 60 cm².

In some embodiments, the ratio of the size of the dressing, e.g., absorbent gauze to the size of the cryopreserved skin substitute overlaid upon the dressing is about 1:1 to about 1.5:1. In some embodiments, the ratio of the size of the dressing, e.g., absorbent gauze to the size of the cryopreserved skin substitute overlaid upon the dressing is about 1:1, about 1.1:1, about 1.2:1, about 1.3:1, about 1.4:1, or about 1.5:1.

In some embodiments, the cryopreserved skin substitute is packaged or stored in a container. In some embodiments, the cryopreserved skin substitute overlaid on a sterile dressing is packaged or stored in a container. In some embodiments, container is sterile. In some embodiments, container is sealed using heat, e.g., heat-sealable or heat-sealed. In some embodiments, container is sterile and heat-sealable or heat-sealed. In some embodiments, the container is clear. In some embodiments, the container comprises polyester resin. In some embodiments, the container is a bag. In some embodiments, the skin substitute packaged or stored in the container in enclosed by a package. In some embodiments, one or both of the container and the package is(are) sterile and/or heat-sealable. In some embodiments, the cryopreserved skin substitute, optionally stored in a container, can be stored at about -20°C, - 30°C, -40°C, -50°C, -60°C, -70°C, or -80°C.

In some embodiments, the cryopreserved skin substitute can be stored for up to six months. In some embodiments, the cryopreserved skin substitute can be stored for up to six months and retain the function of providing, e.g., secreting, a recombinant growth factor and recombinant insulin at effective amounts. In some embodiments, the cryopreserved skin substitute can be stored for up to five months and retain the function of providing, e.g., secreting, a recombinant growth factor and recombinant insulin at effective amounts. In some embodiments, the cryopreserved skin substitute can be stored for up to four months and retain the function of providing, e.g., secreting, a recombinant growth factor and recombinant insulin at effective amounts. In some embodiments, the cryopreserved skin substitute can be stored for up to three months and retain the function of providing, e.g., secreting, a recombinant growth factor and recombinant insulin at effective amounts.

### IV. METHODS OF USING A SKIN SUBSTITUTE

In some aspects, provided herein are methods of using a skin substitute comprising a stratified epidermis, the cells of which produce, e.g., secrete, a growth hormone and insulin, or a variant thereof. In some embodiments, the skin substitutes provided herein can be applied to a subject, e.g., a human subject, to improve the condition of a wound and/or promote wound healing. In some embodiments, the skin substitutes provided herein can be applied to a diabetic subject to promote wound healing and/or prevent microbial infection of a wound.

### A. Advanced Glycation End-Products

In some embodiments, a skin substitute as provided herein can be used to improve a condition involving advanced glycation end-products (AGEs), such as compromised or prolonged wound healing. An elevated concentration of reducing sugars (e.g., glucose) in the blood and in the intracellular environment of an animal, namely a human, typically results in the non-enzymatic formation of glycation and dehydration condensation complexes, AGEs. AGE complex products form on free amino groups, on proteins, on lipids and on DNA (Bucala and Cerami, Adv Pharmacol 1992;23:1-34; Bucala et al., Proc Natl Acad Sci 1993;90:6434-6438; Bucala et al., Proc Natl. Acad Sci 1984;81:105-109). In one example, AGE levels in diabetic patients increase markedly as a result of sustained high blood sugar levels and often leads to tissue damage through a variety of mechanisms, including alteration of tissue protein structure and function, stimulation of cellular responses through AGE specific receptors and/or the generation of reactive oxygen species (ROS) (Boel et al., J Diabetes Complications 1995;9:104-29). AGEs have been shown to cause complications in patients suffering from diabetes mellitus who experience wounds such as nicks, cuts, burns, sores, ulcers, abscesses and/or any other form of bodily injury. In some embodiments, a growth factor and insulin secretable from a skin substitute as provided herein can promote angiogenesis and reduce the amount of AGEs in the skin of a subject, without affecting systemic glucose levels.

The accumulation of AGEs can lead to diminished function of the skin, especially as it pertains to wound healing in diabetic patients (Putte et al., Scars Burn Heal. 2016; 5(2):1-14). AGEs are implicated in delayed or defective wound healing of the skin (Peppa et al., Diabetes 2003; 52(11):2805-2813) and even the bone (Santana et al., Diabetes 2003;52(6):1502-10) of diabetic subjects. In delayed or prolonged wound healing, the time for a wound to heal, as indicated by wound closure, in some cases, is extended beyond the time to healing observed in a healthy or non-diabetic subject. In some embodiments, a skin substitute as provided herein can reduce wound healing time (e.g., time to wound closure) in a diabetic subject to a comparable amount of time necessary for the same effect to be achieved in a non-diabetic subject.

In some cases, defective wound healing refers to abnormalities in epithelial organization during the wound healing process, which can include a reduced ability to form scars. Scar formation is a major part of wound healing. Scars are areas of fibrous tissue that form during the wound healing process in place of the normal skin that was present prior to the wound formation. A scar exhibits an altered extracellular matrix and has a reduced level of elastin fibers relative to normal skin. On healthy skin, nearly every wound results in some degree of scarring. In some embodiments, a skin substitute as provided herein can induce scar formation, thereby facilitating the wound healing process.

Wounds can be described as acute or chronic. Acute wounds are typically the result of an injury to the skin that occurs suddenly rather than over time (e.g., a surgical wound or a traumatic wound). In normal subjects, acute wounds typically heal at predictable and expected rates according to the normal wound healing process. In contrast, chronic wounds are wounds that fail to progress through the phases of wound healing in an orderly and timely fashion (e.g., showing no significant progress towards healing in 30 days). In some embodiments, a skin substitute as provided herein can be applied to an acute wound. In some embodiments, a skin substitute as provided herein can be applied to a chronic wound. Non-limiting examples of chronic wounds include venous ulcers, diabetic foot ulcers, and pressure ulcers. Non-healing wounds present a major healthcare burden. Non-healing wounds can result in prolonged hospital stays, diminished quality of life, increased risk of mortality, need for amputation, and increased likelihood of being discharged to a long-term care facility.

### B. Diabetic Foot Wounds and Infections

Diabetic foot wounds, including ulcers, sores, lesions and/or abscesses, stem from two common complications of diabetes, peripheral neuropathy and vascular insufficiency. Diabetic foot disease, referred to hereinafter as diabetic foot, is a condition with high morbidity, which negatively impacts a patient's quality of life due in part to frequent hospital admissions, longer hospital stays, and amputations (Alosaimi et al., Journal of Foot and Ankle Research 2019; 12:57). It is estimated that one in ten people diagnosed with type 2 diabetes (mellitus) have risk factors for foot lesions (Boulton et al., Lancet 2005; 366(9498):1719-24).

About 15% to 25% of all diabetic patients will develop a foot or leg ulcer during the course of their disease (Boulton et al., Lancet 2005; 366(9498):1719-24, and Pinzur and Dart, Foot Ankle Clin. 2001;6(2):205-142005). Risk factors include long-term diabetes mellitus (>10 years), age (>50 years), a history of ulcer or amputation, the presence of neuropathy, arthropathy, or vascular disease, the presence of other diabetes complications, low socioeconomic status of the patient and/or social isolation, poor diet, poor education on foot care, and other risk factors associated with vascular disease (Nongmaithem et al., J Family Med Prim Care 2016;5(2):399-403).

A diabetic foot diagnosis carries the risk of injuries and/or amputations. Wounds or lesions especially threaten high-risk patients, including smokers and those with previous vascular complications of lower extremities. Early detection of diabetic foot is performed through a foot exam, including the use of monofilament, palpation, visual and sensitivity examination. If preventive measures fail and injury occurs or a high-risk foot is confirmed, a multidisciplinary management should be performed, especially in those cases with a history of ulcer or amputation of the other extremity.

The Wagner classification (which grades lesions on a scale from 0 to 5, where 5 indicates the most severe disease) is widely used to determine management strategies for the neuropathic diabetic foot. The primary goal of treatment is wound closure. Management at a primary, less severe level may include resting, foot elevation and oral antibiotic therapy, if required. If no response is observed to these measures, the patient should be referred for more aggressive interventions. Subjects having lesions that do not respond to initial treatment and/or present a severe (high grade) wound and/or infection are recommended for surgical intervention and/or intravenous antibiotic therapy (Frykberg, Am Fam Physician. 2002;66(9):1655-1663).

**Table 1 Wagner Diabetic Foot Ulcer Grade Classification System**

| GRADE | LESION |
|---|---|
| 0 | No open lesions; may have deformity or cellulitis |
| 1 | Superficial diabetic ulcer (partial or full thickness) |
| 2 | Ulcer extension to ligament, tendon, joint capsule, or deep fascia without abscess or osteomyelitis |
| 3 | Deep ulcer with abscess, osteomyelitis, or joint sepsis |
| 4 | Gangrene localized to portion of forefoot or heel |
| 5 | Extensive gangrenous involvement of the entire foot |

| | |
|---|---|
| *From Frykberg, Am Fam Physician. 2002;66(9):1655-1663 | |

Currently available skin substitutes or skin equivalents are of a variety of different compositions, e.g., keratinocyte stem cells, normal immortalized keratinocytes (NIKS), and/or human fibroblasts, and structures, e.g., a composition comprising a structure in the form of an epidermal layer or a composition comprising a structure in the form of a dermal and an epidermal layer. These skin substitutes can secrete a range of different molecules, e.g., growth factors, collagen, and/or extracellular matrix proteins. For example, Dermagraft^{®} and Apligraf^{®} are skin substitutes that are FDA-approved to treat wounds in diabetic patients, e.g., diabetic foot ulcers and/or venous leg ulcers. However, currently available skin substitutes have many limitations, including requirement for several applications coupled with high cost. In some embodiments, a skin substitute as provided herein can reduce wound healing time in diabetic subjects to a comparable amount of time necessary to achieve the same effect in non-diabetic subjects with only one application. In some embodiments, a skin substitute as provided herein can reduce wound healing time in diabetic subjects to a comparable amount of time necessary to achieve the same effect in non-diabetic subjects with only two applications. In some embodiments, a skin substitute as provided herein can reduce wound healing time in diabetic subjects to a comparable amount of time necessary to achieve the same effect in non-diabetic subjects with only three applications. In some embodiments, a skin substitute as provided herein can reduce wound healing time in diabetic subjects to a comparable amount of time necessary to achieve the same effect in non-diabetic subjects with only four applications.

In some embodiments, a skin substitute as provided herein is applied to the skin or the wound of a subject. In some embodiments, a skin substitute overlaid on an absorbent dressing, e.g., gauze, as provided herein is applied to the skin or the wound of a subject. In some embodiments, a skin substitute as provided herein can be changed once every 10 days, once every 11 days, once every 12 days, once every 13 days, once every 14 days, once every 15 days, once every 16 days, once every 17 days, once every 18 days, once every 19 days, once every 20 days, or once every 21 days. In some embodiments, a skin substitute as provided herein can remain applied to a subject, unchanged and undisturbed for at least 10 days, at least 11 days, at least 12 days, at least 13 days, at least 14 days, at least 15 days, at least 16 days, at least 17 days, at least 18 days, at least 19 days, at least 20 days, or at least 21 days.

In some embodiments, a skin substitute as provided herein can provide sustained release of a recombinant growth factor and recombinant insulin. In some embodiments, sustained release of a recombinant growth factor and recombinant insulin from a skin substitute as provided herein persists for up to or about 2 days, up to or about 3 days, up to or about 4 days, up to or about 5 days, up to or about 6 days, up to or about 7 days, up to or about 8 days, up to or about 9 days, up to or about 10 days, up to or about 11 days, up to or about 12 days, up to or about 13 days, or up to or about 14 days. In some embodiments, sustained release of a recombinant growth factor and recombinant insulin from a skin substitute as provided herein persists for up to or about one week, up to or about two weeks, up to or about three weeks, up to or about one to two weeks, or up to or about two to three weeks.

The microbiome of the skin is composed of various viruses, bacteria and fungi, and the wounds of diabetic subjects are particularly prone to infection. The mere presence of microbes does not indicate infection. Infection must be diagnosed clinically, by the presence of systemic signs (e.g., fever, chills, and leukocytosis), pus or secretions, or local symptoms of inflammation (e.g., warmth, redness, pain or tenderness, and induration).

Complicating diagnosis of infections, chronic wounds can additionally display delayed healing, abnormal coloration, friability, or foul odor. Infection should be suspected at the first appearance of a foot problem and evidence of a systemic infection or of a metabolic disorder. Vascular abnormalities, including peripheral neuropathy or ischemia, can either mask or mimic inflammation (Lipsky, Clinical Infectious Diseases 2004;39:S104-14). Interestingly, signs of systemic toxicity, e.g., sepsis, systemic inflammatory response syndrome (SIRS), or multiple organ dysfunction syndrome (MODS), are rarely reported in diabetic foot infections. However, when, for example, SIRS is evident, a diabetic foot infection has a higher likelihood of becoming not only limb threatening but life threatening (Lin et al., J. Clin. Med. 2019;8(10):1538). A suspected infection should be pursued aggressively, especially considering how quickly the severity can escalate, sometimes in a few hours.

In one example, diabetic foot wounds can result in infections ranging from superficial to severe, wherein the infection spreads to deeper layers of the skin and/or bone. Infections are a risk factor for surgical intervention, which can include minor, foot sparing operations, or major surgery, such as amputation. By one estimate, 60% of amputations are preceded by an infected foot ulcer (Lipsky, Clinical Infectious Diseases 2004; 39:S104-14). Methods of preventing an infection are of utmost importance given the severe consequences to the patient. Further, prevention avoids the need for antibiotic therapy, which can be costly and incur harmful, off-target effects to the patient.

**In** some embodiments, a skin substitute as provided herein can be used to prevent a microbial infection. **In** some embodiments, a skin substitute as provided herein can be used to prevent a bacterial infection. **In** some embodiments, a skin substitute as provided herein can be used to prevent a viral infection. **In** some embodiments, a skin substitute as provided herein can be used to prevent a microbial infection of one or more wound(s) of a subject. **In** some embodiments, a skin substitute as provided herein can be used to prevent a bacterial infection of one or more wound(s) of a subject. In some embodiments, a skin substitute as provided herein can be used to prevent a viral infection of one or more wound(s) of a subject. In some embodiments, a skin substitute as provided herein can be used to prevent a microbial infection of one or more wound(s) of a diabetic subject. In some embodiments, a skin substitute as provided herein can be used to prevent a bacterial infection of one or more wound(s) of a diabetic subject. In some embodiments, a skin substitute as provided herein can be used to prevent a viral infection of one or more wound(s) of a diabetic subject.

### V. EXAMPLES

The following examples are included for illustrative purposes only and are not intended to limit the scope of the invention.

### Example 1 : Differentiation of Keratinocytes Into Epidermis and Methods for Adenoviral Transduction

This Example describes generation of a skin substitute by culture of human keratinocytes from the cellular HaCaT line (human keratinocytes immortalized by spontaneous mutation) and their differentiation until all of the epithelium layers were obtained (basal, spinous, granulose and corneous layers), followed by transduction with an adenoviral vector for transgene expression from the differentiated epithelial layer.

Prior to differentiation, HaCaT cells were kept in a low calcium (0.03 mM) media for 4 weeks in order to change the cellular characteristics to obtain a basal layer that was amenable to transduction with a type 5 adenovirus. For differentiation, HaCaT cells were seeded in 75 mm² culture bottles then subcultured in a 1:4 proportion once they reached approximately 80%confluency. Cells were cultured in serum free and calcium free keratinocyte medium (ThermoFisher, Cat. No. 37010022) modified by addition of calcium to a final concentration of 0.03 mM, and supplemented with 0.2 ng/mL endothelial growth factor (EGF) and 30 µg/mL bovine pituitaries extract (BPE).

Transwell^{®} polyester inserts (3 µM pores and 75 mm diameter) were covered with 23.5 mL of a neutralized bovine collagen solution containing human use certified bovine collagen (2.5 mg/mL) that had been neutralized with 1M NaOH to reach pH 7.4. The inserts covered with the neutralized bovine collagen solution were incubated for 45 minutes at 37° C. Once the collagen jellified, two washes with 1X PBS were performed. Serum free and calcium free keratinocyte medium supplemented with 0.03 mM of calcium, 0.2 ng/mL EGF, and 30 µg/ml BPE was added above and below the transwell insert for cell seeding. Cultured HaCaT cells (20 x 10⁶) were then seeded in the low calcium medium, which was replaced daily for 4 days.

At day 5, medium above the insert was discarded, and the low calcium medium was exchanged for supplemented serum free keratinocyte medium having 0.2 ng/mL EGF, 30 µg/mL bovine pituitary extract, 0.4 µg/mL hydrocortisone, and 2.4 mM calcium. An air-liquid interface was introduced so that only the basal area of cells was in contact with the medium, while the top of the cell surface was exposed to air. Cells were incubated at 37°C and 5% CO₂, and medium was replaced daily for 21 days. After this period, a completely stratified epidermis with a 150 µM thickness was produced, as shown by staining with eosin and hematoxylin at day 25 in **FIG. 1B****.**

Increasing the concentration of calcium (from 0.01-0.1 mM to 2.4 mM) in the medium increases the presence of occludin and claudin proteins. The expression of occludin and claudins, major transmembrane proteins of the tight junction that influence diffusion across the epithelium, are required for a proper basal-lateral transduction. From Day 5 to Day 21, the skin substitute was kept in high calcium concentrations.

On Day 26, the skin was detached from the insert and removed from the insert with tweezers to obtain the skin substitute, prior to exposing the basal layer to a non-replicative type 5 adenovirus. As a model for transgene expression, the skin substitute was transduced with a non-replicative type 5 adenovirus expressing GFP (Ad-CMV-GFP). Transduction of the skin substitute was performed in serum free medium after washing with 1X PBS. Adenovirus was prepared in the keratinocyte medium before a one hour after incubation at 37°C and 5% CO₂ with the skin substitute.

Evaluation with fluorescence microscopy showed that basal cells were permeable to Ad-CMV-GFP transduction and that the GFP expression co-localized to basal cells of the skin substitute (data not shown). These results indicate that the basal cells of the skin substitute were transduced by the adenovirus, and that the skin substitute epidermis was capable of producing proteins encoded by a transduced adenoviral vector.

24 hours after transduction, the skin substitute was formulated for packing by adding a cryoprotectant medium, placing the skin substitute on a Vaseline gauze and placing the gauze pack in a container. The cryoprotectant medium was composed of human albumin (0.02 g/mL) and D-glucose (0.09 g/mL) added to the serum free keratinocyte medium. The container can then be preserved for up to six months at -20°C. The process for generating and transducing the skin substitute is depicted in **FIG. 1A****.**

### Example 2 : Generation of an Adenoviral Expression Vector Encoding Insulin and VEGF

This Example describes the structure of an adenoviral vector that was used to facilitate protein expression in an engineered skin substitute. The skin substitute described herein was transduced to express and release insulin and VEGF.

A type 5 adenovirus designated Ad-CAG-VEGF-INS was generated with nucleotides encoding human modified proinsulin (SEQ ID NO: 2) and human VEGF (SEQ ID NO: 4) separated by an IRES (SEQ ID NO: 3) for bicistronic expression of the transgenes under control of the CAG promoter (SEQ ID NO: 1). The encoded human VEGF corresponds to isoform 165 (VEGF165; SEQ ID NO: 7). The encoded human modified proinsulin sequence (SEQ ID NO: 6, mature sequence without signal peptide) contains mutations compared to the precursor sequence encoding wild-type proinsulin set forth in SEQ ID NO: 5 (amino acids 25-110 of wild-type human insulin NP_000198.1, encoded by nucleotides 60-389 of wild-type human insulin NM_000207.3). The mutations were included so that the human insulin could be adequately secreted and functional following release from the skin substitute described herein, which is composed of human keratinocytes immortalized by spontaneous mutation (HaCaT cells). Mature insulin is processed by β cells of the pancreas by cleaving between C and B chains (Arg-Arg dibasic site) and cleaving between the C-A chains (Lys-Arg dibasic site). These enzymatic cuts are performed by the endopeptidases PC3 and PC2, respectively, which are not present in the HaCaT cells that form the skin substitute. Thus, these modifications were achieved by generating furin cleavage sites in the desired positions for processing by HaCaT resident enzymes. Specifically, furin consensus sequences (e.g., R-X-[R/K]-R) (SEQ ID NO: 8 or SEQ ID NO: 9, e.g. SEQ ID NO: 10) were introduced in the A-C and B-C junctions, and position 10 of the B-chain was modified from a histidine (H) residue to aspartic acid (D).

The adenovirus was made replication deficient by deleting the E1 and E3 region, and an expression cassette containing the CAG-VEGF-INS sequences was substituted into the E1 region. Specifically, the expression cassette was subcloned into the Dual-Basic adeno-viral shuttle vector and recombined with Ad5 (DE1/DE3) vector (Vector Biolabs, Philadelphia, Pa.). The adenovirus was packaged in HEK293 cells, purified with cesium chloride ultracentrifugation and titrated using the conventional HEK293 plaque assay. **FIG. 2** shows the structure of the adenoviral vector and expression cassette.

### Example 3 : In vitro Evaluation of Transduced Epidermal Cells Reveals Continuous Expression and Release of Insulin and VEGF

This examples describes generation of a skin substitute composed of genetically modified basal keratinocytes that continuously release VEGF-A and insulin. A skin substitute was generated by differentiating human keratinocytes of the cellular HaCaT line by increasing the level of calcium in the culture medium until a completely stratified epidermis was formed, as described in Example 1, followed by transducing cells of the stratified epidermis with the Ad-CAG-VEGF-INS expression construct described in Example 2.

Release of insulin and VEGF from the skin substitute was evaluated in vitro over the span of one week to determine the protein expression and release profiles of the transduced epidermal cells. The level and duration of protein expression are important factors, especially when determining frequency of application.

The adenoviral vector described in Example 2 was used to transduce basal cells of the skin substitute (n=3) at a multiplicity of infection (MOI) of 12, using the methods described in Example 1. The transduced skin substitutes were then cryopreserved and stored in a -20 °C freezer. The transduced skin substitutes were thawed in advance of this experiment. A time course experiment was performed by collecting 100 µL of culture media every 24 hours for seven days. Expression of human insulin and VEGF was determined by ELISA according to the manufacturer's instructions (Invitrogen and Cloud-Clone, respectively). Experiments were performed in triplicate. Specifically, insulin was measured by detecting C-peptide, which is a peptide composed of 31 amino acids that is released during cleavage of insulin from proinsulin (corresponding to residues 33-63 of SEQ ID NO: 5 and 6).

**FIG. 3A** and **FIG. 3B** show the average detected levels of C-peptide and VEGF, respectively, over the span of 7 days. Both proteins remained detectable over the course of the experiment, with observed levels of VEGF less than 4.9 ng/mL and levels of insulin less than 0.49 IU (about 0.0011 pmol/L, based on IU= about 2.247 ng/mL insulin). These results indicate that insulin and VEGF are released from basal cells, and expression of the proteins persists for at least one week. Further, the detected levels are below those that would elicit systemic secondary effects, or side effects, and they may even be considered sub-therapeutic individually.

### Example 4 : In Vitro Evaluation of Synergy Between VEGF and Insulin

Synergy between VEGF and insulin was evaluated at protein levels within range of those measured in Example 3. The study was conducted to determine whether the combination of insulin and VEGF produces a greater therapeutic benefit than either protein alone.

An endothelial cell tube formation assay was performed to evaluate the impact of VEGF and/or insulin on angiogenesis. In this assay, culture of endothelial cells on a basement membrane matrix (Matrigel) leads to the formation of tubular structures resembling the capillary blood vessels characteristic of the angiogenic phenotype. In accordance with the range of detected proteins in Example 3, the activity of 2 ng/mL VEGF in combination with 0.1 UI of insulin was evaluated. To prepare for the experiment, a 96-well plate was covered with 30 µL matrigel (Corning Cat.) diluted in a proportion of 1:1. The plate was incubated for 20 min at 37°C and, once jellified, washed with DMEM-F12 without serum. Approximately 42,000 mouse hemangioendothelioma endothelial cells (EOMA cells) were seeded in 60 µL of DMEM-F12 containing 0.5% of simulated body fluid (SBF). The EOMA cells were incubated for 20 minutes at 37°C 5% CO₂, after which 40 µL of the media was discarded without disturbing adherent cells.

A stimulus or control solution (60 µL) was then added to the cells. Stimuli included supernatants from NIH3T3 cells transfected with adenovirus encoding human VEGF 165 (SEQ ID NO: 7) at an MOI=12, NIH3T3 cells transfected with adenovirus encoding proinsulin modified as described in Example 2 (SEQ ID NO: 6) at an MOI=24, and NIH3T3 cells transfected with a combination of proinsulin and VEGF (24 MOI and 12 MOI, respectively). The negative control was a supernatant of non-transfected NIH3T3 cells, and the positive control was DMEM-F12 with 5% SBF. Cells were incubated at 37°C and 5% CO₂ for 1.5 hours, at which point an additional 60 µL of each supernatant stimulus was added. The experiment was performed in triplicate. After a total of three hours from initial stimulus exposure, at least four pictures were taken of each sample in every group. The Angiogenesis Analyzer for Image J was used to quantify endothelial tube formation.

**FIG. 4A-4C** show the various wound healing features observed in each experimental group. **FIG. 4A** shows the number of webs, which are defined as a closed circuit surrounded by two or more nodes. **FIG. 4B** shows the number of nodes, also referred to as unions, which are defined as bond sites of at least three chords. **FIG. 4C** shows the number of main segments, which are the chords that connect two nodes together. In each case, the number of wound healing features observed in the combination group greatly exceeded observed features in single treatment groups (VEGF alone or insulin alone). Results showed that individual expression of concentrations of VEGF of 2 ng/mL or 0.1 UI of insulin did not induce the formation of segments, nodes or angiogenic nets for wound healing. Compared to insulin alone, insulin combined with VEGF exhibited significantly greater wound healing features in every assessment (webs, nodes, and main segments). In comparison to VEGF alone, the combination yielded a consistently greater amount of wound healing features across all groups, reaching the level of significance in an evaluation of main segments.

Together, these results indicate that the therapeutic benefits of insulin and VEGF can be synergistically enhanced when they are delivered in combination. This study further supports that a therapeutic effect can be maximized at low levels of each protein, in amounts that are unlikely to elicit systemic effects or toxicity.

### Example 5 : The Insulin- and VEGF-Releasing Skin Substitute Improves Wound Healing In Diabetic Rats and Hyperglycemic Pigs

Two animal models, diabetic rats and diabetic pigs, were used to evaluate the impact of the VEGF/insulin skin substitute on wound healing.

### A. Rats

Thirty Wistar rats (4 weeks old) weighing between 200 g and 250 g were kept in a temperature-controlled room (22°C) with a set humidity of 53%. Rats were housed in micro-insulators with 12 hour cycles of light and darkness. The rats were allowed to acclimate to this environment for one week before the beginning of the experiment.

In order to induce diabetes, 60 mg/kg streptozocin (also known as streptozotocin) (Sigma-Aldrich) was administered to rats via the intraperitoneal (I.P.) route. Streptozocin was prepared immediately before I.P. injection in a 0.01 M sodium citrate buffer (pH 4.5). Rats having a glucose concentration of 350 mg/dl (15 mM) or higher at 72 hours post-injection were diagnosed as diabetic. Blood glucose levels were determined with reactive strips using the Accu-Chek^{®} Instant glucometer (Roche Corp).

Diabetic rats, which maintained a hyperglycemic state over two months, were divided into two groups: the diabetic control group (no skin substitute) and the diabetic treatment group (skin substitute). Healthy rats were included as a third group in the study. All rats in each group were wounded on the back with a 1 cm² punch wound. Wounds in the control diabetic and healthy groups were dressed with Vaseline gauze. The wounds of diabetic rats in the treatment group were dressed with the VEGF/insulin skin substitute. Gauze dressings were changed every three days throughout the duration of the study. In contrast, the skin substitute was only applied once. Wounds were measured every three days until total wound closure was observed.

**FIG. 5A** shows the percentage of open wound area over a 21-day period across the three experimental groups. Time from wound initiation to total wound closing was 21 days in both the healthy control group and the diabetic group treated with skin substitute. In comparison, time to healing was extended by over one week in the diabetic group treated with gauze dressings (time to wound closure=30 days). Representative images of wounds at Day 1 and Day 21 are shown in **FIG. 5B****.**

In a further assessment of wound healing activity, histological examination was performed to determine the effects of the skin substitute on scar formation. For each experimental group, transverse sections of the scarring area were collected for histopathological examination at day 21 post-wound initiation. As shown in **FIG. 6****,** the structure and remodeling of both the dermis and epidermis was comparable between healthy rats and diabetic rats treated with the skin substitute. In contrast, examination of the diabetic sample revealed inflammation, cellular infiltration, and thickening of the tissue. Together, these results present in vivo confirmation of the wound healing activity of the skin substitute, consistent with the findings described in Examples 3 and 4.

### B. Pigs

A surgical approach was taken to induce hyperglycemia in pigs. Using sterile techniques, the left jugular vein on the ventral fascia of the neck was exposed and an 18G x 30 mm catheter was then inserted in the left jugular vein to collect 15 mL of blood. Following sample collection, the catheter was fixed by means of suturing to the blood vessel, and venous perfusion equipment was connected to maintain vascular access. To obtain a baseline blood sugar measurement, one blood sample was taken from each ear. One single dose of 124 mg/kg streptozocin was administered through the vascular access over 15 minutes using an infusion pump. This was followed by administration of a 5% glucose solution (200 mL) over the course of 30 minutes. Once the streptozocin and glucose had been administered, a new glucose measurement was taken from each ear to confirm a rise in blood glucose levels.

Pigs exhibiting normal behavior and a glycemic level of less than 180 mg/dL during the first 48 hours post-infusion were included in the control group. Glucose measurements were performed when the pigs were in a fasted state and one hour after their morning meal. When a glucose level of over 350 mg/dL was measured, insulin aspart (NovoMix^{®}), insulin isophane (Aurax^{®}) and insulin glargine (Lantus^{®}) were administered in order to maintain a hyperglycemia level of about 250 mg/dL. Pigs were maintained in a hyperglycemic state for two months to induce the effects of glycation on their skin. There were a total of three pigs in each group.

After the two month period, three 4x3 cm wounds were inflicted on the backs of anesthetized pigs, using the spine as a reference. Wounds were located at 2 cm from the middle line and had a space of 5 cm between them. The wounds were identified using location-based designations, i.e., Wound #1 was located on the left side of the skull, Wound #2 was located on the caudal left side, and Wound #3 was located on the central right side.

Once the effects of the anesthetic had dissipated, 0.01-0.03 mg/kg buprenorphine (Brospine^{®}) was administered via intramuscular injection to provide pain relief. On the first three days following wound initiation (days 1-3), one dose of buprenorphine was administered every eight hours. On the following three days (days 4-6), one dose was administered per hour. On days 7-9, one dose was given every 24 hours. At the discretion of the treating physician, an antibacterial treatment was administered in the event of an infection, either 3.0 mg/kg enrofloxacin (every 24 hours) or 15 mg/kg 3 Sulfas^{®} (sulfamethazine, sulfamerazine, and sulfadiazine in equal parts) (every 12 hours) administered via the intramuscular (I.M.) route for 8 days.

Photos were taken of the wounds with a reference centimeter scale on days 0, 1, 4, 7, 11, 18, 21, 25 and 28 post-wound initiation. The wounds were cleaned with a saline solution during the first seven days, and new sterile gauze and dressings were provided. In the case of the wounds dressed with the skin substitute, washing was only performed around the wound to avoid wetting or displacing the device. After the first seven days, gauze and dressings were changed every other day, including the days when photographs were taken. In comparison, the VEGF/insulin skin substitute was only applied once. Surgical debridement was performed under sedation on wounds that showed signs of infection. Wound's closure was evaluated by measuring the area in cm² using ImageI software.

**FIG. 7A** shows a comparison of wound area (cm²) between the healthy pigs, diabetic pigs (no skin substitute), and diabetic pigs treated with the VEGF/insulin skin substitute. Comparable to the results of the rat study, total wound closing was observed in the same time period for the healthy control group and the group treated with the VEGF/insulin skin substitute expressing. Total wound closing was noted at day 25 for the healthy controls and the pigs treated with the skin substitute, while an observable wound area persisted up to day 28 for the diabetic pigs (no skin substitute). Given that an open wound persisted for the diabetic group, observations were extended until total wound closure. As shown in **FIG. 7B****,** total wound healing was not achieved until day 52 for the diabetic group, nearly four weeks after healing was observed in the skin substitute-treated group. **FIG. 8** shows representative images of wound healing over time across the three groups of pigs. The pictured wound in the skin substitute group began closing at day 14, and no sign of infection was observed throughout the study. As shown by the top and bottom panels, wounds of the healthy control and diabetic treated with the VEGF/insulin skin substitute, respectively, closed at day 23. On this same day, wounds on a diabetic pig were still open, and the presence of an infection was noted (middle panel).

**FIG. 9** shows wounds in diabetic pigs treated with wound cleaning and gauze dressings and diabetic pigs treated with the VEGF/insulin skin substitute at day 1 and day 7 after the wound was inflicted. Infections were noted in diabetic pig wounds (no skin substitute) from day 3. In comparison, diabetic pigs treated with VEGF/insulin showed no sign of infection, and therefore did not require antibiotic therapy. Treatment of infections in diabetic pigs (no skin substitute) consisted of oral, e.g., 3 Sulfas^{®} and topical antibiotics, e.g., topical silver sulfadiazine, for 45 days.

Glucose levels were measured from one week before wound initiation (day -7) to 11 days post-wound to assess the systemic effects of the insulin- and VEGF-releasing epidermis. **FIG. 10** shows the detected levels of blood glucose (mg/dL) across all experimental groups. Levels were maintained at a relatively low level (~100 mg/dL) in the healthy group. For both diabetic groups, blood glucose fluctuated by as much as ~100 mg/dL throughout the course of the study. Relative to the diabetic control, treatment with the VEGF/insulin skin substitute did not dramatically alter blood glucose levels. These results are consistent with an observation that the enhanced synergy between the molecules not only allows a faster and organized healing and scarring, but it also ensures the lack of toxicity and safety of the product.

Together, these in vivo studies support the potent wound healing activity and safety of the VEGF/insulin skin substitute for treating wounds in diabetic mice. In both rats and pigs, diabetic skin treated with the VEGF/insulin skin substitute healed on a timeline comparable to that of healthy skin. Further, treatment with the VEGF/insulin skin substitute was not associated with microbial infection and did not dramatically alter systemic blood glucose levels, indicating that insulin and VEGF are not present in amounts that would greatly impact glycaemia. In addition to the therapeutic benefit, the current study demonstrates that frequent replacement of the skin substitute is unnecessary, an advantage with positive implications for patient compliance.

### Example 6 : Continuous Release of Insulin and VEGF From a VEGF/Insulin Skin Substitute Decreases the Presence of Advanced Glycation End Products (AGEs) in Pig Skin

Hyperglycemia causes structural changes on the proteins and lipids of cells and tissues, such as by glycation as a result of covalent attachment of a sugar molecule (glucose or fructose) to a protein or a lipid. Glycated proteins or lipids are referred to AGEs. High AGE concentrations are found in the skin of diabetics and serve to inhibit the healing process. The skin of pigs from Example 5 was examined to determine the effect of the skin substitute on advanced glycation end product (AGE) concentration.

Skin biopsies were performed on the experimental groups described in the pig study in Example 5: healthy pigs, diabetic pigs (no skin substitute), and diabetic pigs treated with the VEGF/insulin skin substitute. As described in Example 5, pigs in each diabetic group maintained a glycemic level of >350 mg/dL for two months. For pigs treated with the VEGF/insulin skin substitute, samples of the dermis and epidermis were collected both at the time of wound initiation and two days after wound healing was evident (at or about day 25). For healthy and diabetic controls, skin samples biopsies were taken at the moment of inflicting the wound and when it healed 21 days later (healthy pigs) or 60 days later (diabetic pigs).

For each biopsy, a portion of the collected sample was separated and weighed using an analytic scale. About 20 mg of tissue was disaggregated with TissueRuptor in 500 µL of 1X PBS with protease inhibitor Complete (Sigma). The sample was then centrifuged at 4,000 rpm for 5 minutes to achieve precipitation of aggregated tissue, and the supernatant was removed. The Bradford method (Sigma) of protein estimation was used to adjust protein concentration to 50 µg/mL. An ELISA kit was used to quantify AGEs, with a 1:10 dilution of sample. ELISAs were performed according to the manufacturer's instructions. Briefly, 50 µL of the diluted sample and 50 µL of the diluted biotinylated antibody (1:100) were incubated for 45 minutes. After this initial incubation, three washes were completed with about 359 µL of 1X washing solution. 100 µL of the secondary antibody with HRP (1:100) was then added and incubated at 37°C for 30 minutes. At the end of this incubation, five washes were performed with 350 µL of 1X washing solution. Following removal of the washing solution, 90 µL of 3,3',5,5'-tetramethylbenzidine (TMB) was added and incubated for 15 minutes at 37°C. The reaction was stopped by adding 50 µL of stopping solution. Absorbance was read at 450 nm, and calculations were made to obtain AGE concentrations using the second order polynomial regression equation obtained with data from the standard curve (R² =0.99).

The concentrations in ng for each mg of protein were calculated from the concentrations obtained with the AGEs ELISA kit (data in ng/mL). FIG. 11 shows the amount of AGEs (ng for each mg of protein) for each experimental group, before and after wound healing, as calculated from the ELISAs. Results showed that the level of AGEs increased from wound initiation to healing in the healthy group, and AGEs were maintained at a relatively high level in the diabetic (no skin substitute) group. Treatment with the VEGF/insulin skin substitute decreased the number of AGEs from wound initiation to healing. Notably, at the time of wound initiation, AGEs in the VEGF/insulin skin substitute-treated group were similar to those of the diabetic group, but treatment with the VEGF/insulin skin substitute decreased AGEs to a level observed in healthy skin.

Reduction of AGEs is associated with improved angiogenesis and control over infections and inflammation. This study supports that treatment with the VEGF/insulin skin substitute is capable of decreasing AGEs, even in skin with advanced glycation (levels of AGEs were approximately double the amount present in healthy skin). These results indicate that the wound healing effects of the VEGF/insulin skin substitute may be mediated by AGE reduction.

### Example 7 : VEGF/Insulin Skin Substitute is Non-Tumorigenic

Tumor formation was evaluated after subcutaneous transplantation of 20 mm² of non-transduced skin substitute (control) or 20 mm² of skin substitute transduced with Ad-CAG-VEFG-INS adenovirus into the interscapular region of 6-week-old nude mice (BALB/c nu/nu). Tumor growth was measured at weekly intervals. Skin substitutes transduced with the Ad-CAG-VEFG-INS adenovirus did not show tumor formation after 4 months of being transplanted into nude mice (data not shown). Positive control nude mice injected with MCF-7 human breast cancer cells showed tumor growth one month after subcutaneous injection (data not shown).

### Example 8 : Assessment of Cytogenetic Properties of Human Keratinocyte Cell Line For Producing the Skin Substitute

As described in above Examples, production of the skin substitute involves first culturing human keratinocytes of the cellular HaCat line in low calcium media to generate a basal layer amenable to transduction followed by their differentiation. HaCaT cells closely resemble normal human keratinocytes in their growth and differentiation potential; however, they are an immortalized cell line with some chromosomal abnormalities. Although HaCaT cells retain a stable chromosome content and remain nontumorigenic, experiments were carried out to characterize the cytogenetic features of cells after culture in low calcium and serum free media to culture a basal layer.

The HaCat line was grown in low calcium (0.03 mM) media without fetal bovine serum for 4 weeks under conditions to culture a basal layer, and were termed HaLow cells (HaCat without serum in low calcium). The chromosomal constitution of HaLow cells was followed during propagation, starting with passage 2, when the cells had been in culture for a total of 3 months. Semiconfluent cell cultures were treated for 2 h at 37°C with 0.08 µg/ml KaryoMAX^{™} Colcemid^{™} (ThermoFisher 15212012). Cells were detached by subsequent treatment with Recombinant Trypsin EDTA Solution (Sartorius 03-079-1A 10 min), centrifuged, and the cell pellet resuspended in a hypotonic solution of 75 mM KC1. After incubation for 15min at room temperature, cells were fixed by three changes of methanol/acetic acid (3:1), spread on glass slides, and G-banding carried out after 16 hours. Usually 15 metaphases were analyzed microscopically and at least 5 karyograms were constructed.

As shown in **FIG. 12****,** at passage 7 cytogenetic analysis of the cells showed the following karyogram: 65, XXX, +1, -2, add(3)(p25), -3, add(4)(p15), -4, -4, -5, 6, -7, +8, i(9)(q10), -9, -10, +der(11)del11(q23), +11, + 12, +13, -14, 15, +16, +17, +18, +19, +20, +21, +22, +6mar, +min. The majority of cells were hypotriploid with an average of 65 chromosomes resulting from full or partial monosomies of chromosomes involved in the formation of marker chromosomes. All metaphases had the XO sex chromosome constitution (lacking the Y chromosome). This cytogenetic profile indicates cell's adaptation to growing under modified culture conditions but the karyogram is consistent with cells originated from HaCat cells. Together with the results in Example 7, the results show that after incubation in low calcium and without serum the cell line HaLow has certain cytogenetic characteristics and when used in the skin substitute is non tumorigenic.

### Example 9 : Expression and Release of Epidermal Growth Factor from Skin Substitutes

Epidermal growth factor (EGF) is an important promoter of wound repair and regeneration in diabetic foot ulcers (DFU). In DFU, the wound healing process is hindered by the accumulation of advanced glycation end products (AGEs), which is due to the high blood glucose levels of patients with diabetes. AGEs competitively bind to the EGF receptor, thus preventing the binding of EGF and perpetuating the initial injury to the vascular endothelial cells and fibroblasts. Current technologies for the delivery of growth factors, including EGF, into the wound environment are not effective, mainly due to the very short in vivo half-live of EGF when administered directly into the extracellular matrix.

EGF expression and release into the media was evaluated in cultures of skin substitutes transduced with an adenovirus expressing human EGF (Ad-CMV-hEGF), using methods substantially described in above examples except in which an adenovirus expressing human EGF was transduced into the skin substitute. Skin substitutes were transduced with 5x10⁶ IFU (infective units) of adenovirus expressing human epidermal growth factor (Ad-CMV-hEGF, GenBank: BC113461). Culture media from the transduced and non-transduced skin substitute cultures were collected after 72 hours of incubation, and the hEGF protein secreted from the skin substitute into the culture media was quantified with a commercial enzyme-linked immunosorbent assay (ELISA) kit (R&D Systems).

As shown in **FIG. 13** the concentration of human epidermal growth factor (hEGF) was significantly higher in the culture media of skin substitutes transduced with an adenovirus expressing hEGF (Ad-CMV-hEGF) than in the culture media of the non-transduced experimental controls.

These results show that, when transduced with an adenovirus carrying a polynucleotide codifying for hEGF, the skin substitutes provided herein can effectively express and release hEGF into the culture media.

These results support that other recombinant growth factors, such as EGF, can be used to produce a skin substitute to release the growth factor during wound healing by genetically modifying the cells from the skin substitute with an adenovirus that expresses and secretes growth factors. Without wishing to be bound by theory, the results support that other recombinant growth factors could be used in combination with insulin in a skin substitute, in which release of insulin into the wound from the skin substitute inhibits the presence of AGEs and permits the activation of EGF receptor.

### SEQUENCES

| # | SEQUENCE | ANNOTATIO N |
|---|---|---|
| 1 | GCGATCGCGGCTCCCGACATCTTGGACCATTAGCTCCACAGGT ATCTTCTTCCCTCTAGTGGTCATAACAGCAGCTTCAGCTACCTC TCAATTCAAAAAACCCCTCAAGACCCGTTTAGAGGCCCCAAG GGGTTATGCTATCAATCGTTGCGTTACACACACAAAAAACCAA CACACATCCATCTTCGATGGATAGCGATTTTATTATCTAACTG CTGATCGAGTGTAGCCAGATCTATAGTAATCAATTACGGGGTC ATTAGTTCATAGCCCATATATGGAGTTCCGCGTTACATAACTT ACGGTAAATGGCCCGCCTGGCTGACCGCCCAACGACCCCCGC CCATTGACGTCAATAATGACGTATGTTCCCATAGTAACGCCAA TAGGGACTTTCCATTGACGTCAATGGGTGGACTATTTACGGTA AACTGCCCACTTGGCAGTACATCAAGTGTATCATATGCCAAGT ACGCCCCCTATTGACGTCAATGACGGTAAATGGCCCGCCTGGC ATTATGCCCAGTACATGACCTTATGGGACTTTCCTACTTGGCA GTACATCTACGTATTAGTCATCGCTATTACCATGCGTCGAGGT GAGCCCCACGTTCTGCTTCACTCTCCCCATCTCCCCCCCCTCCC CACCCCCAATTTTGTATTTATTTATTTTTTAATTATTTTATGCAG CGATGGGGGCGGGGGGGGGGGGGGCGCGCGCCAGGCGGGGC GGGGCGGGGCGAGGGGCGGGGCGGGGCGAGGCGGAGAGGTG CGGCGGCAGCCAATCAGAGCGGCGCGCTCCGAAAGTTTCCTTT TATGGCGAGGCGGCGGCGGCGGCGGCCCTATAAAAAGCGAAG CGCGCGGCGGGCGGGAGTCGCTGCGTTGCCTTCGCCCCGTGCC CCGCTCCGCGCCGCCTCGCGCCGCCCGCCCCGGCTCTGACTGA CCGCGTTACTCCCACAGGTGAGCGGGCGGGACGGCCCTTCTCC CTCCGGGCTGTAATTAGCGCTTGGTTTAATGACGGCTCGTTTCT TTTCTGTGGCTGCGTGAAAGCCTTAAAGGGCTCCGGGAGGGCC TTTGTGCGGGGGGGAGCGGCTCGGGGGGTGCGTGCGTGTGTG TGTGCGTGGGGAGCGCCGCGTGCGGCCCGCGCTGCCCGGCGG CTGTGAGCGCTGCGGGCGCGGCGCGGGGCTTTGTGCGCTCCGC GTGTGCGCGAGGGGAGCGCGGGCCGGGGGCGGTGCCCCGCGG TGCGGGGGGGCTGCGAGGGGAACAAAGGCTGCGTGCGGGGTG TGTGCGTGGGGGGGTGAGCAGGGGGTGTGGGCGCGGCGGTCG GGCTGTAACCCCCCCCTGGCACCCCCCTCCCCGAGTTGCTGAG CACGGCCCGGCTTCGGGTGCGGGGCTCCGTGCGGGGCGTGGC GCGGGGCTCGCCGTGCCGGGCGGGGGGTGGCGGCAGGTGGGG GTGCCGGGCGGGGCGGGGCCGCCTCGGGCCGGGGAGGGCTCG GGGGAGGGGCGCGGCGGCCCCGGAGCGCCGGCGGCTGTCGAG GCGCGGCGAGCCGCAGCCATTGCCTTTTATGGTAATCGTGCGA GAGGGCGCAGGGACTTCCTTTGTCCCAAATCTGGCGGAGCCG AAATCTGGGAGGCGCCGCCGCACCCCCTCTAGCGGGCGCGGG CGAAGCGGTGCGGCGCCGGCAGGAAGGAAATGGGCGGGGAG GGCCTTCGTGCGTCGCCGCGCCGCCGTCCCCTTCTCCATCTCCA GCCTCGGGGCTGCCGCAGGGGGACGGCTGCCTTCGGGGGGGA CGGGGCAGGGCGGGGTTCGGCTTCTGGCGTGTGACCGGCGGC TTTAGAGCCTCTGCTAACCATGTTCATGCCTTCTTCTTTTTCCT | CAG promoter |
| | ACAGCTCCTGGGCAACGTGCTGGTTGTTGTGCTGTCTCATCAT TTTGGCAAAGATCTTTCCACC | |
| 2 | ATGGCCCTGTGGATGCGCCTCCTGCCCCTGCTGGCGCTGCTGG CCCTCTGGGGACCTGACCCAGCCGCAGCCTTTGTGAACCAACA CCTGTGCGGCTCAGACCTGGTGGAAGCTCTCTACCTAGTGTGC GGGGAACGAGGCTTCTTCTACACACCCAGGACCAAGCGGGAG GCAGAGGACCTGCAGGTGGGGCAGGTGGAGCTGGGCGGGGG CCCTGGTGCAGGCAGCCTGCAGCCCTTGGCCCTGGAGGGGTCC CGGCAGAAGCGTGGCATTGTGGAACAATGCTGTACCAGCATC TGCTCCCTCTACCAGCTGGAGAACTACTGCAACTAG | Human insulin |
| 3 | TATGCTAGTACGTCTCTCAAGGATAAGTAAGTAATATTAAGGT ACGGGAGGTATTGGACAGGCCGCAATAAAATATCTTTATTTTC ATTACATCTGTGTGTTGGTTTTTTGTGTGAATCGATAGTACTAA CATACGCTCTCCATCAAAACAAAACGAAACAAAACAAACTAG CAAAATAGGCTGTCCCCAGTGCAAGTGCAGGTGCCAGAACAT TTCTCTGGCCTAACTGGCCGGTACCTGAGCTCTAGTTTCACTTT CCCTAGTTTCACTTTCCCTAGTTTCACTTTCCCTAGTTTCACTTT CCCTAGTTTCACTTTCCCCTCGAGGATATCAAGATCTGGCCTC GGCGGCCAG | IRES |
| 4 | ATGAACTTTCTGCTGTCTTGGGTGCATTGGAGCCTTGCCTTGCT GCTCTACCTCCACCATGCCAAGTGGTCCCAGGCTGCACCCATG GCAGAAGGAGGAGGGCAGAATCATCACGAAGTGGTGAAGTTC ATGGATGTCTATCAGCGCAGCTACTGCCATCCAATCGAGACCC TGGTGGACATCTTCCAGGAGTACCCTGATGAGATCGAGTACAT CTTCAAGCCATCCTGTGTGCCCCTGATGCGATGCGGGGGCTGC TGCAATGACGAGGGCCTGGAGTGTGTGCCCACTGAGGAGTCC AACATCACCATGCAGATTATGCGGATCAAACCTCACCAAGGC CAGCACATAGGAGAGATGAGCTTCCTACAGCACAACAAATGT GAATGCAGACCAAAGAAAGATAGAGCAAGACAAGAAAATCC CTGTGGGCCTTGCTCAGAGCGGAGAAAGCATTTGTTTGTACAA GATCCGCAGACGTGTAAATGTTCCTGCAAAAACACAGACTCG CGTTGCAAGGCGAGGCAGCTTGAGTTAAACGAACGTACTTGC AGATGTGACAAGCCGAGGCGGTGA | VEGF |
| 5 | FVNQHLCGSHLVEALYLVCGERGFFYTPKTRREAEDLQVGQVEL GGGPGAGSLQPLALEGSLQKRGIVEQCCTSICSLYQLENYCN | Human insulin proprotein (amino acids 25-110 of NP_000198.1, encoded by NM ₋000207.3) |
| 6 | FVNQHLCGSDLVEALYLVCGERGFFYTPRTKREAEDLQVGQVEL GGGPGAGSLQPLALEGSRQKRGIVEQCCTSICSLYQLENYCN | Modified human insulin proprotein |
| 7 | MNFLLSWVHWSLALLLYLHHAKWSQAAPMAEGGGQNHHEVV KFMDVYQRSYCHPIETLVDIFQEYPDEIEYIFKPSCVPLMRCGGCC NDEGLECVPTEESNITMQIMRIKPHQGQHIGEMSFLQHNKCECRP KKDRARQENPCGPCSERRKHLFVQDPQTCKCSCKNTDSRCKARQ LELNERTCRCDKPRR | VEGF-A, isoform VEGF 165; signal peptide (a.a. positions 1-26), VEGF-A (a.a. positions 27-191) |
| 8 | R-X-R-R, X is any amino acid | Furin Consensus Sequence |
| 9 | R-X-K-R, X is any amino acid | Furin Consensus Sequence |
| 10 | RTKR | Furin Consensus Sequence |
| 11 | MNFLLSWVHWSLALLLYLHHAKWSQAAPMAEGGGQNHHEVV KFMDVYQRSYCHPIETLVDIFQEYPDEIEYIFKPSCVPLMRCGGCC NDEGLECVPTEESNITMQIMRIKPHQGQHIGEMSFLQHNKCECRP KKDRARQEKKSVRGKGKGQKRKRKKSRYKSWSVYVGARCCLM PWSLPGPHPCGPCSERRKHLFVQDPQTCKCSCKNTDSRCKARQL ELNERTCRCDKPRR | VEGF-A, isoform VEGF 206; Accession No. P15692-1; signal peptide (a.a. positions 1-26), VEGF-A (a.a. positions 27-232) |
| 12 | MSPLLRRLLLAALLQLAPAQAPVSQPDAPGHQRKVVSWIDVYTR ATCQPREVVVPLTVELMGTVAKQLVPSCVTVQRCGGCCPDDGL ECV PTGQHQVRMQILMIRYPSSQLGEMSLEEHSQCECRPKKKDSAVK PDRAATPHHRPQPRSVPGWDSAPGAPSPADITHPTPAPGPSAHAA PSTTSALTPGPAAAAADAAASSVAKGGA | VEGF-B, Isoform VEGF-B186; Accession No. P49765-1; signal peptide (a.a. positions 1-21), VEGF-B (a.a. positions 22-207) |
| 13 | MYREWVVVNVFMMLYVQLVQGSSNEHGPVKRSSQSTLERSEQQ IRAASSLEELLRITHSEDWKLWRCRLRLKSFTSMDSRSASHRSTRF AATFYDIETLKVIDEEWQRTQCSPRETCVEVASELGKSTNTFFKPP CVNVFRCGGCCNEESLICMNTSTSYISKQLFEISVPLTSVPELVPV KVANHTGCKCLPTAPRHPYSIIRRSIQIPEEDRCSHSKKLCPIDML WDSNKCKCVLQEENPLAGTEDHSHLQEPALCGPHMMFDEDRCE CVCKTPCPKDLIQHPKNCSCFECKESLETCCQKHKLFHPDTCSCE DRCPFHTRPCASGKTACAKHCRFPKEKRAAQGPHSRKNP | VEGF-D; Accession No. 043915-1; signal peptide (a.a. positions 1-21), propeptides (22-88 and 206-354), VEGF-D (a.a. positions 89-205) |
| 14 | | PDGF-A, isoform long; Accession No. P04085-1; signal peptide (a.a. positions 1-20), propeptide (a.a. positions 21-86), PDGF-A(a.a. positions 87-211) |
| 15 | | PDGF-B, isoform 1; Accession No. P01127-1; signal peptide (a.a. positions 1-20), PDGF-B (a.a. positions 82-190) propeptides (a.a. positions 21-81 and 191-241) |
| 16 | MPVMRLFPCFLQLLAGLALPAVPPQQWALSAGNGSSEVEVVPFQ EVWGRSYCRALERLVDVVSEYPSEVEHMFSPSCVSLLRCTGCCG DENLHCVPVETANVTMQLLKIRSGDRPSYVELTFSQHVRCECRH SPGRQSPDMPGDFRADAPSFLPPRRSLPMLFRMEWGCALTGSQS AVWPSSPVPEEIPRMHPGRNGKKQQRKPLREKMKPERCGDAVPR R | Placenta growth factor (PLGF), isoform PlGF-3; Accession No. P49763-1; signal peptide (a.a. positions 1-18) and PLGF (a.a. positions 19-221) |
| 17 | MALWTRLRPLLALLALWPPPPARAFVNQHLCGSHLVEALYLVC GERGFFYTPKARREVEGPQVGALELAGGPGAGGLEGPPQKRGIV EQCCASVCSLYQLENYCN | Bovine insulin; Accession No. P01317-1; signal peptide (a.a. positions 1-24), Insulin B chain (a.a. positions 25-54), C-peptide (a.a. positions 57-82), and Insulin A chain (a.a. positions 85-105) |
| 18 | MALWTRLLPLLALLALWAPAPAQAFVNQHLCGSHLVEALYLVC GERGFFYTPKARREAENPQAGAVELGGGLGGLQALALEGPPQKR GIVEQCCTSICSLYQLENYCN | Porcine insulin; Accession No. P01315-1; signal peptide (a.a. positions 1-24), Insulin B chain (a.a. positions 25-54), C-peptide (a.a. positions 57-85) and Insulin A chain (a.a. positions 88-108) |
| 19 | MNFLLSWVHWSLALLLYLHHAKWSQAAPMAEGGGQNHHEVV KFMDVYQRSYCHPIETLVDIFQEYPDEIEYIFKPSCVPLMRCGGCC NDEGLECVPTEESNITMQIMRIKPHQGQHIGEMSFLQHNKCECRP KKDRARQEKKSVRGKGKGQKRKRKKSRYKSWSVPCGPCSERRK HLFVQDPQTCKCSCKNTDSRCKARQLELNERTCRCDKPRR | VEGF-A, isoform VEGF 189; Accession No. P15692-2 |
| 20 | MNFLLSWVHWSLALLLYLHHAKWSQAAPMAEGGGQNHHEVV KFMDVYQRSYCHPIETLVDIFQEYPDEIEYIFKPSCVPLMRCGGCC NDEGLECVPTEESNITMQIMRIKPHQGQHIGEMSFLQHNKCECRP KKDRARQEKKSVRGKGKGQKRKRKKSRPCGPCSERRKHLFVQD PQTCKCSCKNTDSRCKARQLELNERTCRCDKPRR | VEGF-A, isoform VEGF 183; Accession No. P15692-3 |
| 21 | MNFLLSWVHWSLALLLYLHHAKWSQAAPMAEGGGQNHHEVV KFMDVYQRSYCHPIETLVDIFQEYPDEIEYIFKPSCVPLMRCGGCC NDEGLECVPTEESNITMQIMRIKPHQGQHIGEMSFLQHNKCECRP KKDRARQENPCGPCSERRKHLFVQDPQTCKCSCKNTDSRCKM | VEGF-A, isoform VEGF 148; Accession No. P15692-5 |
| 22 | MNFLLSWVHWSLALLLYLHHAKWSQAAPMAEGGGQNHHEVV KFMDVYQRSYCHPIETLVDIFQEYPDEIEYIFKPSCVPLMRCGGCC NDEGLECVPTEESNITMQIMRIKPHQGQHIGEMSFLQHNKCECRP KKDRARQEKKSVRGKGKGQKRKRKKSRYKSWSVCDKPRR | VEGF-A, isoform VEGF 145; Accession No. P15692-5 |
| 23 | MNFLLSWVHWSLALLLYLHHAKWSQAAPMAEGGGQNHHEVV KFMDVYQRSYCHPIETLVDIFQEYPDEIEYIFKPSCVPLMRCGGCC NDEGLECVPTEESNITMQIMRIKPHQGQHIGEMSFLQHNKCECRP KKDRARQENPCGPCSERRKHLFVQDPQTCKCSCKNTDSRCKARQ LELNERTCRSLTRKD | VEGF-A, isoform VEGF 165B; Accession No. P15692-8 |
| 24 | MNFLLSWVHWSLALLLYLHHAKWSQAAPMAEGGGQNHHEVV KFMDVYQRSYCHPIETLVDIFQEYPDEIEYIFKPSCVPLMRCGGCC NDEGLECVPTEESNITMQIMRIKPHQGQHIGEMSFLQHNKCECRP KKDRARQEKCDKPRR | VEGF-A, isoform VEGF 121; Accession No. P15692-9 |
| 25 | MNFLLSWVHWSLALLLYLHHAKWSQAAPMAEGGGQNHHEVV KFMDVYQRSYCHPIETLVDIFQEYPDEIEYIFKPSCVPLMRCGGCC NDEGLECVPTEESNITMQIMRIKPHQGQHIGEMSFLQHNKCECRC DKPRR | VEGF-A, isoform VEGF 111; Accession No. P15692-10 |
| 26 | MTDRQTDTAPSPSYHLLPGRRRTVDAAASRGQGPEPAPGGGVEG VGARGVALKLFVQLLGCSRFGGAVVRAGEAEPSGAARSASSGRE EPQPEEGEEEEEKEEERGPQWRLGARKPGSWTGEAAVCADSAPA ARAPQALARASGRGGRVARRGAEESGPPHSPSRRGSASRAGPGR ASETMNFLLSWVHWSLALLLYLHHAKWSQAAPMAEGGGQNHH EVVKFMDVYQRSYCHPIETLVDIFQEYPDEIEYIFKPSCVPLMRCG GCCNDEGLECVPTEESNITMQIMRIKPHQGQHIGEMSFLQHNKCE CRPKKDRARQENPCGPCSERRKHLFVQDPQTCKCSCKNTDSRCK ARQLELNERTCRCDKPRR | VEGF-A, isoform L-VEGF 165; Accession No. P15692-11 |
| 27 | MTDRQTDTAPSPSYHLLPGRRRTVDAAASRGQGPEPAPGGGVEG VGARGVALKLFVQLLGCSRFGGAVVRAGEAEPSGAARSASSGRE EPQPEEGEEEEEKEEERGPQWRLGARKPGSWTGEAAVCADSAPA ARAPQALARASGRGGRVARRGAEESGPPHSPSRRGSASRAGPGR ASETMNFLLSWVHWSLALLLYLHHAKWSQAAPMAEGGGQNHH EVVKFMDVYQRSYCHPIETLVDIFQEYPDEIEYIFKPSCVPLMRCG GCCNDEGLECVPTEESNITMQIMRIKPHQGQHIGEMSFLQHNKCE CRPKKDRARQEKCDKPRR | VEGF-A, isoform L-VEGF 121; Accession No. P15692-12 |
| 28 | MTDRQTDTAPSPSYHLLPGRRRTVDAAASRGQGPEPAPGGGVEG VGARGVALKLFVQLLGCSRFGGAVVRAGEAEPSGAARSASSGRE EPQPEEGEEEEEKEEERGPQWRLGARKPGSWTGEAAVCADSAPA ARAPQALARASGRGGRVARRGAEESGPPHSPSRRGSASRAGPGR ASETMNFLLSWVHWSLALLLYLHHAKWSQAAPMAEGGGQNHH EVVKFMDVYQRSYCHPIETLVDIFQEYPDEIEYIFKPSCVPLMRCG GCCNDEGLECVPTEESNITMQIMRIKPHQGQHIGEMSFLQHNKCE CRPKKDRARQEKKSVRGKGKGQKRKRKKSRYKSWSVPCGPCSE RRKHLFVQDPQTCKCSCKNTDSRCKARQLELNERTCRCDKPRR | VEGF-A, isoform L-VEGF 189; Accession No. P15692-13 |
| 29 | MTDRQTDTAPSPSYHLLPGRRRTVDAAASRGQGPEPAPGGGVEG VGARGVALKLFVQLLGCSRFGGAVVRAGEAEPSGAARSASSGRE EPQPEEGEEEEEKEEERGPQWRLGARKPGSWTGEAAVCADSAPA ARAPQALARASGRGGRVARRGAEESGPPHSPSRRGSASRAGPGR ASETMNFLLSWVHWSLALLLYLHHAKWSQAAPMAEGGGQNHH EVVKFMDVYQRSYCHPIETLVDIFQEYPDEIEYIFKPSCVPLMRCG GCCNDEGLECVPTEESNITMQIMRIKPHQGQHIGEMSFLQHNKCE CRPKKDRARQEKKSVRGKGKGQKRKRKKSRYKSWSVYVGARC CLMPWSLPGPHPCGPCSERRKHLFVQDPQTCKCSCKNTDSRCKA RQLELNERTCRCDKPRR | VEGF-A, isoform L-VEGF 206; Accession No. P15692-14 |
| 30 | MTDRQTDTAPSPSYHLLPGRRRTVDAAASRGQGPEPAPGGGVEG VGARGVALKLFVQLLGCSRFGGAVVRAGEAEPSGAARSASSGRE EPQPEEGEEEEEKEEERGPQWRLGARKPGSWTGEAAVCADSAPA ARAPQALARASGRGGRVARRGAEESGPPHSPSRRGSASRAGPGR ASETMNFLLSWVHWSLALLLYLHHAKWSQAAPMAEGGGQNHH EVVKFMDVYQRSYCHPIETLVDIFQEYPDEIEYIFKPSCVPLMRCG GCCNDEGLECVPTEESNITMQIMRIKPHQGQHIGEMSFLQHNKCE CRPKKDRARQENPCGPCSERRKHLFVQDPQTCKCSCKNTDSRCK ARQLELNERTCRSLTRKD | VEGF-A, isoform 15; Accession No. P15692-15 |
| 31 | MTDRQTDTAPSPSYHLLPGRRRTVDAAASRGQGPEPAPGGGVEG VGARGVALKLFVQLLGCSRFGGAVVRAGEAEPSGAARSASSGRE EPQPEEGEEEEEKEEERGPQWRLGARKPGSWTGEAAVCADSAPA ARAPQALARASGRGGRVARRGAEESGPPHSPSRRGSASRAGPGR ASETMNFLLSWVHWSLALLLYLHHAKWSQAAPMAEGGGQNHH EVVKFMDVYQRSYCHPIETLVDIFQEYPDEIEYIFKPSCVPLMRCG GCCNDEGLECVPTEESNITMQIMRIKPHQGQHIGEMSFLQHNKCE CRPKKDRARQEKKSVRGKGKGQKRKRKKSRPCGPCSERRKHLF VQDPQTCKCSCKNTDSRCKARQLELNERTCRCDKPRR | VEGF-A, isoform 16; Accession No. P15692-16 |
| 32 | MTDRQTDTAPSPSYHLLPGRRRTVDAAASRGQGPEPAPGGGVEG VGARGVALKLFVQLLGCSRFGGAVVRAGEAEPSGAARSASSGRE EPQPEEGEEEEEKEEERGPQWRLGARKPGSWTGEAAVCADSAPA ARAPQALARASGRGGRVARRGAEESGPPHSPSRRGSASRAGPGR ASETMNFLLSWVHWSLALLLYLHHAKWSQAAPMAEGGGQNHH EVVKFMDVYQRSYCHPIETLVDIFQEYPDEIEYIFKPSCVPLMRCG GCCNDEGLECVPTEESNITMQIMRIKPHQGQHIGEMSFLQHNKCE CRPKKDRARQENPCGPCSERRKHLFVQDPQTCKCSCKNTDSRCK M | VEGF-A, isoform 17; Accession No. P15692-17 |
| 33 | MTDRQTDTAPSPSYHLLPGRRRTVDAAASRGQGPEPAPGGGVEG VGARGVALKLFVQLLGCSRFGGAVVRAGEAEPSGAARSASSGRE EPQPEEGEEEEEKEEERGPQWRLGARKPGSWTGEAAVCADSAPA ARAPQALARASGRGGRVARRGAEESGPPHSPSRRGSASRAGPGR ASETMNFLLSWVHWSLALLLYLHHAKWSQAAPMAEGGGQNHH EVVKFMDVYQRSYCHPIETLVDIFQEYPDEIEYIFKPSCVPLMRCG GCCNDEGLECVPTEESNITMQIMRIKPHQGQHIGEMSFLQHNKCE CRCDKPRRs | VEGF-A, isoform 18; Accession No. P15692-18 |
| 34 | GIVEQCCTSICSLYQLENYCG | Insulin glargine A chain |
| 35 | FVNQHLCGSHLVEALYLVCGERGFFYTPKTRR | Insulin glargine B chain |
| 36 | GIVEQCCTSICSLYQLENYCN | Insulin A chain |
| 37 | FVNQHLCGSHLVEALYLVCGERGFFYTKPT | Insulin Lispro B chain |
| 38 | FVNQHLCGSHLVEALYLVCGERGFFYTDKT | Insulin aspart B chain |
| 39 | FVKQHLCGSHLVEALYLVCGERGFFYTPET | Insulin glulisine B chain |
| 40 | FVNQHLCGSHLVEALYLVCGERGFFYTPKT | Insulin B chain |
| 41 | FVNQHLCGSDLVEALYLVCGERGFFYTPRT | AspB10 insulin B chain |
| 42 | RQKR | Furin cleavage site |
| 43 | MALWMRLLPLLALLALWGPDPAAA | Signal peptide |
| 44 | APMAEGGGQNHHEVVKFMDVYQRSYCHPIETLVDIFQEYPDEIE YIFKPSCVPLMRCGGCCNDEGLECVPTEESNITMQIMRIKPHQGQ HIGEMSFLQHNKCECRPKKDRARQENPCGPCSERRKHLFVQDPQ TCKCSCKNTDSRCKARQLELNERTCRCDKPRR | VEGF-A 165, mature |
| 45 | MNFLLSWVHWSLALLLYLHHAKWSQA | VEGF signal peptide |

## Claims

1. A skin substitute comprising a stratified epidermis comprising a basal layer, a spinous layer, a granular layer and a stratum corneum, wherein cells of the stratified epidermis express a recombinant growth factor and a recombinant insulin:
wherein the recombinant growth factor is a vascular endothelial growth factor (VEGF).

2. The skin substitute of claim 1, wherein:
(a) the recombinant growth factor and recombinant insulin are secretable from cells of the stratified epidermis, optionally wherein the cells of the stratified epidermis that express the recombinant growth factor and the recombinant insulin comprise cells of the basal layer;
(b) the recombinant insulin is or comprises a recombinant human insulin;
(c) the recombinant insulin comprises:
(i) the sequence of amino acids set forth in SEQ ID NO: 5;
(ii) a functional variant that has a sequence of amino acids that has at least 85%, at least 90%, or at least 95% sequence identity to SEQ ID NO: 5; or
(iii) a two-chain form of (i) or (ii) that comprises an A-chain and a B-chain, optionally wherein the A-chain and B-chain are linked by a disulfide bond;
(d) the recombinant insulin is an AspB 10 insulin analog comprising a histidine to aspartic acid mutation at position 10 in the B chain of the modified human proinsulin compared to wild-type insulin set forth in SEQ ID NO: 5;
(e) the skin substitute comprises a polynucleotide encoding a proinsulin comprising at least one furin recognition sequence in place of the endopeptidase Arg31-Arg32 cleavage site or the endopeptidase Lys64-Arg65 cleavage site, optionally wherein the at least one furin recognition sequence:
(i) is in place of the endopeptidase Arg31-Arg32 cleavage site and the endopeptidase Lys64-Arg65 cleavage site;
(ii) comprises the consensus sequence R-X-R-R, where X is any amino acid (SEQ ID NO: 8), or R-X-K-R, where X is any amino acid (SEQ ID NO: 9); and/or
(iii) is RTKR (SEQ ID NO: 10) or RQKR (SEQ ID NO: 42);
(f) the recombinant insulin comprises or has:
(i) the sequence of amino acids set forth in SEQ ID NO: 6;
(ii) a functional variant that has a sequence of amino acids that has at least 85%, at least 90%, or at least 95% sequence identity to SEQ ID NO: 6; or
(iii) a two-chain form of (i) or (ii) that comprises an A-chain and a B-chain, optionally wherein the A-chain and B-chain are linked by a disulfide bond, further optionally wherein the A chain is set forth in SEQ ID NO: 36 and a B chain is set forth in SEQ ID NO: 41; and/or
(g) the VEGF comprises a polypeptide sequence:
(i) that has at least at or about 85%, at least at or about 90% or at least at or about 95% sequence identity to the amino acid sequence set forth in SEQ ID NO: 7 or a sequence thereof that lacks a signal peptide, optionally wherein the VEGF comprises the sequence set forth in SEQ ID NO: 7 or a sequence thereof that lacks the signal peptide; or
(ii) that has at least at or about 85%, at least at or about 90% or at least at or about 95% sequence identity to the amino acid sequence set forth in SEQ ID NO: 44, optionally wherein the VEGF comprises the polypeptide sequence set forth in SEQ ID NO: 44.

3. The skin substitute of claim 1 or claim 2, wherein:
(a) the recombinant growth factor and the recombinant insulin are encoded by a bicistronic expression cassette comprising a polynucleotide encoding the recombinant growth factor and a polynucleotide encoding the recombinant insulin separated by a bicistronic element, optionally wherein:
(i) the bicistronic element is an IRES; and/or
(ii) the polynucleotide encoding the recombinant growth factor is upstream of the polynucleotide encoding the recombinant insulin in the bicistronic expression cassette;
(b) polynucleotides encoding the recombinant growth factor and the recombinant insulin are operably linked to a promoter, optionally wherein the promoter is a CAG promoter;
(c) cells of the stratified epidermis continuously secrete the recombinant growth factor and the recombinant insulin for up to or about 2 days, up to or about 3 days, up to or about 4 days, up to or about 5 days, up to or about 6 days, up to or about 7 days, up to or about 8 days, up to or about 9 days, up to or about 10 days, up to or about 11 days, up to or about 12 days, up to or about 13 days, up to or about 14 days or up to three weeks; and/or
(d) the cells of the stratified epidermis are differentiated from keratinocytes, optionally human keratinocytes further optionally HaCaT keratinocyte cells.

4. A bicistronic expression cassette comprising a polynucleotide encoding a recombinant human growth factor and a recombinant insulin, wherein:
the recombinant growth factor is a VEGF.

5. The bicistronic expression cassette of claim 4, wherein:
(a) the recombinant insulin is or comprises a recombinant human insulin;
(b) the encoded recombinant insulin has:
(i) the sequence of amino acids set forth in SEQ ID NO: 5: or
(ii) is a functional variant that has a sequence of amino acids that has at least 85%, at least 90%, or at least 95% sequence identity to SEQ ID NO: 5;
(c) the encoded recombinant insulin comprises the sequence of amino acids set forth in SEQ ID NO: 5;
(d) the encoded recombinant insulin is an AspB10 insulin analog comprising a histidine to aspartic acid mutation at position 10 in the B chain of the modified human proinsulin compared to wild-type insulin set forth in SEQ ID NO: 5;
(e) the polynucleotide encoding the recombinant insulin encodes a proinsulin comprising at least one furin recognition sequence in place of the endopeptidase Arg31-Arg32 cleavage site or the endopeptidase Lys64-Arg65 cleavage site, optionally wherein the at least one furin recognition sequence:
(i) is in place of the endopeptidase Arg31-Arg32 cleavage site and the endopeptidase Lys64-Arg65 cleavage site;
(ii) comprises the consensus sequence R-X-R-R, where X is any amino acid (SEQ ID NO: 8), or R-X-K-R, where X is any amino acid (SEQ ID NO: 9); and/or
(iii) is RTKR (SEQ ID NO: 10) or RQKR (SEQ ID NO: 42);
(f) the encoded recombinant insulin:
(i) comprises the sequence set forth in SEQ ID NO: 6;
(ii) has the sequence of amino acids set forth in SEQ ID NO: 6, or
(iii) is a functional variant that has a sequence of amino acids that has at least 85%, at least 90%, or at least 95% sequence identity to SEQ ID NO: 6; and/or
(g) the polynucleotide encoding the recombinant insulin comprises the sequence set forth in SEQ ID NO: 2 or has at least at or about 85%, at least at or about 90%, or at least at or about 95% sequence identity to the sequence set forth in SEQ ID NO: 2.

6. The bicistronic expression cassette of claim 4 or claim 5, wherein:
(a) the polynucleotide encoding the growth factor comprises the sequence set forth in SEQ ID NO: 4, or has at least at or about 85%, at least at or about 90%, or at least at or about 95% sequence identity to the sequence set forth in SEQ ID NO: 4;
(b) the encoded VEGF comprises a sequence that has at least at or about 85%, at least at or about 90% or at least at or about 95% sequence identity to the sequence set forth in SEQ ID NO: 7 or a sequence thereof that lacks the signal peptide, optionally wherein the encoded VEGF comprises the sequence set forth in SEQ ID NO: 7 or a sequence thereof that lacks a signal peptide;
(c) the encoded VEGF comprises a sequence that has at least at or about 85%, at least at or about 90% or at least at or about 95% sequence identity to the sequence set forth in SEQ ID NO: 44, optionally wherein the encoded VEGF comprises the sequence set forth in SEQ ID NO: 44;
(d) the polynucleotide encoding the recombinant growth factor and the polynucleotide encoding the recombinant insulin are separated by a bicistronic element, optionally wherein the bicistronic element is an IRES; and/or
(e) the polynucleotides encoding the recombinant growth factor and the recombinant insulin are operably linked to a promoter, optionally wherein:
(i) the polynucleotides encoding the recombinant growth factor and the recombinant insulin are operably linked to the same promoter;
(ii) the promoter is a CAG promoter; and/or
(iii) the polynucleotide encoding the recombinant growth factor is upstream of the polynucleotide encoding the recombinant insulin in the bicistronic expression cassette.

7. A vector comprising the bicistronic expression cassette of any of claims 4-6, optionally wherein:
(a) the vector is a viral vector, optionally wherein the viral vector is an adenoviral vector;
(b) the vector is a non-replicative type 5 adenovirus, optionally wherein the non-replicative adenovirus lacks or is deleted in a functional E1 and E3 region, optionally wherein the E1 and E3 comprise a genetic disruption; and/or
(c) the bicistronic expression cassette is inserted into the E1 region.

8. A method of manufacturing a skin substitute, wherein the method comprises:
1) differentiating keratinocytes into a stratified epidermis, wherein the stratified epidermis comprises a basal layer, a spinous layer, a granular layer and a stratum corneum; and
2) introducing a bicistronic expression cassette of any of claims 4-6 or the vector of claim 7 into cells of the stratified epidermis to produce a skin substitute, wherein the skin substitute comprises a recombinant growth factor and a recombinant insulin; or
2) transducing a viral vector of claim 7 into cells of the stratified epidermis to produce a skin substitute, wherein the skin substitute comprises a growth factor and an insulin.

9. The method of claim 8, wherein:
(a) the introducing or the transducing is into cells of the basal layer;
(b) prior to the differentiating in step 1), the method comprises culturing the keratinocytes in a low calcium medium for 2 weeks, 3 weeks, 4 weeks, 5 weeks, or 6 weeks, optionally at or about 4 weeks, optionally wherein the low calcium medium comprises a calcium concentration of 0.01-0.1 mM at the time of seeding the cells or during the culturing; and/or
(c) the keratinocytes are human keratinocytes, optionally wherein the keratinocytes are HaCaT keratinocyte cells.

10. The method of claim 8 or claim 9, wherein step 1) comprises culturing the keratinocytes on an extracellular matrix substrate, optionally wherein:
(a) the extracellular matrix substrate is collagen;
(b) the culturing in step (1) is for about 23 to 28 days; and/or
(c) the culturing in step (1) comprises a first incubation in low calcium medium and a second incubation in a high calcium medium, optionally wherein:
(i) the first incubation in low calcium medium is for about 3-5 days, and the second incubation in high calcium medium is for about 20-23 days;
(ii) the low calcium medium comprises 0.01-0.1 mM calcium;
(iii) the high calcium medium comprises 1.0-3.0 mM calcium;
(iv) the low calcium medium and the high calcium medium further comprise EGF and BPE, optionally wherein:
(A) the low calcium medium and the high calcium medium comprise 0.05 ng/mL to 1 ng/ml EGF and from 1 µg/ml to 100 µg/ml BPE; and/or
(B) the low calcium medium and the high calcium medium comprise at or about 0.2 ng/ml EGF and at or about 30 µg/ml BPE; and/or
(v) the high calcium medium further comprises hydrocortisone, optionally wherein the high calcium medium comprises from 0.1 to 1.0 µg/ml hydrocortisone, optionally wherein the high calcium medium comprises at or about 0.4 µg/ml hydrocortisone.

11. A cryopreserved skin substitute, comprising the skin substitute of any of claims 1-3 and a cryoprotectant, optionally wherein the cryoprotectant comprises human albumin (0.02 g/mL) and D-glucose (0.09 g/mL).

12. A skin substitute dressing comprising the skin substitute of any of claims 1-3 or the cryopreserved skin substitute of claim 11 and an absorbent gauze, wherein the cryopreserved skin substitute is overlaid on the absorbent gauze.

13. A container comprising the skin substitute of any of claims 1-3, the cryopreserved skin substitute of claim 11, or the skin substitute dressing of claim 12.

14. A method for preparing a skin substitute dressing, the method comprising placing the skin substitute of any of claims 1-3 or the cryopreserved skin substitute of claim 11 on an absorbent gauze.

15. The skin substitute of any of claims 1-3, the cryopreserved skin substitute of claim 11 or the skin substitute dressing of claim 12 for use in a method of promoting wound healing, wherein the method comprises applying to a wound the skin substitute, the cryopreserved skin substitute or the skin substitute dressing, optionally wherein:
(a) the wound is selected from the group consisting of: a sore, an open wound, an ulcer, and an abscess;
(b) the skin substitute is applied to a wound on a diabetic patient;
(c) the wound is a diabetic ulcer;
(d) the wound is a diabetic foot ulcer; and/or
(e) the wound is a venous leg ulcer.

## Patentansprüche

1. Hautersatz, umfassend eine mehrschichtige Epidermis, die eine Basalschicht, eine Spinozenschicht, eine Granularschicht und eine Hornschicht umfasst, wobei Zellen der mehrschichtigen Epidermis einen rekombinanten Wachstumsfaktor und ein rekombinantes Insulin exprimieren:
wobei der rekombinante Wachstumsfaktor ein vaskulärer endothelialer Wachstumsfaktor (VEGF) ist.

2. Hautersatz nach Anspruch 1, wobei:
(a) der rekombinante Wachstumsfaktor und das rekombinante Insulin von Zellen der mehrschichtigen Epidermis sekretiert werden können, optional wobei die Zellen der mehrschichtigen Epidermis, die den rekombinanten Wachstumsfaktor und das rekombinante Insulin exprimieren, Zellen der Basalschicht umfassen;
(b) das rekombinante Insulin ein rekombinantes Humaninsulin ist oder ein solches umfasst;
(c) das rekombinante Insulin umfasst:
(i) die in SEQ ID NO: 5 angegebene Aminosäuresequenz;
(ii) eine funktionelle Variante, deren Aminosäuresequenz eine Sequenzidentität von mindestens 85 %, mindestens 90 % oder mindestens 95 % zu SEQ ID NO: 5 aufweist; oder
(iii) eine Zweikettenform von (i) oder (ii), die eine A-Kette und eine B-Kette umfasst, optional wobei die A-Kette und die B-Kette durch eine Disulfidbindung verbunden sind;
(d) das rekombinante Insulin ein AspB10-Insulinanalogon ist, das im Vergleich zum in SEQ ID NO: 5 dargestellten Wildtyp-Insulin eine Mutation von Histidin zu Asparaginsäure an Position 10 in der B-Kette des modifizierten humanen Proinsulins umfasst;
(e) der Hautersatz ein Polynukleotid umfasst, das für ein Proinsulin codiert, das anstelle der Endopeptidase-Spaltstelle Arg31-Arg32 oder der Endopeptidase-Spaltstelle Lys64-Arg65 mindestens eine Furin-Erfassungssequenz umfasst, optional wobei die mindestens eine Furin-Erfassungssequenz:
(i) anstelle der Endopeptidase-Spaltstelle Arg31-Arg32 und der Endopeptidase-Spaltstelle Lys64-Arg65 vorliegt;
(ii) die Konsensussequenz R-X-R-R umfasst, wobei X eine beliebige Aminosäure (SEQ ID NO: 8) ist, oder R-X-K-R, wobei X eine beliebige Aminosäure (SEQ ID NO: 9) ist; und/oder
(iii) RTKR (SEQ ID NO: 10) oder RQKR (SEQ ID NO: 42) ist;
(f) das rekombinante Insulin umfasst oder aufweist:
(i) die in SEQ ID NO: 6 angegebene Aminosäuresequenz;
(ii) eine funktionelle Variante, deren Aminosäuresequenz eine Sequenzidentität von mindestens 85 %, mindestens 90 % oder mindestens 95 % zu SEQ ID NO: 6 aufweist; oder
(iii) eine Zweikettenform von (i) oder (ii), die eine A-Kette und eine B-Kette umfasst, optional wobei die A-Kette und die B-Kette durch eine Disulfidbindung verbunden sind, ferner optional wobei die A-Kette in SEQ ID NO: 36 und die B-Kette in SEQ ID NO: 41 angegeben ist; und/oder
(g) das VEGF eine Polypeptidsequenz umfasst:
(i) die mindestens etwa 85 %, mindestens etwa 90 % oder mindestens etwa 95 % Sequenzidentität mit der in SEQ ID NO: 7 dargestellten Aminosäuresequenz oder einer Sequenz davon aufweist, der das Signalpeptid fehlt, optional wobei das VEGF die in SEQ ID NO: 7 dargestellte Sequenz oder eine Sequenz davon umfasst, der das Signalpeptid fehlt; oder
(ii) das eine Sequenzidentität von mindestens etwa 85 %, mindestens etwa 90 % oder mindestens etwa 95 % zu der in SEQ ID NO: 44 angegebenen Aminosäuresequenz aufweist, optional wobei das VEGF die in SEQ ID NO: 44 angegebene Polypeptidsequenz umfasst.

3. Hautersatz nach Anspruch 1 oder Anspruch 2, wobei:
(a) der rekombinante Wachstumsfaktor und das rekombinante Insulin von einer bicistronischen Expressionskassette codiert werden, die ein Polynukleotid, das den rekombinanten Wachstumsfaktor codiert, und ein Polynukleotid, das das rekombinante Insulin codiert, umfasst, die optional durch ein bicistronisches Element getrennt sind, optional wobei:
(i) das bicistronische Element ein IRES ist; und/oder
(ii) das für den rekombinanten Wachstumsfaktor codierende Polynukleotid in der bicistronischen Expressionskassette stromaufwärts des für das rekombinante Insulin codierenden Polynukleotids liegt;
(b) die für den rekombinanten Wachstumsfaktor und das rekombinante Insulin codierenden Polynukleotide funktionsfähig mit einem Promotor verbunden sind, optional wobei der Promotor optional ein CAG-Promotor ist;
(c) Zellen der mehrschichtigen Epidermis den rekombinanten Wachstumsfaktor und das rekombinante Insulin kontinuierlich für bis zu oder etwa 2 Tage, bis zu oder etwa 3 Tage, bis zu oder etwa 4 Tage, bis zu oder etwa 5 Tage, bis zu oder etwa 6 Tage, bis zu oder etwa 7 Tage, bis zu oder etwa 8 Tage, bis zu oder etwa 9 Tage, bis zu oder etwa 10 Tage, bis zu oder etwa 11 Tage, bis zu oder etwa 12 Tage, bis zu oder etwa 13 Tage, bis zu oder etwa 14 Tage oder bis zu drei Wochen ausscheiden; und/oder
(d) die Zellen der mehrschichtigen Epidermis von Keratinozyten abstammen, optional von menschlichen Keratinozyten und ferner optional von HaCaT-Keratinozyten.

4. Bicistronische Expressionskassette, die ein Polynukleotid umfasst, das für einen rekombinanten menschlichen Wachstumsfaktor und ein rekombinantes Insulin codiert, wobei:
der rekombinante Wachstumsfaktor ein VEGF ist.

5. Bicistronische Expressionskassette nach Anspruch 4, wobei:
(a) das rekombinante Insulin ein rekombinantes Humaninsulin ist oder ein solches umfasst;
(b) das codierte rekombinante Insulin:
(i) die in SEQ ID NO: 5 angegebene Aminosäuresequenz aufweist oder
(ii) eine funktionelle Variante ist, deren Aminosäuresequenz eine Sequenzidentität von mindestens 85 %, mindestens 90 % oder mindestens 95 % zu SEQ ID NO: 5 aufweist;
(c) das codierte rekombinante Insulin die in SEQ ID NO: 5 dargestellte Aminosäuresequenz umfasst;
(d) das codierte rekombinante Insulin ein AspB10-Insulinanalogon ist, das im Vergleich zum in SEQ ID NO: 5 dargestellten Wildtyp-Insulin eine Mutation von Histidin zu Asparaginsäure an Position 10 in der B-Kette des modifizierten humanen Proinsulins umfasst;
(e) das für das rekombinante Insulin codierende Polynukleotid ein Proinsulin codiert, das anstelle der Endopeptidase-Spaltstelle Arg31-Arg32 oder der Endopeptidase-Spaltstelle Lys64-Arg65 mindestens eine Furin-Erfassungssequenz umfasst, optional wobei die mindestens eine Furin-Erfassungssequenz:
(i) anstelle der Endopeptidase-Spaltstelle Arg31-Arg32 und der Endopeptidase-Spaltstelle Lys64-Arg65 vorliegt;
(ii) die Konsensussequenz R-X-R-R umfasst, wobei X eine beliebige Aminosäure (SEQ ID NO: 8) ist, oder R-X-K-R, wobei X eine beliebige Aminosäure (SEQ ID NO: 9) ist; und/oder
(iii) RTKR (SEQ ID NO: 10) oder RQKR (SEQ ID NO: 42) ist;
(f) das codierte rekombinante Insulin:
(i) die in SEQ ID NO: 6 angegebene Sequenz umfasst;
(ii) die in SEQ ID NO: 6 angegebene Aminosäuresequenz aufweist, oder
(iii) eine funktionelle Variante ist, deren Aminosäuresequenz eine Sequenzidentität von mindestens 85 %, mindestens 90 % oder mindestens 95 % zu SEQ ID NO: 6 aufweist; und/oder
(g) das Polynukleotid, das das rekombinante Insulin codiert, die in SEQ ID NO: 2 angegebene Sequenz umfasst oder eine Sequenzidentität von mindestens etwa 85 %, mindestens etwa 90 % oder mindestens etwa 95 % zu der in SEQ ID NO: 2 angegebenen Aminosäuresequenz aufweist.

6. Bicistronische Expressionskassette nach Anspruch 4 oder Anspruch 5, wobei:
(a) das Polynukleotid, das den Wachstumsfaktor codiert, die in SEQ ID NO: 4 angegebene Sequenz umfasst oder eine Sequenzidentität von mindestens etwa 85 %, mindestens etwa 90 % oder mindestens etwa 95 % zu der in SEQ ID NO: 4 angegebenen Aminosäuresequenz aufweist;
(b) das codierte VEGF eine Sequenz umfasst, die mindestens etwa 85 %, mindestens etwa 90 % oder mindestens etwa 95 % Sequenzidentität zu der in SEQ ID NO: 7 angegebenen Sequenz aufweist, oder eine Sequenz davon, der das Signalpeptid fehlt, optional wobei das codierte VEGF die in SEQ ID NO: 7 dargestellte Sequenz oder eine Sequenz davon umfasst, der ein Signalpeptid fehlt; oder
(c) das codierte VEGF eine Sequenz umfasst, die eine Sequenzidentität von mindestens etwa 85 %, mindestens etwa 90 % oder mindestens etwa 95 % zu der in SEQ ID NO: 44 angegebenen Aminosäuresequenz aufweist, optional wobei das codierte VEGF die in SEQ ID NO: 44 angegebene Sequenz umfasst;
(d) das den rekombinanten Wachstumsfaktor codierende Polynukleotid und das das rekombinante Insulin codierende Polynukleotid durch ein bicistronisches Element voneinander getrennt sind, optional wobei das bicistronische Element ein IRES ist; und/oder
(e) die für den rekombinanten Wachstumsfaktor und das rekombinante Insulin codierenden Polynukleotide funktionsfähig mit einem Promotor verbunden sind, optional wobei:
(i) die für den rekombinanten Wachstumsfaktor und das rekombinante Insulin codierenden Polynukleotide funktionsfähig mit demselben Promotor verbunden sind;
(ii) der Promotor ein CAG-Promotor ist; und/oder
(iii) das für den rekombinanten Wachstumsfaktor codierende Polynukleotid in der bicistronischen Expressionskassette stromaufwärts des für das rekombinante Insulin codierenden Polynukleotids liegt.

7. Vektor, der die bicistronische Expressionskassette nach einem der Ansprüche 4-6 umfasst, optional wobei:
(a) der Vektor ein viraler Vektor ist, optional wobei der virale Vektor ein adenoviraler Vektor ist;
(b) der Vektor ein nicht-replikativer Typ-5-Adenovirus ist, optional wobei dem nichtreplikativen Adenovirus eine funktionelle E1- und E3-Region fehlt oder diese deletiert ist, optional wobei die E1- und E3-Regionen eine genetische Disruption umfassen; und/oder
(c) die bicistronische Expressionskassette in die E1-Region eingefügt ist.

8. Verfahren zur Herstellung eines Hautersatzes, wobei das Verfahren umfasst:
1) Differenzieren von Keratinozyten zu einer mehrschichtigen Epidermis, wobei die mehrschichtige Epidermis eine Basalschicht, eine Stachelschicht, eine Granularschicht und eine Hornschicht umfasst; und
2) Einbringen einer bicistronischen Expressionskassette nach einem der Ansprüche 4-6 oder des Vektors nach Anspruch 7 in Zellen der mehrschichtigen Epidermis, um einen Hautersatz herzustellen, wobei der Hautersatz einen rekombinanten Wachstumsfaktor und ein rekombinantes Insulin umfasst; oder
2) Transduzieren eines viralen Vektors nach Anspruch 7 in Zellen der mehrschichtigen Epidermis, um einen Hautersatz herzustellen, wobei der Hautersatz einen Wachstumsfaktor und ein Insulin umfasst.

9. Verfahren nach Anspruch 8, wobei:
(a) das Einbringen oder das Transduzieren in Zellen der Basalschicht erfolgt;
(b) vor der Differenzierung in Schritt 1) das Verfahren die Kultivierung der Keratinozyten in einem kalziumarmen Medium für 2 Wochen, 3 Wochen, 4 Wochen, 5 Wochen oder 6 Wochen, optional bei oder um 4 Wochen umfasst, optional wobei das kalziumarme Medium zum Zeitpunkt der Zellaussaat oder während des Kultivierens eine Kalziumkonzentration von 0,01-0,1 mM aufweist; und/oder
(c) die Keratinozyten menschliche Keratinozyten sind, optional wobei die Keratinozyten HaCaT-Keratinozyten sind.

10. Verfahren nach Anspruch 8 oder Anspruch 9, wobei Schritt 1) die Kultivierung der Keratinozyten auf einem extrazellulären Matrixsubstrat umfasst, optional wobei:
(a) das extrazelluläre Matrixsubstrat Kollagen ist;
(b) die Kultivierung in Schritt (1) etwa 23 bis 28 Tage dauert; und/oder
(c) die Kultivierung in Schritt (1) eine erste Inkubation in einem Medium mit niedrigem Kalziumgehalt und eine zweite Inkubation in einem Medium mit hohem Kalziumgehalt umfasst, optional wobei:
(i) die erste Inkubation in einem Medium mit niedrigem Kalziumgehalt etwa 3-5 Tage dauert, und die zweite Inkubation in einem Medium mit hohem Kalziumgehalt etwa 20-23 Tage dauert;
(ii) das kalziumarme Medium 0,01-0,1 mM Kalzium umfasst;
(iii) das kalziumreiche Medium 1,0-3,0 mM Kalzium umfasst;
(iv) das kalziumarme Medium und das kalziumreiche Medium ferner EGF und BPE umfassen, optional wobei:
(A) das kalziumarme Medium und das kalziumreiche Medium 0,05 ng/ml bis 1 ng/ml EGF und 1 µg/ml bis 100 µg/ml BPE umfassen; und/oder
(B) das kalziumarme Medium und das kalziumreiche Medium etwa 0,2 ng/ml EGF und etwa 30 µg/ml BPE umfassen; und/oder
(v) das kalziumreiche Medium ferner Hydrocortison umfasst, optional wobei das kalziumreiche Medium 0,1 bis 1,0 µg/ml Hydrocortison umfasst, optional wobei das kalziumreiche Medium 0,4 µg/ml oder etwa 0,4 µg/ml Hydrocortison umfasst.

11. Kryokonservierter Hautersatz, der den Hautersatz nach einem der Ansprüche 1-3 und ein Kryoprotektivum umfasst, wobei das Kryoprotektivum optional Humanalbumin (0,02 g/ml) und D-Glukose (0,09 g/ml) umfasst.

12. Hautersatzverband, der den Hautersatz nach einem der Ansprüche 1-3 oder den kryokonservierten Hautersatz nach Anspruch 11 und eine saugfähige Gaze umfasst, wobei der kryokonservierte Hautersatz auf die saugfähige Gaze aufgelegt ist.

13. Behälter, der den Hautersatz nach einem der Ansprüche 1-3, den kryokonservierten Hautersatz nach Anspruch 11 oder den Hautersatzverband nach Anspruch 12 enthält.

14. Verfahren zur Herstellung eines Hautersatzverbands, wobei das Verfahren das Aufbringen des Hautersatzes nach einem der Ansprüche 1-3 oder des kryokonservierten Hautersatzes nach Anspruch 11 auf eine saugfähige Gaze umfasst.

15. Hautersatz nach einem der Ansprüche 1-3, kryokonservierter Hautersatz nach Anspruch 11 oder Hautersatzverband nach Anspruch 12 zur Verwendung in einem Verfahren zur Förderung der Wundheilung, wobei das Verfahren das Aufbringen des Hautersatzes, des kryokonservierten Hautersatzes oder des Hautersatzverbands auf eine Wunde umfasst, optional wobei:
(a) die Wunde aus der Gruppe ausgewählt ist, bestehend aus: einer Wundstelle, einer offenen Wunde, einem Geschwür und einem Abszess;
(b) der Hautersatz auf eine Wunde eines Diabetespatienten aufgebracht wird;
(c) die Wunde ein diabetisches Geschwür ist;
(d) die Wunde ein diabetisches Fußgeschwür ist; und/oder
(e) die Wunde ein venöses Beingeschwür ist.

## Revendications

1. Substitut cutané comprenant un épiderme stratifié comprenant une couche basale, une couche épineuse, une couche granulaire et une couche cornée, dans lequel des cellules de l'épiderme stratifié expriment un facteur de croissance recombinant et une insuline recombinante :
dans lequel le facteur de croissance recombinant est un facteur de croissance endothélial vasculaire (Vascular Endothelial Growth Factor, VEGF).

2. Substitut cutané de la revendication 1, dans lequel :
(a) le facteur de croissance recombinant et l'insuline recombinante peuvent être sécrétés à partir de cellules de l'épiderme stratifié, facultativement dans lequel les cellules de l'épiderme stratifié qui expriment le facteur de croissance recombinant et l'insuline recombinante comprennent des cellules de la couche basale ;
(b) l'insuline recombinante est ou comprend une insuline humaine recombinante ;
(c) l'insuline recombinante comprend :
(i) la séquence d'acides aminés présentée dans SEQ ID n° : 5 ;
(ii) une variante fonctionnelle qui a une séquence d'acides aminés qui a au moins 85 %, au moins 90 %, ou au moins 95 % d'identité de séquence avec SEQ ID n° : 5 ; ou
(iii) une forme à deux chaînes de (i) ou de (ii) qui comprend une chaîne A et une chaîne B, facultativement dans lequel la chaîne A et la chaîne B sont liées par une liaison disulfure ;
(d) l'insuline recombinante est un analogue d'insuline AspB10 comprenant une mutation histidine-acide aspartique à la position 10 dans la chaîne B de la proinsuline humaine modifiée par comparaison à de l'insuline de type sauvage présentée dans SEQ ID n° : 5 ;
(e) le substitut cutané comprend un polynucléotide encodant une proinsuline comprenant au moins une séquence de reconnaissance de furine à la place du site de clivage Arg31-Arg32 d'endopeptidase ou du site de clivage Lys64-Arg65 d'endopeptidase, facultativement dans lequel l'au moins une séquence de reconnaissance de furine :
(i) est à la place du site de clivage Arg31-Arg32 d'endopeptidase et du site de clivage Lys64-Arg65 d'endopeptidase ;
(ii) comprend la séquence de consensus R-X-R-R, où X est un quelconque acide aminé (SEQ ID n° : 8), ou R-X-K-R, où X est un quelconque acide aminé (SEQ ID n° : 9) ; et/ou
(iii) est RTKR (SEQ ID n° : 10) ou RQKR (SEQ ID n° : 42) ;
(f) l'insuline recombinante comprend ou a :
(i) la séquence d'acides aminés présentée dans SEQ ID n° : 6 ;
(ii) une variante fonctionnelle qui a une séquence d'acides aminés qui a au moins 85 %, au moins 90 %, ou au moins 95 % d'identité de séquence avec SEQ ID n° : 6 ; ou
(iii) une forme à deux chaînes de (i) ou de (ii) qui comprend une chaîne A et une chaîne B, facultativement dans lequel la chaîne A et la chaîne B sont liées par une liaison disulfure, facultativement en outre dans lequel la chaîne A est présentée dans SEQ ID n° : 36 et une chaîne B est présentée dans SEQ ID n° : 41 ; et/ou
(g) le VEGF comprend une séquence polypeptidique :
(i) qui a au moins une quantité à, ou environ à, 85 %, au moins une quantité à, ou environ à, 90 %, ou au moins une quantité à, ou environ à, 95 % d'identité de séquence avec la séquence d'acides aminés présentée dans SEQ ID n° : 7 ou une séquence de celle-ci qui n'a pas un peptide, facultativement dans lequel le VEGF comprend la séquence présentée dans SEQ ID n° : 7 ou une séquence de celle-ci qui n'a pas le peptide ; ou
(ii) qui a au moins une quantité à, ou environ à, 85 %, au moins une quantité à, ou environ à, 90 %, ou au moins une quantité à, ou environ à, 95 % d'identité de séquence avec la séquence d'acides aminés présentée dans SEQ ID n° : 44, facultativement dans lequel le VEGF comprend la séquence polypeptidique présentée dans SEQ ID n° : 44.

3. Substitut cutané de la revendication 1 ou de la revendication 2, dans lequel :
(a) le facteur de croissance recombinant et l'insuline recombinante sont encodés par une cassette d'expression bicistronique comprenant un polynucléotide encodant le facteur de croissance recombinant et un polynucléotide encodant l'insuline recombinante séparé par un élément bicistronique, facultativement dans lequel :
(i) l'élément bicistronique est un IRES ; et/ou
(ii) le polynucléotide encodant le facteur de croissance recombinant est en amont du polynucléotide encodant l'insuline recombinante dans la cassette d'expression bicistronique ;
(b) des polynucléotides encodant le facteur de croissance recombinant et l'insuline recombinante sont opérationnellement liés à un promoteur, facultativement dans lequel le promoteur est un promoteur CAG ;
(c) des cellules de l'épiderme stratifié secrètent en continu le facteur de croissance recombinant et l'insuline recombinante pendant une période pouvant durer, ou d'environ, 2 jours, une période pouvant durer, ou d'environ, 3 jours, une période pouvant durer, ou d'environ, 4 jours, une période pouvant durer, ou d'environ, 5 jours, une période pouvant durer, ou d'environ, 6 jours, une période pouvant durer, ou d'environ, 7 jours, une période pouvant durer, ou d'environ, 8 jours, une période pouvant durer, ou d'environ, 9 jours, une période pouvant durer, ou d'environ, 10 jours, une période pouvant durer, ou d'environ, 11 jours, une période pouvant durer, ou d'environ, 12 jours, une période pouvant durer, ou d'environ, 13 jours, une période pouvant durer, ou d'environ, 14 jours, ou une période pouvant durer trois semaines ; et/ou
(d) les cellules de l'épiderme stratifié sont différentiées de kératinocytes, facultativement de kératinocytes humaines, facultativement en outre de cellules kératinocytes HaCaT.

4. Cassette d'expression bicistronique, comprenant un polynucléotide encodant un facteur de croissance humain recombinant et une insuline recombinante, dans laquelle :
le facteur de croissance recombinant est un VEGF.

5. Cassette d'expression bicistronique de la revendication 4, dans laquelle :
(a) l'insuline recombinante est ou comprend une insuline humaine recombinante ;
(b) l'insuline recombinante encodée a :
(i) la séquence d'acides aminés présentée dans SEQ ID n° : 5 : ou
(ii) est une variante fonctionnelle qui a une séquence d'acides aminés qui a au moins 85 %, au moins 90 %, ou au moins 95 % d'identité de séquence avec SEQ ID n° : 5 ;
(c) l'insuline recombinante encodée comprend la séquence d'acides aminés présentée dans SEQ ID n° : 5 ;
(d) l'insuline recombinante encodée est un analogue d'insuline AspB10 comprenant une mutation histidine-acide aspartique à la position 10 dans la chaîne B de la proinsuline humaine modifiée par comparaison à de l'insuline de type sauvage présentée dans SEQ ID n° : 5 ;
(e) le polynucléotide encodant l'insuline recombinante encode une proinsuline comprenant au moins une séquence de reconnaissance de furine à la place du site de clivage Arg31-Arg32 d'endopeptidase ou du site de clivage Lys64-Arg65 d'endopeptidase, facultativement dans laquelle l'au moins une séquence de reconnaissance de furine :
(i) est à la place du site de clivage Arg31-Arg32 d'endopeptidase et du site de clivage Lys64-Arg65 d'endopeptidase ;
(ii) comprend la séquence de consensus R-X-R-R, où X est un quelconque acide aminé (SEQ ID n° : 8), ou R-X-K-R, où X est un quelconque acide aminé (SEQ ID n° : 9) ; et/ou
(iii) est RTKR (SEQ ID n° : 10) ou RQKR (SEQ ID n° : 42) ;
(f) l'insuline recombinante encodée :
(i) comprend la séquence présentée dans SEQ ID n° : 6 ;
(ii) a la séquence d'acides aminés présentée dans SEQ ID n° : 6, ou
(iii) est une variante fonctionnelle qui a une séquence d'acides aminés qui a au moins 85 %, au moins 90 %, ou au moins 95 % d'identité de séquence avec SEQ ID n° : 6 ; et/ou
(g) le polynucléotide encodant l'insuline recombinante comprend la séquence présentée dans SEQ ID n° : 2 ou a au moins une quantité à, ou environ à, 85 %, au moins une quantité à, ou environ à, 90 %, ou au moins une quantité à, ou environ à, 95 % d'identité de séquence avec la séquence présentée dans SEQ ID n° : 2.

6. Cassette d'expression bicistronique de la revendication 4 ou de la revendication 5, dans laquelle :
(a) le polynucléotide encodant le facteur de croissance comprend la séquence présentée dans SEQ ID n° : 4, ou a au moins une quantité à, ou environ à, 85 %, au moins une quantité à, ou environ à, 90 %, ou au moins une quantité à, ou environ à, 95 % d'identité de séquence avec la séquence présentée dans SEQ ID n° : 4 ;
(b) le VEGF encodé comprend une séquence qui a au moins une quantité à, ou environ à, 85 %, au moins une quantité à, ou environ à, 90 %, ou au moins une quantité à, ou environ à, 95 % d'identité de séquence avec la séquence présentée dans SEQ ID n° : 7 ou une séquence de celle-ci qui n'a pas le peptide, facultativement dans laquelle le VEGF encodé comprend la séquence présentée dans SEQ ID n° : 7 ou une séquence de celle-ci qui n'a pas un peptide ;
(c) le VEGF encodé comprend une séquence qui a au moins une quantité à, ou environ à, 85 %, au moins une quantité à, ou environ à, 90 %, ou au moins une quantité à, ou environ à, 95 % d'identité de séquence avec la séquence présentée dans SEQ ID n° : 44, facultativement dans laquelle le VEGF encodé comprend la séquence présentée dans SEQ ID n° : 44 ;
(d) le polynucléotide encodant le facteur de croissance recombinant et le polynucléotide encodant l'insuline recombinante sont séparés par un élément bicistronique, facultativement dans laquelle l'élément bicistronique est un IRES ; et/ou
(e) les polynucléotides encodant le facteur de croissance recombinant et l'insuline recombinante sont opérationnellement liés à un promoteur, facultativement dans laquelle :
(i) les polynucléotides encodant le facteur de croissance recombinant et l'insuline recombinante sont opérationnellement liés au même promoteur ;
(ii) le promoteur est un promoteur CAG ; et/ou
(iii) le polynucléotide encodant le facteur de croissance recombinant est en amont du polynucléotide encodant l'insuline recombinante dans la cassette d'expression bicistronique.

7. Vecteur comprenant la cassette d'expression bicistronique de quelconques des revendications 4 à 6, facultativement dans lequel :
(a) le vecteur est un vecteur viral, facultativement dans lequel le vecteur viral est un vecteur adénoviral ;
(b) le vecteur est un adénovirus type 5 non réplicatif, facultativement dans lequel l'adénovirus non réplicatif n'a pas ou est délété en une région E1 et E3 fonctionnelle, facultativement dans lequel E1 et E3 comprennent une disruption génétique ; et/ou
(c) la cassette d'expression bicistronique est insérée dans la région E1.

8. Procédé de fabrication d'un substitut cutané, dans lequel le procédé comprend :
1) la différentiation de kératinocytes en un épiderme stratifié, dans lequel l'épiderme stratifié comprend une couche basale, une couche épineuse, une couche granulaire, et une couche cornée ; et
2) l'introduction d'une cassette d'expression bicistronique de quelconques des revendications 4 à 6 ou du vecteur de la revendication 7 dans des cellules de l'épiderme stratifié pour produire un substitut cutané, dans lequel le substitut cutané comprend un facteur de croissance recombinant et une insuline recombinante ; ou
2) la transduction d'un vecteur viral de la revendication 7 dans des cellules de l'épiderme stratifié pour produire un substitut cutané, dans lequel le substitut cutané comprend un facteur de croissance et une insuline.

9. Procédé de la revendication 8, dans lequel :
(a) l'introduction ou la transduction est dans des cellules de la couche basale ;
(b) avant la différentiation dans l'étape 1), le procédé comprend la mise en culture des kératinocytes dans un milieu à basse teneur en calcium pendant 2 semaines, 3 semaines, 4 semaines, 5 semaines, ou 6 semaines, facultativement une période pouvant durer, ou d'environ, 4 semaines, facultativement dans lequel le milieu à basse teneur en calcium comprend une concentration en calcium de 0,01 à 0,1 mM au moment d'ensemencement des cellules ou durant la mise en culture ; et/ou
(c) les kératinocytes sont des kératinocytes humaines, facultativement dans lequel les kératinocytes sont des cellules kératinocytes HaCaT.

10. Procédé de la revendication 8 ou de la revendication 9, dans lequel l'étape 1) comprend la mise en culture des kératinocytes sur un substrat matrice extracellulaire, facultativement dans lequel :
(a) le substrat matrice extracellulaire est du collagène ;
(b) la mise en culture dans l'étape (1) est pendant environ 23 à 28 jours ; et/ou
(c) la mise en culture dans l'étape (1) comprend une première incubation dans un milieu à basse teneur en calcium et une seconde incubation dans un milieu à haute teneur en calcium, facultativement dans lequel :
(i) la première incubation dans le milieu à basse teneur en calcium est pendant environ 3 à 5 jours, et la seconde incubation dans milieu à haute teneur en calcium est pendant environ 20 à 23 jours ;
(ii) le milieu à basse teneur en calcium comprend de 0,01 à 0,1 mM de calcium ;
(iii) le milieu à haute teneur en calcium comprend de 1,0 à 3,0 mM de calcium ;
(iv) le milieu à basse teneur en calcium et le milieu à haute teneur en calcium comprennent en outre EGF et BPE, facultativement dans lequel :
(A) le milieu à basse teneur en calcium et le milieu à haute teneur en calcium comprennent de 0,05 ng/mL à 1 ng/ml EGF et de 1 µg/ml à 100 µg/ml BPE ; et/ou
(B) le milieu à basse teneur en calcium et le milieu à haute teneur en calcium comprennent une quantité à, ou environ à, 0,2 ng/ml de EGF et une quantité à, ou environ à, 30 µg/ml de BPE ; et/ou
(v) le milieu à haute teneur en calcium comprend en outre de l'hydrocortisone, facultativement dans lequel le milieu à haute teneur en calcium comprend de 0,1 à 1,0 µg/ml d'hydrocortisone, facultativement dans lequel le milieu à haute teneur en calcium comprend une quantité à, ou environ à, 0,4 µg/ml d'hydrocortisone.

11. Substitut cutané cryoconservé, comprenant le substitut cutané de quelconques des revendications 1 à 3 et un agent cryoprotecteur, facultativement dans lequel l'agent cryoprotecteur comprend de l'albumine humaine (0,02 g/mL) et du D-saccharose (0,09 g/mL).

12. Substitut cutané pansement comprenant le substitut cutané de quelconques des revendications 1 à 3 ou le substitut cutané cryoconservé de la revendication 11 et une gaze absorbante, dans lequel le substitut cutané cryoconservé est superposé sur la gaze absorbante.

13. Contenant comprenant le substitut cutané de quelconques des revendications 1 à 3, le substitut cutané cryoconservé de la revendication 11, ou le pansement avec substitut cutané de la revendication 12.

14. Procédé pour préparer un pansement avec substitut cutané, le procédé comprenant le placement du substitut cutané de quelconques des revendications 1 à 3 ou du substitut cutané cryoconservé de la revendication 11 sur une gaze absorbante.

15. Substitut cutané de quelconques des revendications 1 à 3, substitut cutané cryoconservé de la revendication 11, ou pansement avec substitut cutané de la revendication 12 pour utilisation dans une méthode pour favoriser la guérison de blessure, dans lequel la méthode comprend l'application, sur une blessure, du substitut cutané, du substitut cutané cryoconservé, ou du pansement avec substitut cutané, facultativement dans lequel :
(a) la blessure est sélectionnée parmi le groupe constitué de : une plaie, une blessure ouverte, un ulcère, et un abcès ;
(b) le substitut cutané est appliqué sur une blessure sur un patient diabétique ;
(c) la blessure est un ulcère diabétique ;
(d) la blessure est un ulcère de pied diabétique ; et/ou
(e) la blessure est un ulcère de jambe veineux.
